# EUROPEAN PATENT APPLICATION

(11) **EP 1 498 138 A1**
(43) Date of publication of application: **19.01.2005**
(21) Application number: 03719122.8
(22) Date of filing: 17.04.2003
(51) Int. Cl.: A61K 45/00, A61K 31/445, A61K 31/452, A61K 31/454, A61K 31/4545, A61K 31/55, A61P 31/18, A61P 37/04

(54) **PREVENTIVES FOR HIV INFECTION**

(30) Priority: 19.04.2002 JP 2002118055; 16.05.2002 JP 2002141657
(71) Applicant: Takeda Pharmaceutical Company Limited, Osaka (JP)
(72) Inventor: TAKASHIMA, Katsunori, Sennan-gun, Osaka 590-0413 (JP); IIZAWA, Yuji, Muko-shi, Kyoto 617-0002 (JP); SHIRAISHI, Mitsuru, Amagasaki-shi, Hyogo 661-0002 (JP); SUGIHARA, Yoshihiro, Ikoma-shi, Nara 630-0111 (JP); BABA, Masanori, Kagoshima-shi, Kagoshima 891-0103 (JP)
(74) Representative: Lewin, John Harvey
(86) International application number: PCT/JP2003/004908
(87) International publication number: WO 2003/089004

(57) **Abstract**

It is intended to provide a novel drug which exhibits an excellent effect of preventing HIV infection in transfusing blood and using a blood preparation. This object can be achieved by a preventive for HIV infection in transfusing blood and using a blood prepartion characterized by containing a compound having an antagonism to a CC chemokine receptor (preferably antagonism to CCR5 and/or CCR2).

## Description

### Technical Field

This invention relates to a medicine preventing HIV infection during blood transfusion or when blood products are used, which contains a compound with a CC chemokine receptor (hereafter referred to as CCR) antagonistic effect.

### Background Art

HIV-1 uses CCR5 and/or CXCR4, which are one type of G-protein coupled receptors (GPCR), as coreceptors during infection with CD4 positive T cells (for example, see non-patent document 1, non-patent document 2, non-patent document 3, non-patent document 4, non-patent document 5, and non-patent document 6). It has been found that GPCRs other than CCR5 and CXCR4, such as CCR2 (for example, see non-patent document 7), CCR3 (for example, see non-patent document 5), APJ (for example, see non-patent document 8), Chem R23 (for example see non-patent document 9), GPR1, GPR15/BOB (for example, see non-patent document 10), and STRL33/BONZO (for example, see non-patent document 11) are also able to be used as a coreceptor. However, the relationship of coreceptors other than CCR5 and CXCR4 to an actual HIV infection is uncertain. HIV-1 is referred to by different names depending on the coreceptor used for it The HIV-1 which uses CCR5 as a coreceptor is referred to as R5 HIV-1, the HIV-1 which uses CXCR4 as a coreceptor is referred to as X4 HIV-1, and the HIV-1 which uses both of them is referred to as R5X4 HIV-1 (for example, see non-patent document 12).

It is known that the ability of HIV-1 to use a coreceptor is closely related to the condition of the HIV infection. That is, the first infection of HIV is caused by R5 HIV-1, R5 HIV-1 is primarily isolated during the symptomless period before the appearance of AIDS, and R5X4 HIV-1 and X4 HIV-1 gradually come to be significantly isolated as the AIDS period approaches (for example, see non-patent document 13, non-patent document 14, and non-patent document 15).

It has been reported that the number of HIV positive samples in HIV screening tests of blood donors in Japan in 2000 was 67 samples out of about 5.9 million blood donors. Amongst these positive samples, three samples were negative in the antibody test and positive in the gene test and thus in the initial stages of infection, and it is thought that these blood donations were made during the so-called window period (the period from infection with HIV to the time when antibodies are positively detected) (for example, see non-patent document 16). In addition, examples of infection in which infection due to blood transfusion cannot be denied have been reported from the antibody screening inspection has begun up until the present. These cases are thought to have been caused by transfusion during the window period (for example, see non-patent document 17). Although the 22-day window period with the antibody test was shortened to 11 days due to the introduction of the gene test, infections caused by transfusion cannot be entirely prevented by testing alone since there is still an 11-day window period. There are concerns about the increasing possibility of infections caused by transfusion during the window period in the future. In addition, for the same reason, it cannot be denied that blood products such as imported standard management serums and immunoglobulin preparations which are produced from plasma and serums pooled from many people may also become infection sources in the future.

HIV infection during transfusion and when blood products are used is caused by the fact that virus directly invades the blood and infects the CD4 positive lymphocytes. However in this case, the virus uses a CCR such as CCR5 as a coreceptor. Therefore, it is thought that a compound that can obstruct binding of viruses to coreceptors and the invasion of the virus to the host cells, for example, a compound having a CCR antagonistic effect, can prevent HIV infection during blood transfusion and when blood products are used. Existing medicines such as reverse transcriptase inhibitors and protease inhibitors cannot be expected to be useful for the prevention of infection since these act after the virus has invaded the host cells. Up until now, the present inventors have reported that TAK-779 having a CCR5 antagonistic effect exhibits an inhibitory effect against HIV-1 infection caused by R5 HIV-1 (for example, see non-patent document 18). In addition, polymorphism in the genes of the chemokine receptors of Japanese hemophiliacs that have had no occurrence of disease over a long period of time and were negative in HIV antibody test have been examined. It has been reported that a gene polymorphism in CCR2-64I/CCR5-59653T in such high risk factor groups not only takes part in the obstruction of the occurrence of AIDS but also takes part in the obstruction of HIV infection (for example, see non-patent document 19) It is suggested that CCR5 and/or CCR2 plays an important role in the establishment of HIV infection.

Meanwhile, various compounds having a CCR antagonistic effect are known (for example, see patent documents 1-9) However, a compound having a CCR antagonistic effect that prevent HIV infection during blood transfusion and when blood products are used has not been reported.

Prior art documents relating to the invention of the present application are as follows.
Non-patent document 1
   Science 272, 872-877 (1996)
Non-patent document 2:
   Nature 381, 661-666 (1996)
Non-patent document 3:
   Nature 381, 667-673 (1996)
Non-patent document 4:
   Science 272, 1955-1958 (1996)
Non-patent document 5:
   Cell 85, 1135-1148 (1996)
Non-patent document 6:
   Cell 85, 1149-1158 (1996)
Non-patent document 7:
   Cell 87, 437-446 (1996)
Non-patent document 8:
   J.Virol. 72, 6113-6118 (1998)
Non-patent document 9:
   Eur.J.Immunol. 28, 1689-1700 (1998)
Non-patent document 10:
   J.Exp.Med. 186, 405-411 (1997)
Non-patent document 11:
   J.Exp.Med. 185, 2015-2023 (1997)
Non-patent document 12:
   Nature 391, 240 (1998)
Non-patent document 13:
   J.Virol. 66, 1354-1360 (1992)
Non-patent document 14:
   J.Infect.Dis. 169, 968-974 (1994)
Non-patent document 15:
   J.Exp.Med. 185, 621-628 (1997)
Non-patent document 16:
   Sogo Rinsho 50, 2698-2703 (2001)
Non-patent document 17:
   Mebio 15, 6-11 (1999)
Non-patent document 18:
   Proc. Natl. Acad. Sci. USA, 96, 5698-5703(1999)
Non-patent document 19:
   The 15th Annual Congress of the Japanese Society for AIDS Research(December, 2001)
Patent publication 1:
   WO 99/32468
Patent publication 2:
   WO 99/32100
Patent publication 3:
   WO 00/10965
Patent publication 4:
   WO 00/37455
Patent publication 5:
   WO 00/68203
Patent publication 6:
   WO 00/76993
Patent publication 7:
   WO 00/66551
Patent publication 8:
   WO 01/25200
Patent publication 9:
   WO 01/25199

### Objects of the Invention

The present invention provides a new medicine that specifically demonstrates a remarkable effect in the prevention of HIV infection during blood transfusion and when blood products are used.

### Summary of the Invention

There is a possibility that HIV that contaminates blood that is donated or shared in the window period will not detected by screening tests. As a result, there is a possibility that HIV will infect the recipient during blood transfusion or when blood products are used. Thus, the present inventors focused on the possibility that a medicine having a CCR antagonistic effect would be able to work as an HIV infection preventative, and as a result of extensive studies, found that a medicine having a CCR5 antagonistic effect is useful for the prevention of HIV infection, and completed the present invention.

That is, the present invention relates to:
(1) An agent for preventing HIV infection in transfuing blood or using a blood product (blood preparation), which comprises a compound having a CCR antagonistic effect;
(2) An agent as shown in the above (1), wherein CCR is CCR5 and/or CCR2;
(3) An agent as shown in the above (1), wherein the compound having a CCR antagonistic effect is N-(3,4-dichlorophenyl)-1-(methylsulfonyl)-N-{3-[4-({4-[(methylsulfonyl)amino]phenyl}sulfonyl)-1-piperidinyl]propyl}-4-piperidinecarboxamide, N-(3-chlorophenyl)-1-(methylsulfonyl)-N-(3-{4-[4-(methylsulfonyl)benzyl]-1-piperidinyl}propyl)-4-piperidinecarboxamide, N-(3-{4-[4-(aminocarbonyl)benzyl]-1-piperidinyl}propyl)-N-(3,4-dichlorophenyl)-1-(methylsulfonyl)-4-piperidinecarboxamide, 1-acetyl-N-(3-{4-[4-(aminocarbonyl)benzyl]-1-piperidinyl}propyl)-N-(3-chloro-4-methylphenyl)-4-piperidinecarboxamide, N-(3,4-dichlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-1-(methylsulfonyl)-N-(3-{4-[4-(methylsulfonyl)benzyl]-1-piperidinyl}propyl)-4-piperidinecarboxamide, N-(3-{4-[4-(aminocarbonyl)benzyl]-1-piperidinyl}propyl)-N-(3-chloro-4-methylphenyl)-1-(methylsulfonyl)-4-piperidinecarboxamide, N-[3-(4-benzyl-1-piperidinyl)propyl]-N'-(4-chlorophenyl)-N-phenylurea, N'-(4-chlorophenyl)-N-{3-[4-(4-fluorobenzyl)-1-piperidinyl]propyl}-N-phenylurea, N'-(4-chlorophenyl)-N-(3-{4-[4-(4-morpholinylsulfonyl)benzyl]-1-piperidinyl}propyl)-N-phenylurea, N'-(4-chlorophenyl)-N-(3-{4-[4-(4-methylsulfonyl)benzyl]-1-piperidinyl}propyl)-N-phenylurea, 4-{[1-(3-1[(4-chloroanilino)carbonyllanilino}propyl)-4-piperidinyl]methyl}benzamide, N-[3-(4-benzyl-1-piperidinyl)propyl]-N-(3,4-dichlorophenyl)-1-methyl-5-oxo-3-pyrrolidinecarboxamide, 1-benzyl-N-[3-(4-benzyl-1-piperidinyl)propyl]-5-oxo-N-phenyl-3-pyrrolidinecarboxamide, N-[3-(4-benzyl-1-piperidinyl)propyl]-1-(2-chlorobenzyl)-5-oxo-N-phenyl-3-pyrrolidinecarboxamide, N-(3,4-dichlorophenyl)-N-{3-[4-(4-fluorobenzyl)-1-piperidinyl]propyl}-1-methyl-5-oxo-3-pyrrolidinecarboxamide and N-[3-(4-benzyl-1-piperidinyl)propyl]-5-oxo-N-phenyl-1-(2,2,2-trifluoroethyl)-3-pyrrolidinecarboxamide, N-(3,4-dichlorophenyl)-N-(3-{4-[4-(methylsulfonyl)benzyl]-1-piperidinyl}propyl)-2-[1-(methylsulfonyl)-4-piperidinyl]acetamide, N-(3,4-dichlorophenyl)-N-(3-{4-[4-(isopropylsulfonyl)benzyl]-1-piperidinyl}propyl)-2-[1-(methylsulfonyl)-4-piperidinyl]acetamide, 3-(1-acetyl-4-piperidinyl)-N-(3,4-dichlorophenyl)-N-(3-{4-[4-(methylsulfonyl)benzyl]-1-piperidinyl}propyl)propanamide, N-(3,4-dichlorophenyl)-4-hydroxy-1-(methylsulfonyl)-N-(3-{4-[4-(methylsulfonyl)benzyl]-1-piperidinyl}propyl)-4-piperidinecarboxamide or a salt thereof;
(4) A preventing agent as shown in the above (1), wherein the compound having a CCR antagonistic effect is N-methyl-N-[4-[[[2-(4-methylphenyl)-6,7-dihydro-5H-benzocyclohepten-8-yl]carbonyl]amino]benzyl]piperidinium iodide, N-methyl-N-[4-[[[7-(4-methylphenyl)-2,3-dihydro-1-benzoxepin-4-yl]carbonyl]amino]benzyl]piperidinium iodide, N-[4-[N-methyl-N-(tetrahydropyran-4-yl)aminomethyl]phenyl]-7-(4-methylphenyl)-2,3-dihydro-1-benzoxepin-4-carboxamide, N-[4-[N-methyl-N-(tetrahydropyran-4-yl)aminomethyl]phenyl]-7-(4-morpholinophenyl)-2,3-dihydro-1-benzoxepin-4-carboxamide, 7-(4-ethoxyphenyl)-N-[4-[N-methyl-N-(tetrahydropyran-4-yl)aminomethyl]phenyl]-2,3-dihydro-1-benzoxepin-4-carboxamide, N,N-dimethyl-N-[4-[[[2-(4-methylphenyl)-6,7-dihydro-5H-benzocyclohepten-8-yl]carbonyl]amino]benzyl]-N-(tetrahydropyran-4-yl)ammonium iodide, N-methyl-N-[4-[[[7-(4-methylphenyl)-3,4-dihydronaphthalen-2-yl]carbonyl]amino]benzyl]piperidinium iodide, N,N-dimethyl-N-(4-(((2-(4-methylphenyl)-6,7-dihydro-5H-benzocyclohepten-8-yl)carbonyl)amino)benzyl)-N-(4-tetrahydropyranyl)ammonium chloride, N,N-dimethyl-N-(((7-(4-methylphenyl)-2,3-dihydro-1-benzoxepin-4-yl)carbonyl)amino)benzyl)-N-(4-oxocyclohexyl)ammonium chloride, N-(4-(((7-(4-ethoxyphenyl)-2,3-dihydro-1-benzoxepin-4-yl)carbonyl)amino)benzyl)-N,N-dimethyl-N-(4-tetrahydropyranyl)ammonium chloride, N-[4-[N-methyl-N-(tetrahydropyran-4-yl)aminomethyl]phenyl]-7-(4-propoxyphenyl)-1,1-dioxo-2,3-dihydro-1-benzothiepin-4-carboxamide, 7-(4-butoxyphenyl)-N-[4-[N-methyl-N-(tetrahydropyran-4-yl)aminomethyl]phenyl]-1,1-dioxo-2,3-dihydro-1-benzothiepin-4-carboxamide, 7-[4-[N-methyl-N-(2-propoxyethyl)amino]phenyl]-N-[4-[[N-methyl-N-(tetrahydropyran-4-yl)amino]methyl]phenyl]-1,1-dioxo-2,3-dihydro-1-benzothiepin-4-carboxamide, 7-[4-(2-ethoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, N-[4-[[N-methyl-N-(tetrahydropyran-4-yl)amino]methyl]phenyl]-7-[4-(2-propoxyethoxy)phenyl]-1,1-dioxo-2,3-dihydro-1-benzothiepin-4-carboxamide, 7-[4-(2-butoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, 7-[4-(2-ethoxyethoxy)-3,5-dimethylphenyl]-N-[4-[[N-methyl-N-(tetrahydro-2H-pyran-4-yl)amino]methyl]phenyl]-1,1-dioxo-2,3-dihydro-1-benzothiepin-4-carboxamide, 7-[2-chloro-4-(2-propoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, 7-(3-methyl-4-propoxyphenyl)-N-[4-[[N-methyl-N-(tetrahydropyran-4-yl)amino]methyl]phenyl]-1,1-dioxo-2,3-dihydro-1-benzothiepin-4-carboxamide, 7-(3,4-dipropoxyphenyl)-N-(4-((N-methyl-N-(tetrahydro-2H-pyran-4-yl)amino)methyl)phenyl)-1,1-dioxo-2,3-dihydro-1-benzothiepin-4-carboxamide, 7-[4-(2-ethoxyethoxy)phenyl]-1-ethyl-N-[4-[[N-methyl-N-(tetrahydropyran-4-yl)amino]methyl]phenyl]-2,3-dihydro-1-benzoazepin-4-carboxamide, 1-ethyl-7-[4-(2-propoxyethoxy)phenyl]-N-[4-[[N-methyl-N-(tetrahydropyran-4-yl)amino]methyl]phenyl)-2,3-dihydro-1-benzoazepin-4-carboxamide, 7-[4-(2-butoxyethoxy)phenyl]-1-ethyl-N-[4-[[N-methyl-N-(tetrahydropyran-4-yl)amino]methyl]phenyl]-2,3-dihydro-1-benzoazepin-4-carboxamide, 7-[4-(2-ethoxyethoxy)phenyl]-1-formyl-N-[4-[[N-methyl-N-(tetrahydropyran-4-yl)amino]methyl]phenyl]-2,3-dihydro-1-benzoazepin-4-carboxamide, 1-formyl-7-[4-(2-propoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-formyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-5-yl)amino]methyl]phenyl]-1-propyl-2,3-dihydro-1-benzoazepin-4-carboxamide, N-[4-[[N-methyl-N-(tetrahydropyran-5-yl)amino]methyl]phenyl]-7-[4-(2-propoxyethoxy)phenyl]-1-propyl-2,3-dihydro-1-benzoazepin-4-carboxamide, 1-benzyl-7-[4-(2-butoxyethoxy)phenyl]-N-[4-[[N-methyl-N-(tetrahydropyran-4-yl)amino]methyl]phenyl)-2,3-dihydro-1-benzoazepin-4-carboxamide, 7-[4-(2-butoxyethoxy)phenyl]-1-cyclopropylmethyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-phenyl-2,3-dihydro-1-benzoazepin-4-carboxamide, 7-[4-(2-butoxyethoxy)phenyl]-1-(3,4-methylenedioxy)phenyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(2-methyloxazol-5-yl)-N-[4-[[N-methyl-N-(tetrahydropyran-4-yl)amino]methyl]phenyl]-2,3-dihydro-1-benzoazepin-4-carboxamide, 1-allyl-7-[4-(2-butoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(3-thienyl)methyl-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(thiazol-2-yl)methyl-2,3-dihydro-1-benzoazepin-4-carboxamide, 7-[4-(2-butoxyethoxy)phenyl]-1-(1-methylpyrazol-4-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(3-methylisothiazol-5-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(1-ethylpyrazol-4-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-isobutyl-N-[4-[[N-methyl-N-(tetrahydropyran-5-yl)amino]methyl]phenyl]-2,3-dihydro-1-benzoazepin-4-carboxamide, 1-isobutyl-N-[4-[[N-methyl-N-(tetrahydropyran-5-yl)amino]methyl]phenyl]- 7-[4-(2-propoxyethoxy)phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(thiazol-5-yl)methyl-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(1-methyltetrazol-5-yl)methyl-2,3-dihydro-1-benzoazepin-4-carboxamide, or 7-[4-(2-butoxyethoxy)phenyl]-N-[4-[[N-methyl-N-(tetrahydropyran-4-yl)amino]methyl]phenyl]-1-(2-methyltetrazol-5-yl)methyl-2,3-dihydro-1-benzoazepin-4-carboxamide or a salt thereof;
(5) Blood for transfusion or blood product, which contains a compound having a CCR antagonistic effect;
(6) A method for preventing HIV infection, which comprises administering blood for transfusion or a blood product which contains a compound having a CCR antagonistic effect;
(7) A method for preventing HIV infection, which comprises administering an effective amount of a compound having a CCR antagonistic effect at the time of blood transfusion or blood product use;
(8) A method as shown in the above (7), wherein the time of blood transfusion or blood product use is from one hour before blood transfusion or blood product use to the time of blood transfusion or blood product use;
and the like.

### Brief Description of the Drawing

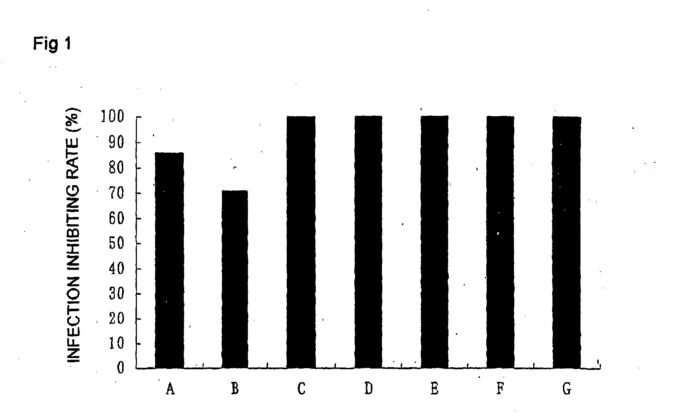

Figure 1 is a graph showing the HIV-1 infection inhibition rate of a compound used in the experimental examples.

### Detailed Description of the Invention

Among the compounds having a CCR antagonistic effect used in the present invention, compounds having a CCR5 and/or a CCR2 antagonistic effect are particularly preferred, especially the compounds having a CCR5 antagonistic effect. That is, compounds having an antagonistic effect on one receptor from amongst the CCR5 and CCR2 receptors or both the receptors are preferable.

For example, anilide compounds shown in WO99/32468, WO99/32100 and WO00/68203 are mentioned as the compounds having CCR2 antagonistic effect. For example, piperidine derivatives (WO00/66551, WO01/25199, WO01/25200, Japanese patent application No. 2000-328851) and anilide derivatives having a quarternal ammonium moiety (for example, compounds shown in non-patent document 18 mentioned above and compounds shown in J. Med. Chem. 2000, 43, 2049-2063) other than the above anilide compounds are mentioned as the compound having a CCRS antagonistic effect.

More specifically, the following compounds are mentioned.
(1) a compound having a CCR5 antagonistic effect which is represented by the formula: 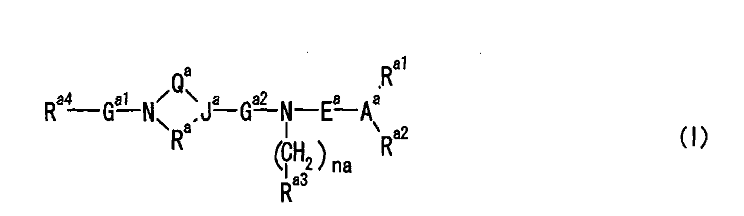 (wherein R^{a1} is a hydrogen atom, a hydrocarbon group which maybe substituted, a non-aromatic heterocyclic group which may be substituted, R^{a2} is a hydrocarbon group which may be substituted, a non-aromatic heterocyclic group which may be substituted, or R^{a1} and R^{a2} may combine to each other together with A^{a}, to form a heterocyclic group which may be substituted; A^{a} is N or N⁺-R^{a5} · Y^{a-} (R^{a5} is a hydrocarbon group; Y^{a-} is a counter anion); R^{a3} is a cyclic hydrocarbon group which may be substituted or a heterocyclic group which may be substituted; na is 0 or 1; R^{a4} is a hydrogen atom, a hydrocarbon group which may be substituted, a heterocyclic group which may be substituted, an alkoxy group which may be substituted, an aryloxy group which may be substituted, or an amino group which may be substituted, E^{a} is a divalent aliphatic hydrocarbon group which may be substituted by a group other than an oxo group; G^{a1} is a bond, CO or SO₂; G^{a2} is CO, SO₂, NHCO, CONH or OCO; J^{a} is methine or a nitrogen atom; and each of Q^{a} and R^{a} is a bond or a divalent C₁₋₃ aliphatic hydrocarbon which may be substituted; provided that J^{a} is methine when G^{a2} is OCO, that one of Q^{a} and R^{a} is not a bond when the other is a bond and that each of Q^{a} and R^{a} is not substituted by an oxo group when G^{a1} is a bond) or a salt thereof.
(2) a compound of the formula: 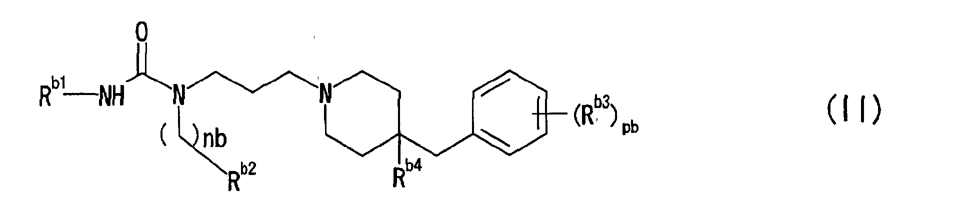 [wherein R^{b1} is a hydrocarbon group which may be substituted;
   R^{b2} is a cyclic hydrocarbon group which may be substituted or a heterocyclic group which may be substituted;
   R^{b3} is a halogen atom, a carbamoyl group which may be substituted, a sulfamoyl group which may be substituted, an acyl group derived from a sulfonic acid, a C₁₋₄ alkyl group which may be substituted, a C₁₋₄ alkoxy group which may be substituted, an amino group which may be substituted, a nitro group or a cyano group;
   R^{b4} is a hydrogen atom or a hydroxy group;
   nb is an integer of 0 or 1;
   pb is an integer of 0 or 1 to 4];
   or a salt thereof,
(3) a compound of the formula: 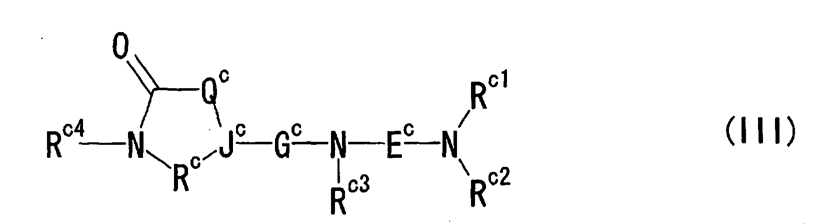 wherein R^{c1} is a hydrocarbon group, R^{c2} is a hydrocarbon group having 2 or more carbon atoms, or R^{c1} and R^{c2} may join to form, together with an adjacent nitrogen atom, a ring optionally having a substituent or substituents, R^{c3} is a hydrocarbon group optionally having a substituent or substituents or a heterocyclic group optionally having a substituent or substituents, R^{c4} is a hydrogen atom, a hydrocarbon group optionally having a substituent or substituents or a heterocyclic group optionally having a substituent or substituents, E^{c} is a divalent chain hydrocarbon group optionally having a substituent or substituents other than an oxo group, G^{c} is CO or SO₂, J^{c} is a nitrogen atom or a methine group optionally having a substituent or substituents, and Q^{c} and R^{c} are each a bond or a divalent chain C₁₋₃ hydrocarbon group optionally having a substituent or substituents, or a salt thereof;
(4) a compound of the formula: 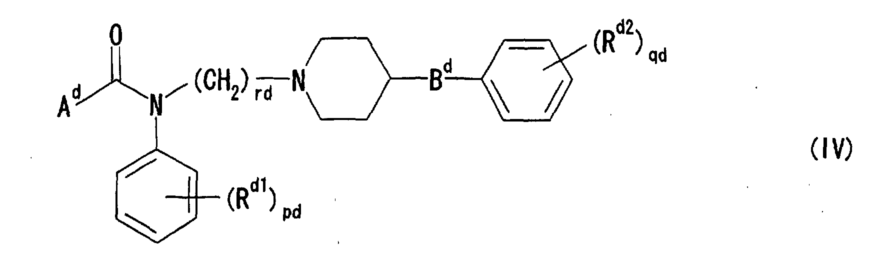 wherein A^{d} is a group represented by the formula: 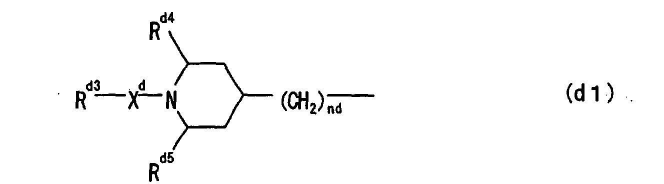 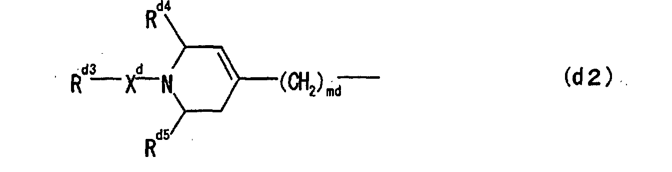 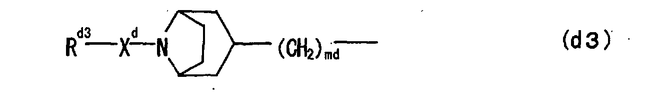 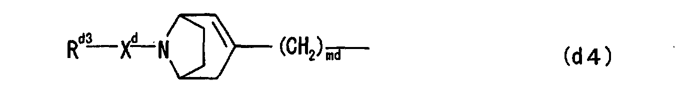 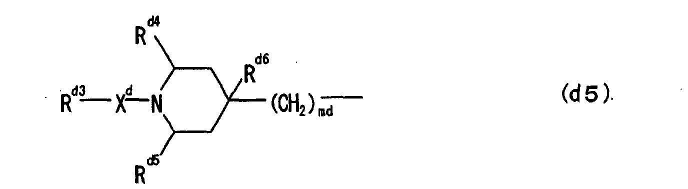 or 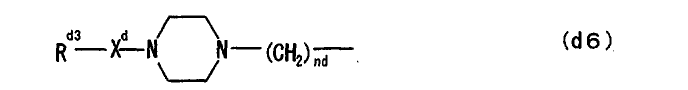 wherein R^{d3} is (1) a hydrocarbon group which may be substituted, (2) a C₁₋₄ alkoxy group which may be substituted or (3) an amino group which may be substituted; X^{d} is a bond, -SO₂- or -CO-; nd is an integer of 1 to 3; md is 0 or an integer of 1 to 3; R^{d4} and R^{d5} are the same or different and each of which is a hydrogen atom or a C₁₋₆ alkyl group; R^{d6} is a hydroxyl group, a C₁₋₆ alkyl group or a C₂₋₆ alkenyl group; rd is an integer of 2 to 4; B^{d} is a bond, -CH₂-, -SO₂-, -SO-, -S-, -O-, -CO-, -N^{da}-SO₂- or -NR^{da}-CO- (wherein R^{da} is a hydrogen atom, a C₁₋₆ alkyl group, a C₂₋₆ alkenyl group or a C₃₋₈ cycloalkyl group); each of pd and qd is 0 or an integer of 1 to 4; R^{d1} is a halogen atom, a C₁₋₆ alkyl group, a C₂₋ alkenyl group, a C₁₋₄ alkanoyl group, a C₁₋₄ alkoxy group, a cyano group, a trifloromethyl group, a nitro group, a hydroxyl group, an amino group or an amidino group; R^{d2} is 1) a halogen, 2) a C₁₋₆ alkyl which may be substituted by a halogen or a C₁₋₄ alkoxy, 3) a C₁₋₄ alkoxy which may be substituted by a halogen or a C₁₋₄ alkoxy, 4) nitro, 5) cyano, 6) hydroxyl, 7) a C₁₋₄ alkanoylamino, 8) SO₂NR^{db}R^{dc}, 9) SO₂R^{dd}, 10) CONR^{db}R^{dc}, 11) NR^{db}R^{dc} or 12) NR^{da}-SO₂R^{dd} {wherein R^{da} has the meaning given above, and R^{db} and R^{dc} may be the same or different, and each of which (1) a hydrogen, (2) a C₁₋₆ alkyl group which may be substituted by a halogen or a C₁₋₄ alkoxy or (3) a C₃₋₈ cycloalkyl group which may be substituted by a halogen or a C₁₋₄ alkoxy, or R^{db} and R^{dc} may, taken together with nitrogen atom form a cyclic amino group; R^{dd} is a C₁₋₆ alkyl group or a C₃₋₈ cycloalkyl group} each R^{d1}, may be the same or different from each other when pd is two or more, and each R^{d2}, may be the same or different from each other when qd is two or more; or a salt thereof.
(5). a compound represented by formula: 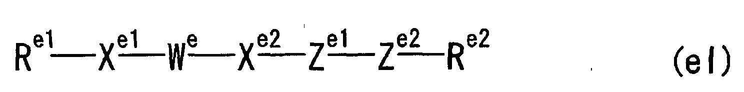 [wherein R^{e1} represents a 5 to 6-membered cyclic ring group which may be substituted, X ^{e1} represents a bond or a bivalent group, in which the number of atoms constituting the straight-chain portion is 1 to 4, W^{e} represents a bivalent group represented by formula: 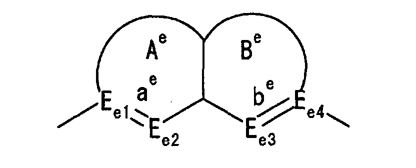 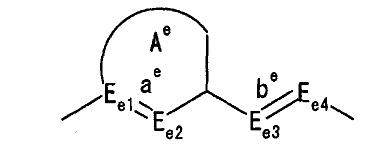 or 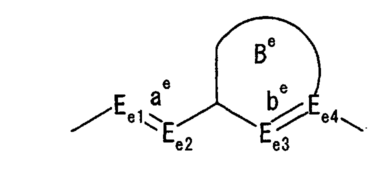 (wherein each of ring A^{e} and ring B^{e} represents a 5- to 7-membered ring group which may be substituted, each of Ee₁ and Ee₄ is the carbon atom which may be substituted or a nitrogen atom which may be substituted, each of Ee₂ and Ee₃ is the carbon atom which may be substituted, the nitrogen atom which may be substituted, a sulfur which may be oxidized or an oxygen atom and each of a^{e} and b^{e} is a single bond or a double bond), X^{e2} is a bivalent group, in which the number of atoms constituting the straight-chain portion is 1 to 4, Z^{e1} is a bond or a bivalent cyclic ring group, Z^{e2} is a bond or a bivalent cyclic ring group, in which the number of atoms constituting the straight-chain portion is 1 to 4, and R^{e2} is (1) an amino group which may be substituted, where the nitrogen atom may be converted into a quaternary ammonium or the N-oxide, (2) a nitrogen-containing heterocyclic ring group which may be substituted and may contain sulfur atom or an oxygen atom as a ring-constituting atom, where the nitrogen atom may be converted into a quaternary ammonium or a N-oxide, (3) a group which is bonded via the sulfur atom, (4) a group represented by formula 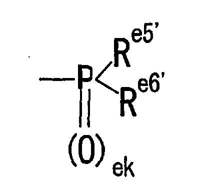 (wherein ek is 0 or 1, the phosphorus atom may form a phosphonium salt when k is 0, and each of R^{e5'} and R^{e6'} is a hydrocarbon atom which may be substituted, a hydroxyl group which may be substituted or an amino group which may be substituted and R^{e5'} and R^{e6'} may bind each other to form a cyclic ring group together with the adjacent phosphorus atom), (5) an amidino group which may be substituted or (6) a guanidino group which may be substituted].

As the compound of the above formula (eI) or a salt thereof, the following compounds are mentioned.
1) a compound of the formula: 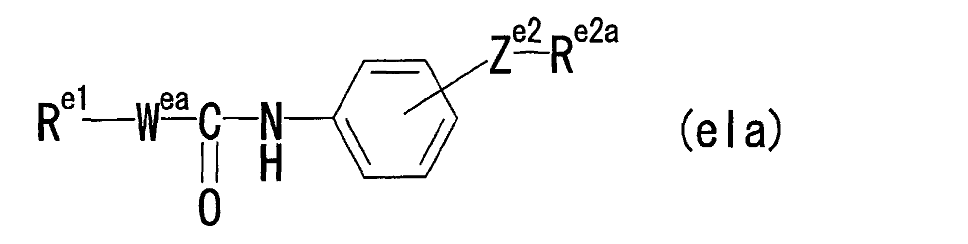 wherein R^{e1} is an optionally substituted 5- to 6-membered ring, W^{ea} is a divalent group represented by the formula: 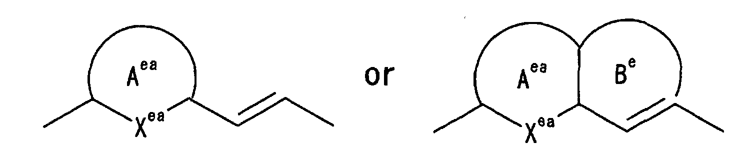 wherein the ring A^{ea} is an optionally substituted 5- to 6-membered aromatic ring, X^{ea} is an optionally substituted carbon atom, an optionally substituted nitrogen atom, a sulfur atom or an oxygen atom, the ring B^{e} is an optionally substituted 5- to 7-membered ring; Z^{e2} is a bond or a divalent group in which the number of carbon atoms constituting the straight-chain portion is 1 to 4, R^{e2a} is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium, (3) a group binding through a sulfur atom or (4) a group of the formula: 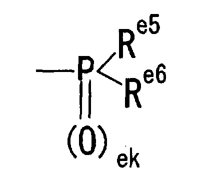 wherein ek is 0 or 1, and when ek is 0, a phosphorus atom may form a phosphonium; and R^{e5} and R^{e6} are independently an optionally substituted hydrocarbon group or an optionally substituted amino group, and R^{e5} and R^{e6} may bind to each other to form a cyclic group together with the adjacent phosphorus atom, or a salt thereof,
2) a compound of the formula: 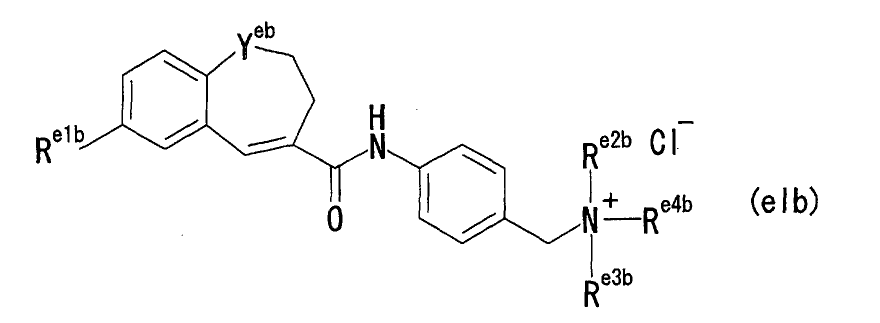 wherein R^{e1b} is an optionally substituted phenyl group or an optionally substituted thienyl group; Y^{eb} is -CH₂-, -O- or -S-; and R^{e2b}, R^{e3b} and R^{e4b} are independently an optionally substituted aliphatic hydrocarbon group or an optionally substituted alicyclic heterocyclic ring group; or a salt thereof,
3) a compound of the formula: 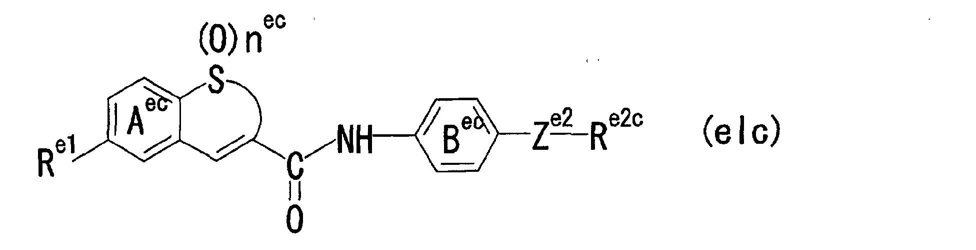 wherein R^{e1} is an optionally substituted 5- to 6-membered ring; the ring A^{ec} is an optionally substituted 6- to 7-membered ring; the ring B^{ec} is an optionally substituted benzene ring; n^{ec} is an integer of 1 or 2; Z^{e2} is a bond or a divalent group in which the number of carbon atoms constituting the straight -chain is 1 to 4; R^{e2c} is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium, (3) a group binding through a sulfur atom or (4) a group of the formula: 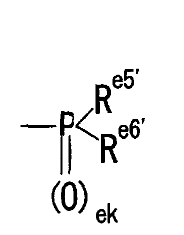 wherein ek is 0 or 1, and when ek is 0, a phosphorus atom may form a phosphonium; and R^{e5'} and R^{e6}' are independently an optionally substituted hydrocarbon group, an optionally substituted hydroxy group or an optionally substituted amino group, and R^{e5}' and R^{e6}' may bind to each other to form a cyclic group together with the adjacent phosphorus atom, or a salt thereof;
4) a compound of the formula: 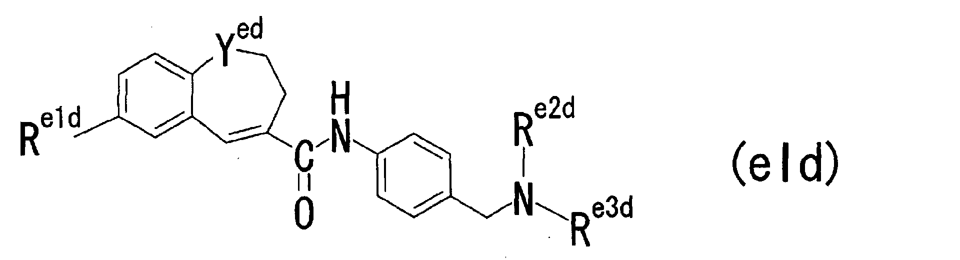
wherein R^{e1d} is a 5- to 6- membered aromatic ring which has a group of the formula: R^{ed}-Z^{e1d}-X^{ed}-Z^{e2d}- wherein R^{ed} is a hydrogen atom or an optionally substituted hydrocarbon group, X^{ed} is an optionally substituted alkylene chain, and Z^{e1d} and Z ^{e2d} are respectively hetero-atoms, and which may have a further substituent, the group R^{ed} may bind to the 5-6 membered aromatic ring to form a ring, Y^{ed} is an optionally substituted imino group, R^{e2d} and R^{e3d} are respectively an optionally substituted aliphatic hydrocarbon group or an optionally substituted alicyclic heterocyclic group; or a salt thereof.

In the formula (I), the "hydroxarbon group" in the "hydrocarbon group which may be substituted" represented by R^{a1} includes e.g. an aliphatic chain hydrocarbon group, an alicyclic hydrocarbon group and an aryl group, preferably, an aliphatic chain hydrocarbon group and an alicyclic hydrocarbon group.

Examples of the "aliphatic hydrocarbon group" include e.g. a straight-chain or branched aliphatic hydrocarbon group such as an alkyl group, an alkenyl group, an alkynyl group, etc., preferably an alkyl group. Examples of the alkyl group include e.g. a C₁₋₁₀ alkyl group (preferably a C₁₋₆ alkyl, etc.) such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, 1-methylheptyl, 1-ethylhexyl, n-octyl, 1-methyl-heptyl, nonyl, etc. Examples of the alkenyl group include e.g. a C₂₋₆ alkenyl group such as vinyl, allyl, isopropenyl, 2-methyl-allyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc. Examples of the alkynyl group include e.g. a C₂₋₆ alkynyl group such as ethynyl, 1-propynyl, 2-propynyl, 1-butynyl, 2-butynyl, 3-butynyl, 1-pentynyl, 2-pentynyl, 3-pentynyl, 9-pentynyl, 1-hexynyl, 2-hexynyl, 3-hexynyl, 4-hexynyl, 5-hexynyl, etc.

Examples of the "alicyclic hydrocarbon group" include e.g. a saturated or unsaturated alicyclic hydrocarbon group such as a cycloalkyl group, a cycloalkenyl group, a cycloalkanedienyl group, etc., preferably cycloalkyl group. Examples of the "cycloalkyl group" include e.g. a C₃₋₉ cycloalkyl (preferably a C₃₋₈ cycloalkyl) such as cyclopropyl, cyclobutyl, cyclopentyl, cyclohexyl, cycloheptyl, cyclooctyl, cyclononyl, etc., and a fused ring such as 1-indanyl, 2-indanyl, etc. Examples of the "cycloalkenyl group" include e.g. a C₃₋₆ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl, etc. Examples of the "cycloalkanedienyl group" include e.g. a C₄₋₆ cycloalkanedienyl group such as 2,4-cyclopentadien-1-yl, 2,4-cyclohexadien-1-yl, 2,5-cyclohexadien-1-yl, etc.

Examples of the "aryl group" include e.g. a monocyclic or fused polycyclic aromatic hydrocarbon group. Among others, a C₆₋₁₄ aryl group such as phenyl, naphthyl, anthryl, phenathryl, acenaphthylenyl, 4-indanyl, 5-indanyl, etc. are preferable. In particular, phenyl, 1-naphthyl, 2-naphthyl, etc. are preferable.

Examples of the "non-aromatic heterocyclic group" in the "optionally substituted non-aromatic heterocyclic group" represented by R^{a1} include a 3- to 8-membered (preferably 5- or 6-membered) saturated or unsaturated (preferably saturated) non-aromatic heterocyclic group (alicyclic heterocyclic group) such as oxiranyl, azetidinyl, oxetanyl, thiethanyl, pyrrolidinyl, tetrahydrofuryl, thiolanyl, piperidinyl, tetrahydropyranyl, morpholinyl, thiomorpholinyl, piperazinyl, etc.

Examples of the substituent of the "optionally substituted hydrocarbon group" and "optionally substituted non-aromatic heterocyclic group" represented by R^{a1} include an optionally substituted alkyl group, an optionally substituted alkenyl group, an optionally substituted alkynyl group, an optionally substituted aryl group, an optionally substituted cycloalkyl or cycloalkenyl group, an optionally substituted heterocyclic group, an optionally substituted amino group, an optionally substituted imidoyl group, an optionally substituted amidino group, an optionally substituted hydroxyl group, an optionally substituted thiol group, an optionally esterified carboxyl group, an optionally substituted carbamoyl group, an optionally substituted thiocarbamoyl group, an optionally substituted sulfamoyl group, a halogen atom (e.g. fluorine, chlorine, bromine, iodine, etc., preferably chlorine, bromine, etc.), a cyano group, a nitro group, an acyl group derived from a sulfonic acid, an acyl group derived from an carboxylic acid, an optionally substituted alkyl-sulfinyl group, an optionally substituted aryl-sulfinyl group, etc. The "optionally substituted hydrocarbon group" and "optionally substituted non-aromatic heterocyclic group" may have 1 to 5 substituents as described above (preferably 1 to 3 substituents) at any possible position.

Examples of the aryl group in the "optionally substituted aryl group" as the substituent include a C₆₋₁₄ aryl group such as phenyl, naphthyl, anthryl, phenathryl, acenaphthylenyl, etc. Examples of the substituent of the aryl group include a lower alkoxy group which may be substituted by halogen (e.g. a C₁₋₆ alkoxy group such as methoxy, ethoxy, propoxy, etc., a C₁₋₄ alkoxy group substituted by halogen such as fluoromethoxy, difluoromethoxy, trifluoromethoxy, 1,1-difluoroethoxy, 2,2-difluoroethoxy, 3,3-difluoropropoxy, 2,2,3,3,3-pentafluoropropoxy, etc.), an aryloxy which may be substituted (e.g., phenoxy, 4-fluorophenoxy, 2-carbamoylphenoxy, etc.), a halogen atom (e.g., fluorine, chlorine, bromine, iodine, etc.), a lower alkyl group which may be substituted (an unsubstituted C₁₋₆ alkyl group such as methyl, ethyl, propyl, etc., a C₁₋₄ alkyl group substituted by halogen such as fluoromethyl, difluoromethyl, trifluoromethyl, 1,1-difluoroethyl, 2,2-difluoroethyl, 3,3-difluoropropyl, 2,2,3,3,3-pentafluoropropyl, etc.), a C₃₋₈ cycloalkyl (e.g., cyclopropyl, cyclobutyl, cyclopentyl, cyclohexyl, cycloheptyl, etc.), an amino group, a mono-substituted amino (e.g., carbamoylamino, methylsulfonylamino, methylamino, ethylamino, propylamino, etc.), di-substituted amino (e.g., dimethylamino, diethylamino, N-methyl-N-methylsulfonylamino, di(methylsulfonyl)amino, etc.), a carbamoyl group which may be substituted by a C₁₋₆ alkyl (e.g., butylcarbamoyl, etc.), formyl, a C₂₋₆ alkanoyl group (e.g., a C₂₋₆ alkanoyl such as acetyl, propionyl, butyryl, etc.), a C₆₋₁₄ aryl group (e.g., phenyl, naphthyl, etc.), a C₆-₁₄ aryl carbonyl (e.g., benzoyl, naphthoyl, etc.), a C₇₋₁₃ aralkyl carbonyl (e.g., benzylcarbonyl, naphthylmethylcarbonyl, etc.), a hydroxyl group, an alkanoyloxy (a C₂₋₅ alkanoyloxy such as acetyloxy, propionyloxy, butyryloxy, etc.), a C₇₋₁₃ aralkyl-carbonyloxy (e.g., benzylcarbonyloxy, etc.), a nitro group, a sulfamoyl group which may be substituted (e.g., unsubstituted sulfamoyl group, N-methylsulfamoyl, etc.), an arylthio group which may be substituted (e.g., phenylthio, 4-methylphenylthio, etc.), -N=N-phenyl, a cyano group, an amidino group, a carboxyl group which may be esterified (free carboxyl group, and a C₁₋₄ alkoxy carbonyl such as methoxycarbonyl, ethoxycarbonyl, t-butoxycarbonyl, etc., etc.), a C₁₋₆ alkylthio, a C₁₋₆ alkylsulfinyl, a C₁₋₆ alkylsulfonyl, a C₆₋₁₄ arylthio, a C₆₋₁₄ arylsulfinyl, a C₆₋₁₄ arylsulfonyl, a heterocyclic group which may be substituted (e.g., pyridyl, thienyl, tetrazolyl, morpholinyl, oxazolyl, etc. and those as mentioned below for the definition of heterocyclic group which may be substituted shown as R^{a3}), etc. One or two of these substituents may be present at any substitutable position.

Examples of the cycloalkyl group in the "optionally substituted cycloalkyl group" as the substituent include e.g.a C₃₋₇ cycloalkyl group such as cyclopropyl, cyclobutyl, cyclopentyl, cyclohexyl, cycloheptyl, etc. Examples of the substitutent of said cycloalkyl groups may have the same number and kind of substituents as those of the above described "optionally substituted aryl group".

Examples of the cycloalkenyl group in the "optionally substituted cycloalkenyl group" as the substituent include e.g. a C₃₋₆ cycloalkenyl group such as cyclopropenyl, cyclobutenyl, cyclopentenyl, cyclohexenyl, etc. Example of the substitutent of the cycloalkenyl groups which may be substituted may have the same number and kind of substituents as those of the above described "optionally substituted aryl group".

Examples of the alkyl group in the "optionally substituted alkyl group" as the substituent include e.g. a C₁₋₆ alkyl etc. such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, etc. Examples of the susbstitutent of said alkyl groups may have the same number and kind of substituents as those of the above described "optionally substituted aryl group".

Examples of the alkenyl group in the "optionally substituted alkenyl group" as the substituent include e.g. a C₂₋₆ alkenyl group such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc. Examples of the substitutents of said alkenyl groups may have the same number and kind of substituents as those of the above described "optionally substituted aryl group".

Examples of the alkynyl group in the "optionally substituted alkynyl group" as the substituent include e.g. a C₂₋₆ alkynyl group such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, etc. Examples of the substituent of said alkynyl groups may have the same kind and number of substituents as those of the above described "optionally substituted aryl group".

Examples of the heterocyclic group in the "optionally substituted heterocyclic group" as the substituent include e.g. an aromatic heterocyclic group, saturated or unsaturated non-aromatic heterocyclic group (alicyclic heterocyclic group) etc., which contains at least one hetero-atom (preferably 1 to 4 hetero-atoms, more preferably 1 to 2 hetero-atoms) consisting of 1 to 3 kinds of hetero-atoms (preferably 1 to 2 kinds of hetero-atoms) selected from an oxygen atom, a sulfur atom, a nitrogen atom, etc.

Examples of the "aromatic heterocyclic group" include an aromatic monocyclic heterocyclic group such as a 5- to 6-membered aromatic monocyclic heterocyclic group (e.g. furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.); an aromatic fused heterocyclic group such as a 8- to 12-membered aromatic fused heterocyclic group (e.g. benzofuranyl, isobenzofuranyl, benzothienyl, indolyl, isoindolyl, 1H-indazolyl, benzindazolyl, benzoxazolyl, 1,2-benzoisooxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.); etc., preferably, a heterocyclic group consisting of the above-mentioned 5- or 6-membered aromatic monocyclic heterocyclic group fused with a benzene ring or a heterocyclic group consisting of the above-mentioned 5- or 6-membered aromatic monocyclic heterocyclic group fused with the same or different above-mentioned 5- or 6-membered aromatic monocyclic heterocyclic group, etc.

Examples of the "non-aromatic heterocyclic group" include e.g. a 3- to 8-membered (preferably 5- or 6-membered) saturated or unsaturated (preferably saturated) non-aromatic heterocyclic group (alicyclic heterocyclic group) such as oxiranyl, azetidinyl, oxetanyl, thiethanyl, pyrrolidinyl, tetrahydrofuryl, thiolanyl, piperidinyl, tetrahydropyranyl, morpholinyl, thiomorpholinyl, piperazinyl, etc.

Examples of the substituent of the "optionally substituted heterocyclic group" as the substituent include a lower alkyl group (e.g. a C₁₋₆ alkyl group such as methyl, ethyl, propyl, etc.), an acyl group (e.g. a C₁₋₆ alkanoyl such as formyl, acetyl, propionyl, pivaloyl, etc., e.g. an aryl carbonyl such as benzoyl, etc., e.g. a C₁₋₆ alkylsulfonyl such as methylsulfonyl, ethylsulfonyl, etc., e.g. a substituted sulfonyl such as aminosulfonyl, methylaminosulfonyl, dimethylaminosulfonyl, etc.), a lower alkyl substituted by a halogen (e.g., trifluoromethyl, 1,1-difluoroethyl, etc.), etc.

Examples of the substituent in the "optionally substituted amino group", "optionally substituted imidoyl group", "optionally substituted amidino group", "optionally substituted hydroxyl group" and "optionally substituted thiol group" as the substituent include e.g. a lower alkyl group (e.g. a C₁₋₆ alkyl group such as methyl, ethyl, propyl, isopropyl, butyl, isobutyl, t-butyl, pentyl, hexyl, etc.), aryl group (e.g., phenyl, 4-methylphenyl, etc.), acyl group (C₁₋₆ alkanoyl (e.g., formyl, acetyl, propionyl, pivaloyl, etc.), e.g. aryl-carbonyl (e.g. benzoyl, etc.), C₁₋₆ alkylsulfonyl (e.g., methylsulfonyl, ethylsulfonyl, etc.), C₆₋₁₄ aryl-sulfonyl (e.g., para-toluenesulfonyl, etc.), etc., substituted-sulfonyl such as aminosulfonyl, methylaminosulfonyl, dimethylaminosulfonyl, etc.), an optionally halogenated C₁₋₆ alkoxy-carbonyl (e.g. trifluoromethoxycarbonyl, 2,2,2-trifluoroethoxycarbonyl, trichloromethoxycarbonyl, 2,2,2-trichloroethoxycarbonyl, etc.), etc. In addition, the "amino group" in the "optionally substituted amino group" as the substituent may be substituted with an optionally substituted imidoyl group (e.g., a C₁₋₆ alkylimidoyl, formimidoyl, amidino, etc.), etc. and two substituents of the "amino group" may form a cyclic amino group together with a nitrogen atom. Examples of said cyclic amino group include e.g. 3- to 8-membered (preferably 5- to 6-membered) cyclic amino group such as 1-azetidinyl, 1-pyrrolidinyl, piperidinol-piperidinyl, morpholino4-morpholinyl, 1-piperazinyl and 1-piperazinyl which may have at the 4-position a lower alkyl group (e.g. a C₁₋₆ alkyl group such as methyl, ethyl, propyl, isopropyl, butyl, t-butyl, pentyl, hexyl, etc.), an aralkyl group (e.g. a C₇₋₁₀ aralkyl group such as benzyl, phenethyl, etc.), an aryl group (e.g. a C₆₋₁₀ aryl group such as phenyl, 1-naphthyl, 2-naphthyl, etc.), etc.

Examples of the "optionally substituted carbamoyl group" include unsubstituted carbamoylas well as a N-mono-substituted carbamoyl group and a N,N-di-substituted carbamoyl group.

The "N-mono-substituted carbamoyl group" is a carbamoyl group having one substituent on the nitrogen atom and said substituent include e.g. a lower alkyl group (e.g. a C₁₋₆ alkyl group such as methyl, ethyl, propyl, isopropyl, butyl, isobutyl, t-butyl, pentyl, hexyl, etc.), a cycloalkyl group (e.g. a C₃₋₆ cycloalkyl group such as cyclopropyl, cyclobutyl, cyclopentyl, cyclohexyl, etc.), an aryl group (e.g. a C₆₋₁₀ aryl group such as phenyl, 1-naphthyl, 2-naphthyl, etc.), an aralkyl group (e.g. a C₇₋₁₀ aralkyl group such as benzyl, phenethyl, etc., preferably a phenyl-C₁₋₄ alkyl group etc.), a heterocyclic group (e.g. the same substituent as those in the above described "heterocyclic group" as the substituent of the "optionally substituted hydrocarbon group" represented by R^{a1}, etc.), etc. Said the lower alkyl group, the cycloalkyl group, the aryl group, the aralkyl group and the heterocyclic group may have a substituent and examples of the substituent include e.g. a hydroxyl group, an optionally substituted amino group [said amino group may have 1 to 2 substituents (e.g. a lower alkyl group (e.g. a C₁₋₆ alkyl group such as methyl, ethyl, propyl, isopropyl, butyl, isobutyl, t-butyl, pentyl, hexyl, etc.), an acyl group (e.g. a C₁₋₆ alkanoyl such as formyl, acetyl, propionyl, pivaloyl, etc., an arylcarbonyl such as benzoyl, etc., a C₁₋₆ alkylsulfonyl such as methylsulfonyl, ethylsulfonyl, etc.), etc.)], a halogen atom (e.g. fluorine, chlorine, bromine, iodine, etc.), a nitro group, a cyano group, a lower alkyl group optionally substituted with 1 to 5 halogen atoms (e.g. fluorine, chlorine, bromine, iodine, etc.), a lower alkoxy group optionally substituted with 1 to 5 halogen atoms (e.g., fluorine, chlorine, bromine, iodine, etc.), etc. Said lower alkyl group include e.g. a C₁₋₆ alkyl group such as methyl, ethyl, n-propyl, isopropyl, n-butyl, isobutyl, sec-butyl, tert-butyl, pentyl, hexyl, etc. and in particular methyl, ethyl, etc. are preferable. Said lower alkoxy group include e.g., a C₁₋₆ alkoxy group such as methoxy, ethoxy, n-propoxy, isopropoxy, n-butoxy, isobutoxy, sec-butoxy, tert-butoxy, etc. and in particular methoxy, ethoxy, etc. are preferable. It is preferable that one, two or three substituents, more preferably two substituents are present.

The "N,N-di-substituted carbamoyl group" is a carbamoyl group having two substituents on the nitrogen atom. Examples of one of the substituents include the same as those of the above described "N-mono-substituted carbamoyl group" and examples of the other substituent include e.g. a lower alkyl group (e.g. a C₁₋₆ alkyl group such as methyl, ethyl, propyl, isopropyl, butyl, t-butyl, pentyl, hexyl, etc.), a C₃₋₆ cycloalkyl group (e.g. cyclopropyl, cyclobutyl, cyclopentyl, cyclohexyl, etc.), a C₇₋₁₀ aralkyl group (e.g. benzyl, phenethyl, etc., preferably phenyl-C₁₋₄ alkyl group, etc.), etc. In addition, two substituents of the "N,N-di-substituted carbamoyl group" may form a cyclic amino-group together with a nitrogen atom. Examples of said cyclic amino-carbamoyl group include e.g. 3- to 8-membered (preferably 5- to 6-membered) cyclic amino-carbamoyl group such as 1-azetidinylcarbonyl, 1-pyrrolidinylcarbonyl, 1-piperidinylcarbonyl, 4-morpholinylcarbonyl, 1-piperazinylcarbonyl and 1-piperazinylcarbonyl which may have at the 4-position a lower alkyl group (e.g. a C₁₋₆ alkyl group such as methyl, ethyl, propyl, isopropyl, butyl, t-butyl, pentyl, hexyl, etc.), an aralkyl group (e.g. a C₇₋₁₀ aralkyl group such as benzyl, phenethyl, etc.), an aryl group (e.g. a C₆₋₁₀ aryl group such as phenyl, 1-naphthyl, 2-naphthyl, etc.), etc.

Examples of the substituent in the "optionally substituted thiocarbamoyl group" include the same substituent as those in the above described "optionally substituted carbamoyl group".
Examples of the "sulfamoyl group which may be substituted" include an unsubstituted-sulfamoyl group, a N-mono-substituted sulfamoyl group and a N,N-di-substituted sulfamoyl group.

The "N-mono-substituted sulfamoyl group" is a sulfamoyl group having one substituent at the nitrogen atom, and examples of the substituent include those mentioned as the substituent of N-mono-substituted carbamoyl group The "N,N-di-substituted sulfamoyl group" is a sulfamoyl group having two substituents at the nitrogen atom, and examples of the substituent include those mentioned as the substituent of the N,N-di-substituted carbamoyl group.

Examples of the optionally esterified carboxyl group include a free carboxyl group as well as a lower alkoxycarbonyl group, an aryloxycarbonyl group, an aralkyloxycarbonyl group, etc.

Examples of the "lower alkoxycarbonyl group" include e.g. a C₁₋₆ alkoxy-carbonyl group such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl, etc. Among them, a C₁₋₃ alkoxy-carbonyl group such as methoxycarbonyl, ethoxycarbonyl, propoxycarbonyl, etc. are preferable.

Examples of the "aryloxycarbonyl group" include e.g. a C₇₋₁₂ aryloxy-carbonyl group such as phenoxycarbonyl, 1-naphthoxycarbonyl, 2-naphthoxycarbonyl, etc.

Examples of the "aralkyloxycarbonyl group" include e.g. a C₇₋₁₀ aralkyloxy-carbonyl group, etc. (preferably, a C₆₋₁₀ aryl-C₁₋₄ alkoxy-carbonyl, etc.) such as benzyloxycarbonyl, phenethyloxycarbonyl, etc.

Said "aryloxycarbonyl group" and "aralkyloxycarbonyl group" may be substituted. Examples of the substituent include the same kind and number of the substituents of the aryl group and aralkyl group as examples of the substituent for the above described N-mono-substituted carbamoyl group.

Examples of the "acyl group derived from a sulfonic acid" as the substituent include a sulfonyl group substituted by a hydrocarbon group, and preferably include an acyl group such as C₁₋₁₀ alkyl-sulfonyl, C₂₋₆ alkenylsulfonyl, C₂₋₆ alkynyl-sulfonyl, C₃₋₉ cyclo-alkyl-sulfonyl, C₃₋₉ cyclo-alkenyl-sulfonyl, C₆₋₁₄ aryl-sulfonyl, C₇₋₁₀ aralkyl-sulfonyl etc. Examples of the C₁₋₁₀ alkyl include, for example, methyl, ethyl, propyl, isopropyl, butyl, isobutyl, t-butyl, pentyl, hexyl, heptyl, octyl, etc. Examples of the C₂₋₆ alkenyl include, for example, vinyl, allyl, 1-propenyl, isopropenyl, 2-butenyl, 3-butenyl, 2-hexenyl, etc. Examples of C₂₋₆ alkynyl include, for example, ethynyl, 2-propynyl, 2-butynyl, 5-hexynyl, etc. Examples of the C₃₋₉ cyclo-alkyl include, for example, cyclopropyl, cyclobutyl, cyclopentyl, cyclohexyl, cyclooctyl, etc.

Examples of the C₃₋₉ cyclo-alkenyl include, for example, 1-cyclopenten-1-yl, 2-cyclopenten-1-yl, 3-cyclopenten-1-yl, 3-cyclohexen-1-yl, 3-cycloocten-1-yl, etc. Examples of the C₆₋₁₄ aryl include, for example, phenyl, 1-naphthyl, 2-naphthyl, etc. Examples of the C₇₋₁₀ aralkyl-sulfonyl include, for example, benzyl, phenethyl, etc. These hydrocarbon groups bonded to the sulfonyl may be substituted. Examples of the substituent include, for example, hydroxyl, amino which may be substituted [The amino may be substituted with 1 or 2 substituents. (Examples of the substituent include lower alkyl group (e.g. C₁₋₆ alkyl etc. such as methyl, ethyl, propyl, isopropyl, butyl, isobutyl, t-butyl, pentyl, hexyl, etc.), an acyl group (e.g., C₁₋₆ alkanoyl such as formyl, acetyl, propionyl, pivaloyl, etc., aryl carbonyl such as benzoyl, etc., C₁₋₆ alkyl-sulfonyl such as methylsulfonyl, ethylsulfonyl, etc.)), halogen atom (for example, fluorine, chlorine, bromine, iodine, etc.), nitro group, cyano group, lower alkyl group which may be substituted by 1 to 5 halogen atom (for example, fluorine, chlorine, bromine, iodine, etc.), lower alkyl group which may be substituted by 1 to 5 halogen atom (for example, fluorine, chlorine, bromine, iodine, etc.) etc. Examples of the lower alkyl group include, for example, C₁₋₆ alkyl group such as methyl, ethyl, n-propyl, isopropyl, n-butyl, isobutyl, sec-butyl, tert-butyl, pentyl, hexyl, etc., and preferably include methyl, ethyl, etc. Examples of the lower alkoxy group include, for example, C₁₋₆ alkoxy group such as methoxy, ethoxy, n-propoxy, isopropoxy, n-butoxy, isobutoxy, sec-butoxy, tert-butoxy, etc, and preferably include methoxy, ethoxy, etc. These substituents may be the same or different from each other, and one, two or three substituents, preferably one or two substituents may be present.

Examples of the "acyl group derived from a carboxylic acid" as the substituent include a carbonyl group having a hydrogen atom or one substituent which the above described "N-mono-substituted carbamoyl group" have on the nitrogen atom, etc., preferably, a C₁₋₆ alkanoyl such as formyl, acetyl, trifluoroacetyl, propionyl, butyryl, isobutyryl, pivaloyl, etc., an acyl of an aryl-carbonyl such as benzoyl etc. Examples of the alkyl in "alkyl-sulfinyl group which may be substituted" as a substituent include a lower alkyl, for example, a C₁₋₆ alkyl such as methyl, ethyl, propyl, isopropyl, butyl, isobutyl, t-butyl, pentyl, hexyl, etc.

Examples of the aryl in "aryl-sulfinyl group which may be substituted" as a substituent include, for example, a C₆₋₁₄ aryl group etc., such as phenyl, naphthyl, anthryl, phenanthryl, acenaphthylenyl, etc. Examples of the substituent of the alkyl group or the aryl group include a lower alkoxy group (e.g. a C₁₋₆ alkoxy group etc, such as methoxy, ethoxy, propoxy, etc.), a halogen atom (e.g., fluorine, chlorine, bromine, iodine, etc.), a lower alkyl group (a C₁₋₆ alkyl group etc., such as methyl, ethyl, propyl, etc.), amino, hydroxyl, cyano, amidino, etc. One or two of these substituents may be present at any substitutable position.

Examples of "hydrocarbon group which may be substituted" and "non-aromatic heterocyclic group which may be substituted" shown as R^{a2} include the same ones as "hydrocarbon groups which may be substituted" and "non-aromatic heterocyclic group which may be substituted" shown by R^{a1}, respectively. Among them, a C₂₋₆ alkyl which may be substituted and a C₃₋₈ cycloalkyl which may be substitutedare preferable.

When R^{a1} and R^{a2} are bind to each other together with the adjacent nitrogen atom to form a heterocyclic ring group which may be substituted, the heterocyclic ring contains one nitrogen atom, and may further contain a nitrogen atom, an oxygen atom and a sulfur atom. Examples of the ring include, for example, a cyclic amino group such a monocyclic ring as 1-azetidinyl, 1-pyrrolidinyl, 1-piperidinyl, 1-homopiperidinyl, heptamethyleneimino, 1-piperazinyl, 1-homopiperazinyl, 4-morpholinyl, 4-thiomorpholinyl, etc., such a fused ring as 2-isoindolinyl, 1,2,3,4-tetrahydro-2-isoquinolyl, 1,2,4,5-tetrahydro-3H-3-benzoazepine-3-yl, etc., such a spiro ring as inden-1-spiro-4'-piperidin-1'-yl, etc. The cyclic amino group may have 1 to 5 substituents, preferably 1 to 3 substituents at chemically substitutable positions on the ring.

Examples of the substituent include a hydroxyl group, a cyano group, a nitro group, an amino group, an oxo group, a halogen atom and a group represented by the formula: -YR^{aa}, wherein R^{aa} is a hydrocarbon group which may be substituted or a heterocyclic group which may be substituted, and Y is a bond (a single bond), -CR^{ab}R^{ac}-, -COO-, -CO-, -CR^{ab} (OH)-, -CO-NR^{ab}-, -CS-NR^{ab}-, -CO-S-, -CS-S-, -CO-NR^{ab}-CO-NR^{ac}-, -C(=NH)-NR^{ab}-, -NR^{ab}-, -NR^{ab}-CO-, -NR^{ab}-CS-, -NR^{ab}-CO-NR^{ac}-, -NR^{ab}-CS-NR^{ac}-, -NR^{ab}-CO-O-, -NR^{ab}-CS-O-, -NR^{ab}-CO-S-, -NR^{ab}-CS-S-, -NRab-C (=NH)-NR^{ac}-, -NR^{ab}-SO₂-, -NR^{ab}-NR^{ac}-, -O-, -O-CO-, -O-CS-, -O-CO-O, -O-CO-NR^{ab}-, -O-C(=NH)-NR^{ab}-, -S-, -SO-, -SO₂-, -CR^{ab}R^{ac}-S-, CR^{ab}R^{ac}-SO₂-, -SO₂-NR^{ab}-, - S-CO-, -S-CS-, -S-CO-NR^{ab}-, -S-CS-NR^{ab}-, -S-C (=NH)-NR^{ab}-, wherein each of R^{ab} and R^{ac} is a hydrogen atom, an alkyl group which may be substituted, an alkenyl group which may be substituted, an alkynyl group which may be substituted, an aryl group which may be substituted, a cycloalkyl group which may be substituted, a cycloalkenyl group which may be substituted, a heterocyclic group which may be substituted, an acyl group derived from sulfonic acid, an acyl group derived from carboxylic acid, etc.

Examples of the "hydrocarbon group" in the "optionally substituted hydrocarbon group" represented by R^{aa} include e.g. an aliphatic hydrocarbon group, an alicyclic hydrocarbon group and an aryl group, etc. Examples of the aliphatic hydrocarbon group, the alicyclic hydrocarbon group and the aryl group include those represented by R^{a1} Examples of the substituent of the "hydrocarbon group optionally substituted" include the same substituent as those in the above described "hydrocarbon group which may be substituted" represented by R^{a1}.

Examples of the heterocyclic group in the "heterocyclic group which may be substituted" represented by R^{aa} include the same heterocyclic group as those of "heterocyclic group which may be substituted" represented by R^{a3} mentioned below. Examples of the "substituent" in the "heterocyclic group which may be substituted" include those mentioned as the "substituent" in a "non-aromatic heterocyclic group which may be substituted" represented by R^{a1}.

Examples of the "alkyl group which may be substituted", "alkenyl group which may be substituted", "alkynyl group which may be substituted", "aryl group which may be substituted", "cyclo-alkyl group which may be substituted", "cyclo-alkenyl group which may be substituted", "heterocyclic group which may be substituted", "acyl group derived from sulfonic acid", and "acyl group derived from carboxylic acid", each of which is represented by R^{ab} and R^{ac}, include those mentioned as the substituent in the "hydrocarbon group which may be substituted" represented by R^{a1}.

R^{a1} and R^{a2} are preferable to bind to each other together with the nitrogen atom to form a heterocyclic ring which may be substituted. More preferably, NR^{a1}R^{a2} is a group represented by the formula: 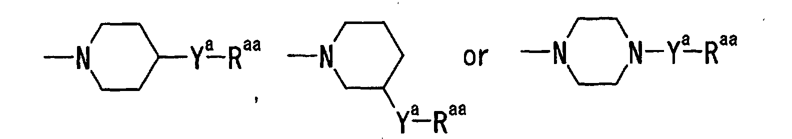 (wherein Y^{a} and R^{aa} have the same meanings given above). In the above, while Y^{a} and R^{aa} have the same meanings given above, R^{aa} is more preferably an aryl group which may be substituted or a heterocyclic group which may be substituted.

Examples of a "cyclic hydrocarbon group" in the "cyclic hydrocarbon group which may be substituted" represented by R^{a3} include e.g. an alicyclic hydrocarbon group, an aryl group, etc.

Examples of the "alicyclic hydrocarbon group" include e.g. a saturated or unsaturated alicyclic hydrocarbon group such as a cycloalkyl group, a cycloalkenyl group, a cycloalkanedienyl group, etc., preferably a cycloalkyl group.

Examples of the "cycloalkyl group" include e.g. a C₃₋₉ cycloalkyl, (preferably a C₃₋₈ acycloalkyl, etc.) such as cyclopropyl, cyclobutyl, cyclopentyl, cyclohexyl, cycloheptyl, cyclooctyl, cyclononyl, etc., and a fused ring such as 1-indanyl, 2-indanyl, etc.

Examples of the "cycloalkenyl group" include e.g. a C₃₋₆ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl, etc.

Examples of the "cycloalkanedienyl group" include e.g. a C₄₋₆ cycloalkanedienyl group such as 2,4-cyclopentadien-1-yl, 2,4-cyclohexadien-1-yl, 2,5-cyclohexadien-1-yl, etc.

Examples of the "aryl group" include e.g. a monocyclic or fused polycyclic aromatic hydrocarbon group. Among them, a C₆₋₁₄ aryl group such as phenyl, naphthyl, anthryl, phenathryl, acenaphthyl, etc. is preferable. In particular, phenyl, 1-naphthyl, 2-naphthyl, etc. are preferable.

Examples of the substituent in the "cyclic hydrocarbon group which may be substituted" represented by R^{a3} include those mentioned as the substituent in the "hydrocarbon group which may be substituted" represented by R^{a1}. The substituent include, for example, a phenyl group, a phenyl group which may be substituted by a C₁₋₆ alkyl group such as a tolyl group, etc., a naphthyl group, etc., when the cyclic hydrocarbon group is alicyclic hydrocarbon group, and is preferably, for example a halogen atom (e.g., chlorine atom, fluorine atom, etc.), a C₁₋₆ alkyl group (methyl, ethyl, propyl, isopropyl, butyl, isobutyl, t-butyl, pentyl, hexyl, etc.), a C₁₋₆ alkoxy group (e.g., methoxy, ethoxy, n-propoxy, isopropoxy, n-butoxy, etc.), a C₃₋₆ cyclo-alkyl group (e.g., cyclopropyl, cyclobutyl, cyclopentyl, cyclohexyl, etc.), a halogenated-C₁₋₆ alkyl group (trifluoromethyl, etc.), a halogenated-C₁₋₆ alkoxy group (trifluoromethyloxy, etc.), a C₁₋₆ alkyl-thio group (methylthio, ethylthio, etc.), a C₁₋₆ alkyl-sulfonyl group (methylsulfonyl, ethylsulfonyl, etc.), cyano group, nitro group, etc., when the cyclic hydrocarbon group is an aryl group.

Examples of the heterocyclic group in the "optionally substituted heterocyclic group" represented by R^{a3} include e.g. an aromatic heterocyclic group, a saturated or unsaturated non-aromatic heterocyclic group (an alicyclic heterocyclic group) etc., which contains, at least one hetero-atom (preferably 1 to 4 hetero-atoms, more preferably 1 to 2 hetero-atoms) consisting of 1 to 3 kinds of hetero-atoms (preferably 1 to 2 kinds of hetero-atoms) selected from an oxygen atom, a sulfur atom, a nitrogen atom, etc. as atom(s) which form a ring (ring atoms).

Examples of the "aromatic heterocyclic group" include an aromatic monocyclic heterocyclic group such as a 5- to 6-membered aromatic monocyclic heterocyclic group, etc. (e.g. furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.); an aromatic fused heterocyclic group such as a 8- to 12-membered aromatic fused heterocyclic group (e.g. benzofuranyl, isobenzofuranyl, benzothienyl, indolyl, isoindolyl, 1H-indazolyl, benzindazolyl, benzoxazolyl, 1,2-benzoisooxazolyl, benzothiazolyl, benzopyranyl, 1,2-benzoisothiazolyl, benzodioxolyl,benxoimidazolyl, 2,1,1-benzoxadiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, pyrazolo[3,4-b]piridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.); etc., preferably, a heterocyclic group consisting of the above-mentioned 5- or 6-membered aromatic monocyclic heterocyclic group fused with a benzene ring or a heterocyclic group consisting of the above-mentioned 5- or 6-membered aromatic monocyclic heterocyclic group fused with the same or different above-mentioned 5- or 6-membered aromatic monocyclic heterocyclic group, etc.

Examples of the "non-aromatic heterocyclic group" include a 3- to 8-membered (preferably 5- or 6-membered) saturated or unsaturated (preferably saturated) non-aromatic heterocyclic group (alicyclic heterocyclic group) such as oxiranyl, azetidinyl, oxetanyl, thiethanyl, pyrrolidinyl, tetrahydrofuryl, thiolanyl, piperidinyl, tetrahydropyranyl, morpholinyl, thiomorpholinyl, piperazinyl, etc.

Examples of the substituent in the "heterocyclic group which may be substituted" represented by R^{a3} include those mentioned as the substituent "non-aromatic heterocyclic group which may be substituted" represented by R^{a1}. R^{a3} is preferably a phenyl group which may be substituted.

Examples of the "hydrocarbon group which may be substituted" represented by R^{a4} include the same "hydrocarbon group which may be substituted" represented by R^{a1}. Examples of the "heterocyclic group which may be substituted" represented by R^{a4} include the same "heterocyclic group which may be substituted" represented by R^{a3}.

Examples of the "alkoxy group" in "alkoxy group which may be substituted" represented by R^{a4} preferably include, for example, a C₁₋₆ alkoxy such as methoxy, ethoxy, n-propoxy, isopropoxy, n-butoxy, isobutoxy, sec-butoxy, tert-butoxy, etc. Examples of the "substituent" include, for example, a cycloalkyl group (e.g. a C₃₋₆ cycloalkyl group such as cyclopropyl, cyclobutyl, cyclopentyl, cyclohexyl, etc.), an aryl group (e.g.a C₆₋₁₀ aryl group, etc., such as phenyl, 1-naphthyl, 2-naphthyl, etc.), an aralkyl group (e.g.a C₇₋₁₀ aralkyl group for example, such as benzyl, phenethyl, etc., preferably a phenyl-C₁₋₄ alkyl group, etc., etc.), a heterocyclic group (e.g., a "heterocyclic group" mentioned as the substituent in the "hydrocarbon group which may be substituted" represented by R^{a1}), etc. Each of the lower alkyl group, the cycloalkyl group, the aryl group, the aralkyl group and the heterocyclic group may be substituted. Examples of the substituents include, for example, a hydroxyl group, an amino group which may be substituted [the amino group may have 1 or 2 substituents such as a lower alkyl group (a C₁₋₆ alkyl group such as methyl, ethyl, propyl, isopropyl, butyl, isobutyl, t-butyl, pentyl, hexyl, etc.), an acyl group (a C₁₋₆ alkanoyl such as formyl, acetyl, propionyl, pivaloyl, etc., an aryl carbonyl such as benzoyl, etc. for example, a C₁₋₆ alkyl-sulfonyl etc. such as methyl-sulfonyl, ethyl-sulfonyl etc.)], a halogen atom (e.g. fluorine, chlorine, bromine, iodine etc.), a nitro group, a cyano group, a lower alkyl group (which may be substituted by 1 to 5 halogen atoms (e.g. fluorine, chlorine, bromine, iodine etc.)), a lower alkoxy group (which may be substituted by 1 to 5 halogen atoms (e.g. fluorine, chlorine, bromine, iodine etc.)), etc. Examples of the lower alkyl group include a C₁₋₆ alkyl group such as methyl, ethyl, n-propyl, isopropyl, n-butyl, isobutyl, sec-butyl, tert-butyl, pentyl, hexyl, etc., and in particular methyl, ethyl, etc. are preferable. Examples of the lower alkoxy group include, for example, a C₁₋₆ alkoxy group etc. such as methoxy, ethoxy, n-propoxy, isopropoxy, n-butoxy, isobutoxy, sec-butoxy, tert-butoxy, etc., and in particular methoxy, ethoxy, etc. are preferable. The above described lower alkoxy group may have 1 or 2 to 3 (preferably 1 or 2) substituents. When the alkoxy group has 2 or 3 substituents, these substituents may be the same or different.

Examples of the "aryl group" in "aryloxy group which may be substituted" represented by R^{a4} include, for example, a C₆₋₁₄ aryl group such as phenyl, naphthyl, anthryl, phenanthryl, acenaphthylenyl, etc. Examples of the substituent include, for example, a lower alkoxy group (e.g. a C₁₋₆ alkoxy group such as methoxy, ethoxy, propoxy, etc.), a halogen atom (e.g., fluorine, chlorine, bromine, iodine, etc.), a lower alkyl group (for example a C₁₋₆ alkyl group such as methyl, ethyl, propyl, etc.), an amino group, a hydroxyl group, a cyano group, an amidino group, etc. These optional aryloxy groups may have 1 or 2 at any possible position.

Examples of the "substituent" in "amino group which may be substituted" represented by R^{a4} include, for example, a lower alkyl group (e.g., a C₁₋₆ alkyl group such as methyl, ethyl, propyl, isopropyl, butyl, isobutyl, t-butyl, pentyl, hexyl, etc.), an acyl group (a C₁₋₆ alkanoyl (e.g., formyl, acetyl, propionyl, pivaloyl, etc.), benzoyl, etc.), a C₁₋₆ alkoxy-carbonyl which may be halogenated (e.g., trifluoromethoxycarbonyl, 2,2,2-trifluoroethoxycarbonyl, trichloromethoxy carbonyl, 2,2,2-trichloroethoxy carbonyl, etc.), etc. In addition, the "amino group" in the "optionally substituted amino group" as the substituent may be substituted with an optionally substituted imidoyl group (e.g., a C₁₋₆ alkylimidoyl, formimidoyl, amidino, etc.), etc. and two substituents of the "amino group" may form a cyclic amino group together with a nitrogen atom. Examples of said cyclic amino group include e.g. a 3- to 8-membered (preferably 5- to 6-membered) cyclic amino group such as 1-azetidinyl, 1-pyrrolidinyl, 1-piperidinyl, 4-morpholinyl, 1-piperazinyl and 1-piperazinyl which may have at the 4-position a lower alkyl group (e.g. a C₁₋₆ alkyl group etc. such as methyl, ethyl, propyl, isopropyl, butyl, t-butyl, pentyl, hexyl, etc.), an aralkyl group (e.g. a C₇₋₁₀ aralkyl group etc. such as benzyl, phenethyl, etc.), an aryl group (e.g. a C₆₋₁₀ aryl group etc. such as phenyl, 1-naphthyl, 2-naphthyl, etc.), etc.

R^{a4} is preferably a C₁₋₃ alkyl, a phenyl which may be substituted, 3-pyridyl, 4-pyridyl, etc.

Examples of the hydrocarbon group represented by R^{a5} include those mentioned as a "hydrocarbon group" in the "hydrocarbon group which may be substituted" represented by R^{a1}. The preferable examples of the hydrocarbon group include a lower alkyl group having 1 to 4 carbon atoms such as methyl, ethyl, n-propyl, isopropyl, butyl, n-butyl, isobutyl, tert-butyl, etc.

Examples of the counter anion represented by Y^{a}- include, for example, Cl⁻, Br⁻, I⁻, NO₃⁻, SO₄²⁻, PO₄³⁻, CH₃SO₃⁻, etc.

Examples of the divalent aliphatic hydrocarbon group in the divalent alipahatic hydrocarbon group which may be substituted by group other than an oxo group represented by E^{a} include, for example, a C₁₋₆ alkylene such as methylene, ethylene, etc., a C₂₋₆ alkenylene such as ethenylene, etc., a C₂₋₆ alkynylene such as ethynylen, etc., and among them, a C₂₋₅ alkylene is more preferable and trimethylene is the most preferable.

The substituent of the divalent hydrocarbon group is a substituent other than an oxo group, and examples of the substituents include, for example, an alkyl group which may be substituted, an aryl group which may be substituted, a cycloalkyl group or a cycloalkenyl group which may be substituted, a carboxyl group which may be esterified, a carbamoyl group or a thiocarbamoyl group which may be substituted, an amino group which may be substituted, a hydroxyl group which may be substituted, a thiol (i.e. mercapto) group which may be substituted, an acyl group derived from carboxylic acid, an acyl group derived from sulfonic acid, a halogen (e.g., fluorine, chlorine, bromine, etc.), nitro, cyano, etc. The divalent hydrocarbon group may have 1 to 3 substituents. Each of the alkyl group or the aryl group which may be substituted, the cycloalkyl group or the cycloalkenyl group which may be substituted, the carboxyl group which may be esterified, the carbamoyl group which may be substituted, the thiocarbamoyl group which may be substituted, the amino group which may be substituted, the hydroxyl group which may be substituted, the thiol group (i.e. mercapto group) which may be substituted, the acyl group derived from carboxylic acid and the acyl group derived from sulfonic acid include those mentioned as the substituent in "heterocyclic group which may be substituted" represented by R^{a3}.

Examples of the C₁₋₃ aliphatic hydrocarbon group in "divalent C₁₋₃aliphatic hydrocarbon group which may be substituted" represented by Q^{a} and R^{a} inclde a divalent aliphatic hydrocarbon group having 1 to 3 carbon atoms in the divalent aliphatic ydrocarbon group in divalent aliphatic hydrocarbon group which may be substituted by a group other than an oxo group represented by E^{a}.

Examples of the substituent in the "divalent C₁₋₃ aliphatic ydrocarbon group which may be substituted" represented by Q^{a} and R^{a} include those mentioned as the substituent in divalent aliphatic ydrocarbon group which may be substituted by a group other than an oxo group represented by E^{a}.

J^{a} is a methine or a nitrogen atom, and methine is preferable.

G^{a1} is a bond, CO or SO₂, and CO or SO₂ is preferable.

G^{a2} is CO, SO₂, NHCO, CONH or OCO, and among them, CO, NHCO and OCO are preferable
The compound of the formula (I) or a salt thereof may be an hydrate.

Following compounds are preferable.

(I-1) A compound as shown in the above (1), wherein R^{a1} is a hydrogen atom, a hydrocarbon group selected from Group a2 which may be substituted by member(s) selected from Group a1, a 3- to 8-membered saturated or unsaturated non-aromatic heterocyclic group which may be substituted by member(s) selected from Group a1; R^{a2} is a hydrocarbon group selected from Group a2 which may be substituted by member(s) selected from Group a1 or a 3- to 8-membered saturated or unsaturated non-aromatic heterocyclic group which may be substituted by member(s) selected from Group a1, or R^{a1} and R^{a2} may combine each other together with A^{a} to form a heterocyclic group selected from Group a4 which may be substituted by member(s) selected from Group a3; A^{a} is N or N⁺-R^{a5} • Y^{a}- (Y^{a}- is Cl⁻, Br⁻, I⁻, NO₃⁻, SO₄²-, PO₄³⁻ 3- or CH₃SO₃⁻; R^{a5} is a hydrocarbon group selected from Group a2); R^{a3} is a cyclic hydrocarbon group selected from Group a5 which may be substituted by member (s) selected from Group a1 or a heterocyclic group selected from Group a6 which may be substituted by member(s) selected from Group a1; R^{a4} is a hydrogen atom, a hydrocarbon group selected from Group a2 which may be substituted by member(s) selected from Group a1, a heterocyclic group, selected from Group a6 which may be substituted by member(s) selected from Group a1, a C₁₋₆ alkoxy group which may be substituted by member(s) selected from Group a7, a C₆₋₁₄ aryloxy group which may be substituted by member (s) selected from Group a8, an amino group which may be substituted by member(s) selected from Group a9 or a cyclic-amino group selected from Group a10; E^{a} is a divalent aliphatic hydrocarbon group selected from Group a12 which may be substituted by member(s) other than oxo group(s) and selected from Group a11; each of Q^{a} and R^{a} is a bond or a divalent C₁₋₃ aliphatic ydrocarbon group selected from Group a13 which may be substituted by member(s) selected from Group a11.

### Group a1

(1) a C₁₋₆ alkyl group which may be substituted by member(s) selected from Group a14, (2) a C₂₋₆ alkenyl group which may be substituted by member(s) selected from Group a14, (3) a C₂₋₆ alkynyl group which may be substituted by member (s) selected from Group a14, (4) a C₆₋₁₄ aryl group which may be substituted by member(s) selected from Group a14, (5) a C₃₋₇ cycloalkyl group which may be substituted by member(s) selected from Group a14, (6) a C₃₋₆ cycloalkenyl group which may be substituted by member(s) selected from Group a14, (7) a heterocyclic group selected from Group a16 which may be substituted by member (s) selected from Group a15, (8) an amino group which may be substituted by a C₁₋₆ alkyl-imidoyl(s), formyl-imidoyl(s), amidino(s) or member(s) selected from Group a17, (9) a cyclic-amino group selected from Group a10, (10) an imidoyl group which may be substituted by member(s) selected from Group a17, (11) an amidino group which may be substituted by member(s) selected from Group a17, (12) a hydroxyl group which may be substituted bya member selected from Group a17, (13) a thiol group which may be substituted by a member selected from Group a17, (14) a carboxyl group, (15) a C₁₋₆ alkoxycarbonyl group which may be substituted by member(s) selected from Group a18, (16) a C₇₋₁₂ aryloxy-carbonyl group which may be substituted by member(s) selected from Group a18, (17) a C₇₋₁₀ aralkyl-oxy-carbonyl group which may be substituted by member(s) selected from Group a18, (18) a carbamoyl group, (19) a mono-substituted carbamoyl group which may be substituted by a member selected from Group a19, (20) a di-substituted carbamoyl group substituted by a member selected from Group a19 and a member selected from Group a20, (21) a cyclic-aminocarbamoyl group selected from Group a21, (22) a thiocarbamoyl group, (23) a mono-substituted thiocarbamoyl group which may be substituted by a member selected from Group a19, (24) a di-substituted thiocarbamoyl group substituted by a member selected from Group a19 and a member selected from Group a20, (25) a cyclic-aminothiocarbamoyl group selected from Group a21, a sulfamoyl group, (26) a N-mono-substituted sulfamoyl group substituted by a member selected from Group a19, (27) a N,N-di-substituted sulfamoyl group substituted by a member selected from Group a19 and a member selected from Group a20, (28) a cyclic-amino-sulfonyl group selected from Group a22, (29) a halogen atom, (30) a cyano group, (31) a nitro group, (32) an acyl group derived from a sulfonic acid selected from Group a22, (33) a formyl group, (34) a C₂₋₆ alkanoyl group, (35) a C₇₋₁₂ aryl-carbonyl group, (36) a C₁₋₆ alkyl-sulfinyl group which may be substituted by member(s) selected from Group a23 and (37) a C₆₋₁₄ aryl-sulfinyl group which may be substituted by member(s) selected from Group a23

### Group a2

(1) a C₁₋₁₀ alkyl group, (2) a C₂₋₆ alkenyl group, (3) a C₂₋₆ alkynyl group, (4) a C₃₋₉ cycloalkyl group which may be condensed with benzene, (5) a C₃₋₆ cycloalkenyl group, (6) a C₄₋₆ cycloalkadienyl group and (7) a C₆₋₁₄ aryl group

### Group a3

(1) a hydroxy group, (2) a cyano group, (3) a nitro group, (4) an amino group, (5) an oxo group, (6) a halogen atom and (7) a group represented by the formula:-B¹R^{aa} [wherein R^{aa} is a hydrocarbon group selected from Group a2 which may be substituted by member(s) selected from Group a1, or a heterocyclic group selected from Group a6 which may be substituted by member(s) selected from Group a1, B¹ is a bond (a single bond), -CR^{ab}R^{ac}-, -COO-, -CO-, -CR^{ab} ( OH )-, -CR^{ab}R^{ac}-S-, -CR^{ab}R^{ac}-SO₂-, -CO-NR^{ab}-, -CS-NR^{ab}-, -CO-S-, -CS-S-, -CO-NR^{ab}-CO-NR^{ac}-, -C (=NH)-NR^{ab}-, -NR^{ab}-, -NR^{ab}-CO-, -NR^{ab}-CS-, -NR^{ab}-CO-NR^{ac}-, -NR^{ab}-CS-NR^{aC}-, -NR^{ab}-CO-O-, -NR^{ab}-CS-O-, -NR^{ab}-CO-S-, -NR^{ab}-CS-S-, -NR^{ab}-C (=NH)-NR^{ac}-, -NR^{ab}-SO₂-, -NR^{ab}-NR^{ac}-, -O-, -O-CO-, -O-CS-, -O-CO-O-, -O-CO-NR^{ab}-, -O-C (=NH)-NR^{ab}-, -S-, -SO-, -SO₂-, -SO₂-NR^{ab}-, -S-CO-, -S-CS-, -S-CO-NR^{ab}-, -S-CS-NR^{ab}-and-S-C (=NH)-NR^{ab}- (wherein each of R^{ab} and R^{ac} is a hydrogen atom, a C₁₋₆ alkyl group which may be substituted by member(s) selected from Group a14, a C₂₋₆ alkenyl group which may be substituted by member(s) selected from Group a14, a C₂₋₆ alkynyl group which may be substituted by member(s) selected from Group a14, a C₆₋₁₄ aryl group which may be substituted by member(s) selected from Group a14, a C₃₋₇ cycloalkyl group which may be substituted by member (s) selected from Group a14, a C₃₋₆ cycloalkenyl group which may be substituted by member(s) selected from Group a14, a heterocyclic group selected from Group a6 which may be substituted by member(s) selected from Group a1, an acyl group derived from a sulfonic acid selected from Group a22, a C₁₋₆ alkanoyl, and a C₇₋₁₂ arylcarbonyl group)]

### Group a4

(1) a monocyclic heterocyclic group, (2) a heterocyclic group condensed with benzene and (3) a heterocyclic spiro compound, each of which contains one nitrogen atom and may further contain one or more atoms selected from the group consisting of a nitrogen atom, a oxygen atom and a sulfur atom

### Group a5

(1) a C₃₋₉ cycloalkyl which may be condensed with benzene, (2) a C₃₋₆ cycloalkenyl group, (3) a C₄₋₆ cycloalkadienyl group and (4) a C₆₋₁₄ aryl group

### Group a6

(1) a 5- to 6-membered aromatic monocyclic heterocyclic group selected from Group a24, (2) a 8- to 12-membered aromatic condensed heterocyclic group selected from Group a26 and (3) a 3- to 8-membered saturated or unsaturated non-aromatic heterocyclic group (aliphatic heterocyclic group) selected from Group a25, each of which contains at least one hetero atom of one to three hetero atoms selected from the group consisting of an oxygen atom, a sulfur atom and a nitrogen atom etc. as a ring formed of atoms (ring atoms)

### Group a7

a C₃₋₆ cycloalkyl group which may be substituted by member (s) selected from Group a18, a C₆₋₁₀ aryl group which may be substituted by member(s) selected from Group a18, a C₇₋₁₀ aralkyl group which may be substituted by member (s) selected from Group a18 and a heterocyclic group selected from Group a16 which may be substituted by member(s) selected from Group a18

### Group a8

a C₁₋₆ alkoxy group, a halogen atom, a C₁₋₆ alkyl group, an amino group, a hydroxyl group, a cyano group and an amidino group

### Group a9

(1) a C₁₋₆ alkyl group, (2) a C₁₋₆ alkanoyl, (3) benzoyl, (4) a C₁₋₆ alkoxy-carbonyl which may be substituted by halogen (s), (5) a C₁₋₆ alkyl-imidoyl, (6) formyl-imidoyl and (7) amidino

### Group a10

(1) 1-azetidinyl, (2) 1-pyrrolidinyl, (3) 1-piperidinyl, (4) 4-morpholinyl and (5) a 1-piperazinyl which may be substituted by member (s) selected from Group a27

### Group a11

(1) a C₁₋₆ alkyl group which may be substituted by member (s) selected from Group a14, (2) a C₆₋₁₄ aryl group which may be substituted by member (s) selected from Group a14, (3) a C₃₋₇ cycloalkyl group which may be substituted by member(s) selected from Group a14, (4) a C₃₋₆ cycloalkenyl group which may be substituted by member(s) selected from Group a14, (5) a carboxyl group, (6) a C₁₋₆ alkoxy-carbonyl group which may be substituted by member(s) selected from Group a18, (7) a C₇₋₁₂ aryloxy-carbonyl group which may be substituted by member(s) selected from Group a18, (8) a C₇₋₁₀ aralkyl-oxy-carbonyl group which may be substituted by member(s) selected from Group a18, (9) a carbamoyl group, (10) a mono-substituted carbamoyl group substituted by a member selected from Group a19, (11) a di-substituted carbamoyl group substituted by a member selected from Group a19 and a member selected from Group a20, (12) a cyclic-aminocarbamoyl group selected from Group a21, (13) a thiocarbamoyl group, (14) a mono-substituted thiocarbamoyl group substituted by a member selected from Group a19, (15) a di-substituted thiocarbamoyl group substituted by a member selected from Group a19 and a member selected from Group a20, (16) a cyclic-aminothiocarbamoyl group selected from Group a21, (17) an amino group which may be substituted by a C₁₋₆ alkyl-imidoyl(s), formyl-imidoyl(s), amidino(s) or member(s) selected from Group a17, (18) a cyclic-amino group selected from Group a10, (19) a hydroxyl group which may be substituted by a member selected from Group a17, (20) a thiol group which may be substituted by a member selected from Group a17, (21) a C₁₋₆ alkanoyl group, (22) a C₇₋₁₂ aryl-carbonyl group, (23) an acyl group derived from a sulfonic acid selected from Group a22, (24) a halogen, (25) nitro and (26) cyano.

### Group a12

a C₁₋₆ alkylene, a C₂₋₆ alkenylene and a C₂₋₆ alkynylene

### Group a13

a C₁₋₃ alkylene, a C₂₋₃ alkenylene and a C₂₋₃ alkynylene

### Group a14

(1) a C₁₋₆ alkoxy group which may be substituted by halogen(s), (2) a phenoxy which may be substituted by halogen (s) or carbamoyl (s), (3) a halogen atom, (4) a C₁₋₆ alkyl group, (5) a C₁₋₄ alkyl group substituted by halogen(s), (6) C₃₋₈ cycloalkyl, (7) an amino group, (8) an amino group substituted by one or two members selected from the group consisting of carbamoyl, C₁₋₄ alkyl and C₁₋₄ alkylsulfonyl, (9) a carbamoyl group which may be substituted by C₁₋₆ alkyl (s), (10) formyl, (11) a C₂₋₆ alkanoyl group, (12) a C₆₋₁₄ aryl group, (13) a C₆₋₁₄ aryl-carbonyl, (14) a C₇₋₁₃ aralkyl-carbonyl, (15) a hydroxyl group, (16) a C₂₋₅ alkanoyl-oxy, (17) a C₇₋₁₃ aralkyl-carbonyloxy (18) a nitro group, (19) a sulfamoyl group, (20) a N-C₁₋₄ alkyl-sulfamoyl, (21) a phenyl-thio, (22) a C₁₋₄ alkyl-phenylthio, (23)-N=N-phenyl, (24) a cyano group, (25) an oxo group, (26) an amidino group, (27) a carboxyl group, (28) a C₁₋₄ alkoxy-carbonyl group, (29) a C₁₋₆ alkyl-thio, (30) a. C₁₋₆ alkyl-sulfinyl, (31) a C₁₋₆ alkyl-sulfonyl, (32) a C₆₋₁₄ aryl-thio, (33) a C₆₋₁₄ aryl-sulfinyl, (34) a C₆₋₁₄ aryl-sulfonyl and (35) a heterocyclic group selected from Group a6

### Group a15

a C₁₋₆ alkyl group, a C₁₋₆ alkanoyl, a C₇₋₁₃ aryl-carbonyl, a C₁₋₆ alkyl-sulfonyl, an aminosulfonyl, a mono-C₁₋₆ alkyl-aminosulfonyl, a di-C₁₋₆ alkyl-aminosulfonyl and a C₁₋₄ alkyl group substituted by halogen

### Group a16

(1) an aromatic heterocyclic group selected from Groups 24 and 26, and (2) a saturated or unsaturated non-aromatic heterocyclic group selected from Group a25, each of which contains at least one hetero atom of one to three hetero atoms selected from the group consisting of an oxygen atom, a sulfur atom and a nitrogen atom as ring constituting atom(s) (a ring atom)

### Group a17

(1) a C₁₋₆ alkyl group which may be substituted by halogen or a C₁₋₆ alkoxy, (2) a C₆₋₁₂ aryl group, (3) a C₆₋₁₂ aryl group substituted by C₁₋₄ alkyl (s), (4) a C₃₋₈ cycloalkyl group which may be substituted by halogen(s) or C₁₋₆ alkoxy(s), (5) a C₁₋₆ alkoxy group, (6) a C₁₋₆ alkanoyl, (7) a C₇₋₁₃ aryl-carbonyl, (8) a C₇₋₁₃ aryl-carbonyl substituted by C₁₋₄ alkyl(s), (9) a C₁₋₆ alkyl-sulfonyl, (10) a C₆₋₁₄ aryl-sulfonyl, (11) a aminosulfonyl, (12) a mono- or di-substituted aminosulfonyl substituted by C₁₋₄ alkyl(s) and (13) a C₁₋₆ alkoxy-carbonyl which may be substituted by halogen(s)

### Group a18

(1) a hydroxyl group, (2) an amino group, (3) a mono or di-substituted amino group substituted by member(s) selected from Group a28, (4) a halogen atom, (5) a nitro group, (6) a cyano group, (7) a C₁₋₆ alkyl group which may be substituted by halogen atom(s) and (8) a C₁₋₆ alkoxy group which may be substituted by halogen atom(s)

### Group a19

a C₁₋₆ alkyl group which may be substituted by member (s) selected from Group a18, a C₃₋₆ cycloalkyl group which may be substituted by member(s) selected from Group a18, a C₆₋₁₀ aryl group which may be substituted by member (s) selected from Group a18, a C₇₋₁₀ aralkyl group which may be substituted by member(s) selected from Group a18, a C₁₋₆ alkoxy group which may be substituted by member(s) selected from Group a18 and a heterocyclic group selected from Group a16 which may be substituted by member(s) selected from Group a18

### Group a20

a C₁₋₆ alkyl group, a C₃₋₆ cycloalkyl group and a C₇₋₁₀ aralkyl group

### Group a21

a 1-azetidinyl-carbonyl, a 1-pyrrolidinyl-carbonyl, a 1-piperidinyl-carbonyl, a 4-morpholinyl-carbonyl and a 1-piperazinyl-carbonyl which may be substituted by member(s) selected from Group a27

### Group a22

a C₁₋₁₀ alkyl-sulfonyl which may be substituted by member(s) selected from Group a18, a C₂₋₆ alkenyl-sulfonyl which may be substituted by member(s) selected from Group a18, a C₂₋₆ alkynyl-sulfonyl which may be substituted by member(s) selected from Group a18, a C₃₋₉ cycloalkylsulfonyl which may be substituted by member(s) selected from Group a18, a C₃₋₉ cycloalkenyl-sulfonyl which may be substituted by member(s) selected from Group a18, a C₆₋₁₄ aryl-sulfonyl which may be substituted by member(s) selected from Group a18 and a C₇₋₁₀ aralkyl-sulfonyl group which may be substituted by member(s) selected from Group a18

### Group a23

a C₁₋₆ alkoxy group, a halogen atom, a C₁₋₆ alkyl group, an amino group, a hydroxyl group, a cyano group and an amidino group

### Group a24

furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl

### Group a25

oxylanyl, azetidinyl, oxetanyl, thietanyl, pyrrolidinyl, tetrahydro furyl, thiolanyl, piperidinyl, tetrahydropyranyl, morpholinyl, thiomorpholinyl and piperazinyl

### Group a26

benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, benzodioxolyl, benzimidazolyl, 2,1,1-benzoxadiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenathrolinyl, indolizinyl, pyrrolo[1,2-b]pyridazinyl, pyrazolo[1,5-a]pyridyl, pyrazolo[3,4-b]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl

### Group a27

a C₁₋₆ alkyl group, a C₇₋₁₀ aralkyl group and a C₆₋₂₀ aryl group

### Group a28

a C₁₋₆ alkyl group, a C₁₋₆ alkanoyl, a C₇₋₁₃ aryl-carbonyl and a C₁₋₆ alkyl-sulfonyl

(I-2) a compound as shown in the above (I-1), wherein the 3- to 8-membered saturated or unsaturated nonaromatic heterocyclic group which may be substituted by member(s) selected from Group a1, represented by each of R^{a1} and R^{a2} is a 3- to 8-membered saturated or unsaturated nonaromatic heterocyclic group selected from Group a25 which may be substituted by member (s) selected from Group a1, and the heterocyclic group, selected from Group a4 which may be substituted by member(s) selected from Group a3 formed by combining R^{a1} and R^{a2} together with A^{a} is a cyclic-amino group selected from Group a29 which may be substituted by member (s) selected from Group a3.

### Group a29

1-azetidinyl, 1-pyrrolidinyl, 1-piperidinyl, 1-homopiperidinyl, heptamethylenimino, 1-piperazinyl, 1-homopiperazinyl, 4-morpholinyl, 4-thiomorpholinyl, 2-isoindolinyl, 1,2,3,4-tetrahydro-2-isoquinolyl, 1,2,4,5-tetrahydro-3H-3-benzazepin-3-yl and indene-1-spiro-4'-piperidine-1'-yl

(I-3) a compound as shown in the above (I-1) wherein R^{a1} and R^{a2} combine each other together with A^{a} to form a 3-to 8-membered saturated or unsaturated non-aromatic heterocyclic group selected from Group a4, which may be substituted by member(s) selected from Group a3.

(I-4) a compound as shown in the above (I-1) wherein R^{a1} and R^{a2} combine each other together with A^{a} to form a 3- to 8-membered saturated or unsaturated non-aromatic heterocyclic group containing one or two nitrogen atoms, which ring may be substituted by member(s) selected from Group a3.

(I-5) a compound as shown in the above (I-3), wherein the group represented by -A^{a}R^{a1}R^{a2} is (1) a piperidinyl or (2) a piperazinyl group, each of which may be substituted by member(s) selected from Group a3.

(I-6) a compound as shown in the above (I-3), wherein the group represented by -A^{a}R^{a1}R^{a2} is a group represented by the formula: 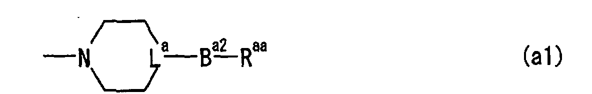 [wherein L^{a} is methine or a nitrogen atom, B^{a2} is a bond, -CH₂-, -SO₂-, -SO-, -S-, -O-, -CO-, -NR^{ab1}-SO₂- (wherein R^{ab1} is a hydrogen atom, a C₁₋₆ alkyl group, a C₂₋₆ alkenyl group, a C₂₋₆ alkynyl group, a C₃₋₆ cycloalkyl group), -CH (OH)-, -NR^{ab2}- (wherein R^{ab2} is a hydrogen atom or a C₂₋₄ alkanoyl group), -NR^{ab1}-CO- (wherein R^{ab1} has the same meaning given above), -NR^{ab1}-CO-O- (wherein R^{ab1} has the same meaning given above), -CH₂SO₂- or -CH₂S-, R^{aa} is a hydrocarbon group selected from Group a2 which may be substituted by member(s) selected from Group al or a heterocyclic group selected from Group a6 which may be substituted by member(s) selected from Group a1].

(I-7) a compound as shown in the above (I-3), wherein the group represented by the formula-A^{a}R^{a1}R^{a2} is a group represented by the formula: 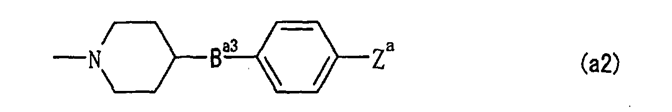 (wherein B^{a3} is-CH₂-, -SO₂-, -SO-, -S-, -O-, -CO-, -NR^{ab1}-SO₂- (wherein R^{ab1} is a hydrogen atom, a C₁₋₄ alkyl group, a C₂₋₆ alkenyl group, a C₂₋₆ alkynyl or a C₃₋₆ cycloalkyl group), -NR^{ab1}-CO-, -NR^{ab1}-CO-O- (wherein R^{ab1} has the same meaning given above), Z^{a} is a halogen, SO₂NR^{ab3}R^{ab4} (wherein each of R^{ab3} and R^{ab4} is (1) a C₁₋₆ alkyl which may be substituted by halogen(s), hydroxyl (s) or C₁₋₆ alkoxy(s), (2) a C₃₋₈ cycloalkyl which may be substituted by halogen(s) or C₁₋₆ alkoxy (s), (3) a C₁₋₆ alkoxy or (4) a hydrogen atom or, R^{ab3} and R^{ab4} are combine each other together with nitrogen to form a cyclic-amino group), SO₂R^{ab5}, (wherein R^{ab5} is (1) a C₁₋₆ alkyl group which may be substituted by halogen(s), hydroxyl(s) or C₁₋₆ alkoxy (s), (2) a C₃₋₈ cycloalkyl group which may be substituted by halogen (s) or C₁₋₆ alkoxy(s)), a CONR^{ab3}R^{ab4} (wherein each of R^{ab3} and R^{ab4} has the meaning given above) or -NR^{ab7}-SO₂R^{ab6} (wherein R^{ab6} is (1) a C₁₋₆ alkyl group which may be substituted by halogen(s) or C₁₋₆ alkoxy (s), (2) a C₃₋₈ cycloalkyl group which may be substituted by halogen(s) or C₁₋₆ alkoxy(s), R^{ab7} is (1) a C₁₋₆ alkyl group which may be substituted by halogen (s) or C₁₋₆ alkoxy (s), (2) a C₃₋₈ cycloalkyl group which may be substituted by halogen(s) or C₁₋₆ alkoxy(s) or (3) a hydrogen atom), a C₁₋₆ alkoxy group, an amino group which may be substituted by C₂₋₄ alkanoyl(s), nitro(s), cyano(s), tetrazolyl(s) or morpholinyl(s)).

(I-8) a compound as shown in the above (I-1), wherein R^{a3} is a C₆₋₁₄ aryl group which may be substituted by member (s) selected from Group a1.

(I-9) a compound as shown in the above (I-1), wherein R^{a3} is a phenyl group which may. be substituted by member (s) selected from Group a1.

(I-10) a compound, wherein E^{a} is -CH₂CH₂-, -CH₂CH₂CH₂-, -CH₂CH₂CH₂CH₂- or -CH₂CH₂CH₂CH₂CH₂-.

(I-11) a compound, wherein E^{a} is-CH₂CH₂CH₂-.

(I-12) a compound, wherein G^{a2} is CO, SO₂, CONH or OCO.

(I-13) a compound, wherein G^{a2} is CO or NHCO.

(I-14) a compound, wherein G^{a2} is CO.

(I-15) a compound, wherein J^{a} is methine.

(I-16) a compound, wherein G^{a1} is CO or SO₂.

(I-17) a compound as shown in the above (I-1),
wherein R^{a4} is a hydrocarbon group selected from Group a2 which may be substituted by member (s) selected from Group a1, a heterocyclic group selected from Group a6 which may be substituted by member(s) selected from Group a1, a C₁₋₆ alkoxy group which may be substituted by member(s) selected from Group a7, or an amino group which may be substituted by member(s) selected from Group a9.

(I-18) a compound, wherein R^{a4} is a C₁₋₃ alkyl.

(I-19) a compound, wherein R^{a4} is methyl.

(I-20) a compound, wherein each of Q^{a} and R^{a} is -CH₂CH₂- .

(I-21) a compound, wherein na is zero.

(I-22) a compound represented by the formula: 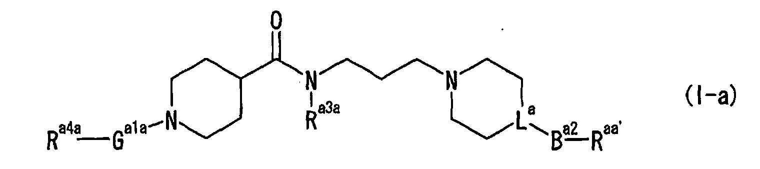 [wherein R^{a4a} is (1) a C₁₋₆ alkyl group which may be substituted by halogen(s), C₁₋₆ alkoxy(s), oxo(s), amino(s), phenyl(s), pyridyl(s) or tetrazolyl(s), (2) a C₁₋₆ alkenyl group, (3) a C₃₋₈ cycloalkyl group which may be substituted by halogen (s), C₁₋₆ alkyl(s) or C₁₋₆ alkoxy(s), (4) a phenyl group which may be substituted by halogen(s), C₁₋₆ alkyl (s), C₁₋₆ alkoxy(s), nitro(s), cyano(s), hydroxyl (s), C₁₋₄ alkanoyl-amino(s), carbamoyl(s) or sulfamoyl(s), (5) an amino group which may be substituted by C₁₋₆ alkyl(s), (6) a C₁₋₆ alkoxy group which may be substituted by phenyl (s), (7) a C₃₋₈ cycloalkyl-oxy group (8) a heterocyclic group which may be substituted by halogen (s), C₁₋₆ alkyl (s) or hydroxyl (s) *,* G^{a1a} is CO or SO₂, R^{a3a} is a C₆₋₁₀ aryl group which may be substituted by (1) halogen (s), (2) C₁₋₆ alkyl(s) which may be substituted by halogen(s), (3) C₁₋₆ alkoxy(s) which may be substituted by halogen(s), (4) C₁₋₆ alkylthio(s), or (5) C6-10 aryl group which may be substituted by C₁₋₆ alkyl-sulfonyl(s), L^{a} is methine or a nitrogen atom, B^{a2} is a bond, -CH₂-, -SO₂-, -SO-, -S-, -O-, -CO-, -NR^{ab1}-SO₂- (wherein R^{ab1} has the same meaning given above), -CH(OH)-, -NR^{ab2}- (wherein R^{ab2} is a hydrogen atom or a C₂₋₄ alkanoyl group), -NR^{ab1}-CO- (wherein R^{ab1} has the same meaning given above), -NR^{ab1}-CO-O- (wherein R^{ab1} has the same meaning given above), -CH₂SO₂- or -CH₂S-, R^{aa}' is ① an aromatic hydrocarbon group which may be substituted by halogen (s), SO₂NR^{ab3}R^{ab4} (wherein each of R^{ab3} and R^{ab4} has the same meaning given above), SO₂R^{ab5} (wherein R^{ab5} is (1) a C₁₋₆ alkyl group which may be substituted by halogen(s), hydroxyl(s) or C₁₋₆ alkoxy(s), (2) a C₃₋₈ cycloalkyl group which may be substituted by halogen(s) or C₁₋₆ alkoxy(s)), CONR^{ab3}R^{ab4} (wherein R^{ab3} and R^{ab4} have the same meanings given above) or-NR^{ab7}-SO₂R^{ab6} (wherein R^{ab6} has the same meaning given above), a C₁₋₆ alkoxy, an amino which may be substituted by a C₂₋₄ alkanoyl (s), a nitro, a cyano, s tetrazolyl or a morpholinyl or ② an aromatic heterocyclic group which may be substituted by substituent(s) selected from the above mentioned substituents of aromatic hydrocarbon group] or salt thereof.

(I-23) a compound as shown in the above (I-22), wherein R^{a3a} is a phenyl group which may be substituted by halogen(s), trifluoromethyl(s) or C₁₋₆ alkyl (s).

(I-24) a compound as shown in the above (I-22), wherein L^{a} is methine.

(I-25) a compound as shown in the above (I-22), wherein B^{a2} is-CH₂-, -SO₂-, -SO-, -S-, -O-, -CO-, -NR^{ab1}-SO₂-, - NR^{ab1}-CO- or NR^{ab1}-CO-O- (wherein R^{ab1} has the same meaning given above).

(I-26) a compound as shown in the above (I-22), wherein R^{aa}' is a phenyl group which may be substituted by (1) halogen(s), (2) SO₂R^{ae} (wherein R^{ae} is a C₁₋₆ alkyl group or a C₃₋₈ cycloalkyl group ), (3) N(R^{ad}) SO₂R^{ae} (wherein R^{ad} is a hydrogen atom or a C₁₋₄ alkyl group, R^{ae} has the same meaning given above), (4) SO₂NR^{af}R^{ag} (wherein each of R^{af} and R^{ag} is a hydrogen atom or a C₁₋₆ alkyl group or R^{af} and R^{ag} may combine each other together with a nitrogen atom to form a cyclic-amino group) or (5) CONR^{af}R^{ag} (wherein R^{af} and R^{ag} are the same or different and independently a hydrogen atom or a C₁₋₆ alkyl group or, R^{af} and R^{ag} combine each other together with a nitrogen atom to form a cyclic-amino group).

(I-27) a compound as shown in the above (I-22), wherein B² is SO₂, CH₂ or N (R^{ad})-SO₂ (wherein R^{ad} is a hydrogen atom or a C₁₋₄ alkyl) ; R^{aa'} is a phenyl which may be substituted by (1) halogen(s), (2) SO₂R^{ae} (wherein R^{ae} is a C₁₋₆ alkyl or a C₃₋₈ cycloalkyl ), (3) N(R^{ad})SO₂R^{ae} (wherein R^{ad} is a hydrogen atom or a C₁₋₄ alkyl, and R^{ae} has the same meaning given above), (4) SO₂NR^{af}R^{ag} (wherein each of R^{af} and R^{ag} is a hydrogen atom or a C₁₋₆ alkyl or R^{af} and R^{ag} may combine each other together with a nitrogen atom to form a cyclic-amino group) or (5) CONR^{af}R^{ag} (wherein each of R^{af} and R^{ag} is a hydrogen atom or a C₁₋₆ alkyl group or R^{af} and R^{ag} may combine each other together with a nitrogen atom to form a cyclic-amino group); R^{a3a} is a phenyl group substituted by one or two members selected from the group of halogen atom and a C₁₋₄ alkyl.

(I-28) a compound as shown in the above (I-22), wherein G^{a1a} is SO₂ or CO, L^{a} is methine, B ^{a 2} is SO₂ or CH₂, R^{aa}' is a group represented by the formula: 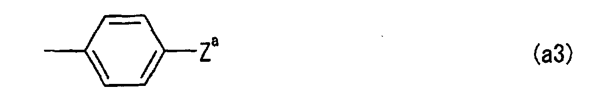 (wherein Z^{a} is a C₁₋₄ alkyl-sulfonyl group, a sulfamoyl group which may be substituted by a C₁₋₄ alkyl or a carbamoyl group); R^{a3a} is a phenyl group which may be substituted by one or two members selected from the group consisting of halogen atom(s) or C₁₋₄ alkyl(s); and R^{a4a} is methyl.

Examples of the "hydrocarbon group" in the "a hydrocarbon group which may be substituted" represented by R^{b1} preferably include, for example, an aliphatic chain hydrocarbon group, an alicyclic hydrocarbon group and an aryl group etc. Examples of the aliphatic chain hydrocarbon group include a C₁₋₆ alkyl group, for example, methyl, ethyl, n-propyl, isopropyl, n-butyl, isobutyl, sec-butyl, tert-butyl, n-pentyl, isopentyl, neopentyl, 1-methylpropyl, n-hexyl, isohexyl etc. Examples of the "alicyclic hydrocarbon group" preferably include a C₃₋₈ cycloalkyl group such as cyclopropyl, cyclobutyl, cyclopentyl, cyclohexyl, cycloheptyl, cyclooctyl, etc.

Examples of the aryl group preferably include a C₆₋₁₄ aryl group such as phenyl, naphthyl (1-naphthyl, 2-naphthyl), etc.

Examples of the "substituent(s)" in the "hydrocarbon group which may be substituted" represented by R^{b1} include a hydrocarbon group which may be substituted, a heterocyclic group which may be substituted, a halogen atom (e.g., fluorine, chlorine, bromine, iodine), a C₁₋₄ alkoxy group which may be substituted, a C₁₋₄ alkylthio group which may be substituted, a C₂₋₆ alkoxycarbonyl group which may be substituted, a C₁₋₆ alkanoyl group which may be substituted, an amino group which may be substituted, a nitro group, a cyano group, a carbamoyl group which may be substituted, a sulfamoyl group which may be substituted, an acyl group derived from a sulfonic acid, etc.

Examples of the "hydrocarbon group(s)" in the "hydrocarbon group which may be substituted" are those similar to the "hydrocarbon group" of the "hydrocarbon group which may be substituted", which is represented by R^{b1} Among these substituents, a C₁₋₆ alkyl group, a C₃₋₈ cycloalkyl group, a C₆₋₁₄ aryl group are preferred. These examples may include the substituents as mentioned above for R^{b1}. Examples of the "substituents" in the "hydrocarbon group which may be substituted" include, for example, a lower alkoxy group (e.g., a C₁₋₆ alkoxy group such as methoxy, ethoxy, propoxy, etc.), a halogen atom (e.g., fluorine, chlorine, bromine, iodine etc.), a lower alkyl group (e.g., a C₁₋₆ alkyl group such as methyl, ethyl, propyl, etc.), a lower alkynyl group (e.g., a C₁₋₄ alkynyl group such as vinyl, 1-propenyl, 2-propenyl, isopropenyl, butenyl, isobutenyl, etc.), an amino group, a hydroxy group, a cyano group, an amidino group etc. The hydrocarbon in "hydrocarbon which may be substituted" may have 1 to 3 substituent(s) as described above at any possible position. Examples of the heterocyclic group in the "heterocyclic group which may be substituted" (the substituent in the "hydrocarbon group which may be substituted, which is represented by R^{b1}") include, for example, an aromatic heterocyclic group, saturated or unsaturated non-aromatic heterocyclic group (alicyclic heterocyclic group) etc., which contains at least one heteroatom(s) (preferably 1 to 4 heteroatom(s), more preferably, 1 to 2 heteroatom(s)) consisting of 1 to 3 kind(s) of heteroatom(s) (preferably 1 to 2 kinds of heteroatom (s)) selected from an oxygen atom, a sulfur atom, a nitrogen atom, etc. as ring constituting atom(s).

Examples of the "aromatic heterocyclic group" include an aromatic monocyclic heterocyclic group such as a 5 or 6-membered aromatic monocyclic heterocyclic group (e.g., furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.); an aromatic fused heterocyclic group such as a 8 to 12-membered aromatic fused heterocyclic group (e.g., benzofuranyl, isobenzofuranyl, benzothienyl, indolyl, isoindolyl, 1H-indazolyl, benzindazolyl, benzoxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.); etc., preferably, a heterocyclic group consisting of the above-mentioned 5- or 6-membered aromatic monocyclic heterocyclic group fused with a benzene ring or heterocyclic group consisting of the above-mentioned 5- or 6-membered aromatic monocyclic heterocyclic group fused with the same or different above-mentioned 5- or 6-membered aromatic monocyclic heterocyclic group, etc.

Examples of the "non-aromatic heterocyclic group" include a 3 to 8-membered (preferably 5 or 6-membered) saturated or unsaturated (preferably saturated) non-aromatic heterocyclic group (aliphatic heterocyclic group) such as oxiranyl, azetidinyl, oxetanyl, thiethanyl, pyrrolidinyl, tetrahydrofuryl, thiolanyl, piperidinyl, tetrahydropyranyl, morpholinyl, thiomorpholinyl, piperazinyl, etc.

Examples of the "substituent(s)" of the "heterocyclic group which may be substituted" (substituent(s) of the hydrocarbon group which may be substituted, which is represented by R^{b1}) are those similar to the "substituent(s)" of the "hydrocarbon group which may be substituted" that is(are) the "substituent (s)" of the hydrocarbon group which may be substituted, which is represented by R^{b1}.

Examples of "C₁₋₄ alkoxy group" in the "C₁₋₄ alkoxy group which may be substituted" include, for example, methoxy, ethoxy, n-propoxy, isopropoxy, n-butoxy, isobutoxy, tert-butoxy, etc. Example of "C₁₋₄ an alkylthio group" in the "C₁₋₄ an alkylthio group which may be substituted" include, for example, methylthio, ethylthio, n-propylthio, isopropylthio, n-butylthio, isobutylthio, tert-butylthio, etc. Example of the "C₂₋₆ alkoxycarbonyl group" in "C₂₋₆ alkoxycarbonyl group which may be substituted" include, for example, methoxycarbonyl, ethoxycarbonyl, n-propoxycarbonyl, isopropoxycarbonyl, n-butoxycarbonyl, isobutoxycarbonyl, tert-butoxycarbonyl, n-pentyloxycarbonyl, etc.
Examples of the "C₁₋₆ alkanoyl group" in the "C₁₋₆ alkanoyl group which may be substituted" include, for example, formyl, acetyl, propionyl, pivaloyl etc. Examples of the substituent in the "C₁₋₄ alkoxy group which may be substituted", "C₁₋₄ alkylthio group which may be substituted", and "C₁₋₆ alkoxycarbonyl group which may be substituted", "C₁₋₆ alkanoyl group which may be substituted" are those similar to the substituent(s) of the "hydrocarbon group which may be substituted", which are the substituent(s) of the "hydrocarbon group which may be substituted" represented by R^{b1}.
Examples of the substituent(s) of the "amino group which may be substituted" include, for example, a lower alkyl group (e.g., a C₁₋₆ alkyl group such as methyl, ethyl, propyl, isopropyl, butyl, isobutyl, t-butyl, pentyl, hexyl, etc.), an acyl group derived from a carboxylic acid (e.g., a C₁₋₆ alkanoyl such as formyl, acetyl, propionyl, pivaloyl, etc.), a C₇₋₁₅ arylcarbonyl such as benzoyl, etc., an acyl group derived from a sulfonic acid (e.g., a C₁₋₆ alkylsulfonyl such as methylsulfonyl, ethylsulfonyl, etc.), an optionally halogenated C₂₋₆ alkoxycarbonyl (e.g., trifluoromethoxycarbonyl, 2,2,2-trifluoroethoxycarbonyl, trichloromethoxycarbonyl, 2,2,2-trichloroethoxycarbonyl, etc.), etc. In addition, the "amino group" in the "amino group which may be substituted" may be substituted with an imidoyl group which may be substituted (e.g., a C₁₋₆ alkylimidoyl, formylimidoyl, amidino, etc.), etc. Alternatively, two substituents of the amino group may form a cyclic amino group together with a nitrogen atom Examples of said cyclic amino group include e.g. 3 to 8-membered (preferably 5 or 6-membered) cyclic amino group such as 1-azetidinyl, 1-pyrrolidinyl, 1-piperidinyl, 4-morpholinyl, 1-piperazinyl and 1-piperazinyl which may have at the 4-position a lower alkyl group (e.g., a C₁₋₆ alkyl group such as methyl, ethyl, propyl, isopropyl, butyl, t-butyl, pentyl, hexyl, etc.), an aralkyl group (e.g. a C₇₋₁₀ aralkyl group such as benzyl, phenethyl, etc.), an aryl group (e.g. a C₆₋₁₀ aryl group such as phenyl, 1-naphthyl, 2-naphthyl, etc.), etc.

Examples of the "carbamoyl group which may be substituted" include unsubstituted carbamoyl, a N-mono-substituted carbamoyl group and a N,N-di-substituted carbamoyl group.

The "N-mono-substituted carbamoyl group" is a carbamoyl group having one substituent on the nitrogen atom and the substituent include, for example, a lower alkyl group (e.g., a C₁₋₆ alkyl group such as methyl, ethyl, propyl, isopropyl, butyl, isobutyl, t-butyl, pentyl, hexyl, etc.), a cycloalkyl group (e.g., a C₃₋₆ cycloalkyl group such as cyclopropyl, cyclobutyl, cyclopentyl, cyclohexyl, etc.), an aryl group (e.g., a C₆₋₁₀ aryl group such as phenyl, 1-naphthyl, 2-naphthyl, etc.), an aralkyl group (e.g., a C₇₋₁₀ aralkyl group, preferably a phenyl-C₁₋₄ alkyl group such as benzyl, phenethyl, etc.), a heterocyclic group (e.g., the above described "heterocyclic group" as the substituent of the "hydrocarbon group which may be substituted" represented by R^{b1}, etc.), etc. The lower alkyl group, the cycloalkyl group, the aryl group, the aralkyl group and the heterocyclic group as described above may have substituent(s), and the substituent(s) include, for example, a hydroxy group, an amino group which may be substituted [the amino group may have 1 or 2 substituent(s) (e.g. a lower alkyl group (e.g., a C₁₋₆ alkyl group such as methyl, ethyl, propyl, isopropyl, butyl, isobutyl, t-butyl, pentyl, hexyl, etc.), an acyl group (e.g., a C₁₋₆ alkanoyl such as formyl, acetyl, propionyl, pivaloyl, etc., an arylcarbonyl such as benzoyl, etc., a C₁₋₆ alkylsulfonyl such as methylsulfonyl, ethylsulfonyl, etc.), etc.)], a halogen atom (e.g., fluorine, chlorine, bromine, iodine, etc.), a nitro group, a cyano group, a lower alkyl group which may be substituted with 1 to 5 halogen atom(s) (e.g., fluorine, chlorine, bromine, iodine, etc.), a lower alkoxy group which may be substituted with 1 to 5 halogen atom(s) (e.g., fluorine, chlorine, bromine, iodine, etc.), etc. The lower alkyl group includes, e.g. a C₁₋₆ alkyl group such as methyl, ethyl, n-propyl, isopropyl, n-butyl, isobutyl, sec-butyl, tert-butyl, pentyl, hexyl, etc. and in particular methyl, ethyl, etc. are preferable. Said lower alkoxy group include e.g. a C₁₋₆ alkoxy group such as methoxy, ethoxy, n-propoxy, isopropoxy, n-butoxy, isobutoxy, sec-butoxy, tert-butoxy, etc. and in particular methoxy, ethoxy, etc. are preferable. The above described lower alkyl group, cycloalkyl group, aryl group, aralkyl group and heterocyclic group may have 1, 2 or 3 (preferably 1 or 2) substituent(s).

The "N,N-di-substituted carbamoyl group" is a carbamoyl group having two substituents on the nitrogen atom Examples of one of the substituents include the same as those of the above described "N-mono-substituted carbamoyl group" and examples of the other substituent include e.g. a lower alkyl group (e.g., a C₁₋₆ alkyl group such as methyl, ethyl, propyl, isopropyl, butyl, t-butyl, pentyl, hexyl, etc.), a C₃₋₆ cycloalkyl group (e.g., cyclopropyl, cyclobutyl, cyclopentyl, cyclohexyl, etc.), a C₇₋₁₀ aralkyl group (e.g., benzyl, phenethyl, etc., preferably phenyl-C₁₋₄ alkyl group, etc.), etc. In addition, two substituents of the "N,N-di-substituted carbamoyl group" may form a cyclic amino group together with a nitrogen atom. Examples of said cyclic aminocarbonyl group include, e.g., 3 to 8-membered (preferably 5 or 6-membered) cyclic aminocarbonyl group such as 1-azetidinylcarbonyl, 1-pyrrolidinylcarbonyl, 1-piperidinylcarbonyl, 4-morpholinylcarbonyl, 1-piperazinylcarbonyl and 1-piperazinylcarbonyl which may have a lower alkyl group (e.g., a C₁₋₆ alkyl group such as methyl, ethyl, propyl, isopropyl, butyl, t-butyl, pentyl, hexyl, etc.), an aralkyl group (e.g., a C₇₋₁₀ aralkyl group such as benzyl, phenethyl, etc.), an aryl group (e.g., a C₆₋₁₀ aryl group such as phenyl, 1-naphthyl, 2-naphthyl, etc.), etc. at the 4-position.

Examples of the "sulfamoyl group which may be substituted" include an unsubstituted sulfamoyl group, a N-mono-substituted sulfamoyl group and a N,N-di-substituted sulfamoyl group.

The "N-mono-substituted sulfamoyl group" is a sulfamoyl group having one substituent at the nitrogen atom, and examples of the substituent include those mentioned for the substituents of N-mono-substituted carbamoyl group The "N,N-di-substituted sulfamoyl group" is a sulfamoyl group having two substituents at the nitrogen atom, and examples of the substituents include those mentioned as the substituents of the N,N-di-substituted carbamoyl group Examples of the "acyl group derived from a sulfonic acid" include a sulfonyl group substituted by a hydrocarbon group, and preferably, include an acyl group such as C₁₋₁₀ alkylsulfonyl, C₂₋₆ alkenylsulfonyl, C₂₋₆ alkynylsulfonyl, C₃₋₉ cycloalkylsulfonyl, C₃₋₉ cycloalkenylsulfonyl, C₆₋₁₄ arylsulfonyl, C₇₋₁₀ aralkylsulfonyl. Examples of the C₁₋₂₀ alkyl include, for example, methyl, ethyl, propyl, isopropyl, butyl, isobutyl, t-butyl, pentyl, hexyl, heptyl, octyl, etc. Examples of the C₂₋₆ alkenyl include, for example, vinyl, allyl, 1-propenyl, isopropenyl, 2-butenyl, 3-butenyl, 2-hexenyl, etc. Examples of C₂₋₆ alkynyl include, for example, ethynyl, 2-propynyl, 2-butynyl, 5-hexynyl, etc.

Examples of the C₃₋₉ cycloalkyl include, for example, cyclopropyl, cyclobutyl, cyclopentyl, cyclohexyl, cyclooctyl, etc. Examples of the C₃₋₉ cycloalkenyl include, for example, 1-cyclopenten-1-yl, 2-cyclopenten-1-yl, 3-cyclopenten-1-yl, 3-cyclohexen-1-yl, 3-cycloocten-1-yl, etc. Examples of the C₆₋₁₄ aryl include, for example, phenyl, 1-naphthyl, 2-naphthyl, etc. Examples of the C₇₋₁₀ aralkyl-sulfonyl include, for example, benzyl, phenethyl, etc. These hydrocarbon groups which are the substituents of the sulfonyl may be substituted. Examples of these substituents include, for example, hydroxy group, amino group which may be substituted [the amino group may be substituted by one or two lower alkyl group (e.g., C₁₋₆ alkyl such as methyl, ethyl, propyl, isopropyl, butyl, isobutyl, t-butyl, pentyl, hexyl, etc.), an acyl group (e.g., a C₁₋₆ alkanoyl such as formyl, acetyl, propionyl, pivaloyl, etc., an aryl carbonyl such as benzoyl, etc., a C₁₋₆ alkylsulfonyl such as methylsulfonyl, ethylsulfonyl, etc.)], a halogen atom (for example, fluorine, chlorine, bromine, iodine, etc.), nitro group, cyano group, a lower alkyl which may be substituted by 1 to 5 halogen atom(s) (e.g. fluorine, chlorine, bromine, iodine, etc.), a lower alkoxy group which may be substituted by 1 to 5 halogen atom(s) (e.g. fluorine, chlorine, bromine, iodine, etc.). Examples of the lower alkyl group include, for example, a C₁₋₆ alkyl group such as methyl, ethyl, n-propyl, isopropyl, n-butyl, isobutyl, sec-butyl, tert-butyl, pentyl, hexyl, etc., and preferably include methyl, ethyl, etc. The lower alkoxy group includes, for example, a C₁₋₆ alkoxy group such as methoxy, ethoxy, n-propoxy, isopropoxy, n-butoxy, isobutoxy, sec-butoxy, tert-butoxy, etc, and preferably include methoxy, ethoxy, etc. Preferably, one, two or three (preferably one or two) from these substituents are used, wherein the substituents may be the same or different "Cyclic hydrocarbon group" in the "cyclic hydrocarbon group which may be substituted" represented by R^{b2} include alicyclic hydrocarbon group and aryl group.

Examples of the "alicyclic hydrocarbon group" include, for example, a saturated or unsaturated alicyclic hydrocarbon group such as a cycloalkyl group, a cycloalkenyl group, a cycloalkanedienyl group, etc. Examples of the "cycloalkyl group" include, for example, a C₃₋₉ cycloalkyl (preferably, a C₃₋₈ cycloalkyl etc.) such as cyclopropyl, cyclobutyl, cyclopentyl, cyclohexyl, cycloheptyl, cyclooctyl, cyclononyl, etc., and a fused ring such as 1-indanyl, 2-indanyl, etc. Examples of the "cycloalkenyl group" include, for example, a C₃₋₆ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl, etc. Examples of the "cycloalkanedienyl group" include, for example, a C₄₋₆ cycloalkanedienyl group such as 2,4-cyclopentanedien-1-yl, 2,4-cyclohexanedien-1-yl, 2,5-cyclohexanedien-1-yl, etc. In particular, a C₃₋₈ cyrloalkyl group such as cyclohexyl is preferable.
Examples of the "aryl group" include, for example, a monocyclic or fused polycyclic aromatic hydrocarbon group. Among others, a C₆₋₁₄ aryl group such as phenyl, naphthyl, anthryl, phenanthryl, acenaphthylenyl, 4-indanyl, 5-indanyl, etc. are preferable. In particular, phenyl, 1-naphthyl, 2-naphthyl, etc. are preferable.

Examples of the "substituent(s)" in the "cyclic hydrocarbon group which may be substituted" represented by R^{b2} are those similar to the "substituent" of the "hydrocarbon group which may be substituted" described as the substituent (s) of the "hydrocarbon group which may be substituted", which are represented by R^{b1}.

Examples of the "heterocyclic group which may be substituted" of R^{b2} are those similar to the "heterocyclic group which may be substituted" described as the substituent(s) of the "hydrocarbon group which may be substituted", which are represented by R^{b1}.

The halogen atom represented by R^{b3} include, for example, fluorine, chlorine, bromine, iodine, etc.

The "carbamoyl group which may be substituted", "sulfamoyl group which may be substituted" and "acyl group derived from a sulfonic acid" represented by R^{b3} are those similar to the "carbamoyl group which may be substituted", "sulfamoyl group which may be substituted" and "acyl group derived from a sulfonic acid", which are represented by R^{b1}. Examples of the "C₁₋₄ alkyl group" of the "C₁₋₄ alkyl group which may be substituted" represented by R^{b3} include, for example, methyl, ethyl, n-propyl, isopropyl, n-butyl, isobutyl, tert-butyl. Examples of the "C₁₋₄ alkoxy group" of the "C₁₋₄ alkoxy group which may be substituted" represented R^{b3} include, for example, methoxy, ethoxy, propoxy, n-butoxy, isobutoxy, tert-butoxy.

Example of the substituent(s) in the "C₁₋₄ alkyl group which may be substituted" and "C₁₋₄ alkoxy group which may be substituted", which is represented by R^{b3} are those similar to the "substituent(s)" of the "hydrocarbon group which may be substituted" which is(are) the "substituent(s)" of "the hydrocarbon group which may be substituted", which is represented by R^{b1}.

Examples of the substituents of "amino group which may be substituted" represented by R^{b3} include, for example, a lower alkyl group (e.g., C₁₋₆ alkyl group such as methyl, ethyl, propyl, isopropyl, butyl, isobutyl, t-butyl, pentyl, hexyl, etc.), an acyl group derived from carboxylic acid (e.g., C₁₋₆ alkanoyl such as formyl, acetyl, propionyl, pivaloyl, etc.), for example, C₇₋₁₅ arylcarbonyl such as benzoyl, etc, an acyl group derived from sulfonic acid (e.g., C₁₋₆ alkylsulfonyl such as methylsulfonyl, ethylsulfonyl, etc.), an optionally halogenated C₁₋₆ alkoxycarbonyl (e.g., trifluoromethoxycarbonyl, 2,2,2-trifluoroethoxycarbonyl, trichloromethoxycarbonyl, 2,2,2-trichloroethoxycarbonyl, etc.), etc. In addition, the "amino group" of the "amino group which may be substituted" may be substituted with an imidoyl group which may be substituted (e.g., a C₁₋₆ alkylimidoyl, formylimidoyl, amidino, etc.), etc. and two substituents of the "amino group" may form a cyclic amino group together with a nitrogen atom. Examples of said cyclic amino group include, for example, 3 to 8-membered (preferably, 5 or 6-membered) cyclic amino group such as 1-azetidinyl, 1-pyrrolidinyl, 1-piperidinyl, 4-morpholinyl, 1-piperazinyl and 1-piperazinyl which may have a lower alkyl group (e.g., a C₁₋₆ alkyl group such as methyl, ethyl, propyl, isopropyl, butyl, t-butyl, pentyl, hexyl, etc.), an aralkyl group (e.g., a C₇₋₁₀ aralkyl group such as benzyl, phenethyl, etc.), an aryl group (e.g., a C₆₋₁₀ aryl group such as phenyl, 1-naphthyl, 2-naphthyl, etc.), etc. at the 4-position.
Examples of the leaving group represented by X include, for example, a halogen atom (e.g., a chlorine atom, a bromine atom, an iodine atom, etc.), an alkyl or aryl sulfonyloxy group (e.g., methanesulfonyloxy, trifluoromethanesulfonyloxy, ethanesulfonyloxy, benzenesulfonyloxy, p-toluenesulfonyloxy, etc.), etc.

Examples of the salt of a compound of the formula (II) of the present invention include a salt with an acid, for example, a salt with inorganic acid (e.g., hydrochloric acid salt, sulfuric acid salt, hydrobromic acid salt, phosphoric acid salt, etc.), a salt of an organic acid (e.g., acetic acid salt, trifluoroacetic acid salt, succinic acid salt, maleic acid salt, fumaric acid salt, propionic acid salt, citric acid salt, tartaric acid salt, lactic acid salt, oxalic acid salt, methanesulfonic acid salt, p-toluenesulfonic acid salt, etc.), etc., a salt with a base (e.g., an alkali metal salt such as potassium salt, sodium salt, lithium salt, etc., an alkaline earth metal salt such as calcium salt, magnesium salt, etc., ammonium salt, a salt with an organic base such as ammonium salt, trimethylamine salt, triethylamine salt, tert-butyl dimethyl amine salt, dibenzyl methylamine salt, benzyl dimethylamine salt, N,N-dimethylaniline salt, pyridine salt, quinoline salt, etc.).

The compound of the formula (II) or salt thereof may also be hydrated. Hereinafter the compound of the formula (II), its salt and its hydrate are referred to as Compound (II).

Among the compounds represented by the formula (II) or salts thereof (hereinafter referred to as Compounds (II)), the following compounds are preferable.

(II-1) the compound wherein R^{b3} is a halogen atom, a C₁₋₄ alkyl group which may be substituted, a C₁₋₄ alkoxy group which may be substituted, an amino group which may be substituted, a nitro group or a cyano group,

(II-2) the compound wherein R^{b1} is an alicyclic hydrocarbon group which may be substituted or an aryl group which may be substituted,

(II-3) the compound wherein R^{b1} is a hydrocarbon group which may be substituted by 1 to 4 substituent(s) selected from 1) a hydrocarbon group which may be substituted, 2) an heterocyclic group which may be substituted, 3) a C₁₋₄ alkoxy group which may be substituted, 4) a C₁₋₄ alkylthio group which may be substituted, 5) a C₂₋₆ alkoxycarbonyl group which may be substituted, 6) a C₁₋₆ alkanoyl group which may be substituted, 7) an amino group which may be substituted, 8) a cyclic amino group, 9) a halogen atom, 10) a nitro group, 11) a cyano group, 12) a carbamoyl group which may be substituted, 13) a sulfamoyl group which may be substituted and 14) an acyl group derived from a sulfonic acid,

(II-4) the compound wherein R^{b1} is a hydrocarbon group which may be substituted by 1 to 4 substituent(s) selected from 1) a hydrocarbon group which may be substituted, 2) a heterocyclic group which may be substituted, 3) a C₁₋₄ alkoxy group which may be substituted, 4) a C₁₋₄ alkylthio group which may be substituted, 5) a C₂₋₆ alkoxycarbonyl group which may be substituted, 6) an amino group which may be substituted, 7) a halogen atom, 8) a nitro group and 9) a cyano group,

(II-5) the compound wherein R^{b1} is a hydrocarbon group which may be substituted by 1 to 4 substituent(s) selected from 1) a hydrocarbon group which may be substituted, 2) a heterocyclic group which may be substituted, 3) a C₁₋₄ alkylthio group which may be substituted, 4) a C₂₋₆ alkoxycarbonyl group which may be substituted, 5) an amino group which may be substituted, 6) a halogen atom and 7) a nitro group,

(II-6) the compound wherein R^{b2} is an cyclic hydrocarbon group which may be substituted,

(II-7) the compound wherein R^{b3} is a halogen, a carbamoyl group which may be substituted, a sulfamoyl group which may be substituted or an acyl group derived from a sulfonic acid,

(II-8) the compound wherein R^{b3} is a halogen,

(II-9) the compound wherein R^{b4} is a hydrogen atom,

(II-10) the compound wherein n is 0,

(II-11) the compound wherein R^{b1} is a hydrocarbon group selected from Group 3 which may be substituted by member(s) selected from Group 1; R^{b2} is a cyclic hydrocarbon group selected from Group 10 which may be substituted by member(s) selected from Group 2, or a heterocyclic group selected from Group 4 which may be substituted by member(s) selected from Group 2; R^{b3} is a halogen atom, a carbamoyl group, a N-mono-substituted carbamoyl group which may be substituted by a member selected from Group 11, a N,N-di-substituted carbamoyl group which may be substituted by a member selected from Group 11 and a member selected from Group 14, a cyclic aminocarbonyl group selected from Group 17, a sulfamoyl group, N-mono-substituted sulfamoyl group which may be substituted by a member selected from Group 11, a N,N-di-substituted sulfamoyl group which may be substituted by a member selected from Group 11 and a member selected from Group 14, a cyclic aminosulfonyl group selected from Group 20, an acyl group derived from a sulfonic acid selected from Group 15, a C₁₋₄ alkyl group which may be substituted by member(s) selected from Group 2, a C₁₋₄ alkoxy group which may be substituted by member(s) selected from Group 2, an amino group which may be substituted by member(s) selected from Group 8, a cyclic amino group selected from Group 9, a nitro group or a cyano group.

### Group 1

1) a hydrocarbon group selected from Group 3 which may be substituted by member(s) selected from Group 2, 2) a heterocyclic group selected from Group 4 which may be substituted by member(s) selected from Group 2, 3) a C₁₋₄ alkoxy group which may be substituted by member(s) selected from Group 2, 4) a C₁₋₄ alkylthio group which may be substituted by member(s) selected from Group 2, 5) a C₂₋₆ alkoxycarbonyl group which may be substituted by member(s) selected from Group 2, 6) a C₁₋₆ alkanoyl group, 7) an amino group which may be substituted by member(s) selected from Group 8, 8) a cyclic amino group selected from Group 9, 9) a halogen atom, 10) a nitro group, 11) a cyano group, 12) a carbamoyl group, 13) a mono-substituted carbamoyl group which is substituted by a member selected from Group 11, 14) di-substituted carbamoyl group which is substituted by a member selected from Group 11 and a member selected Group 14, 15) a cyclic amino carbamoyl group selected from Group 17, 16) a sulfamoyl group, 17) a N-mono substituted sulfamoyl group which is substituted by a member selected from Group 11, 18) a N,N-di-substituted sulfamoyl group which is substituted by a member selected from Group 11 and a member selected Group 14, 19) an acyl group derived from a sulfonic acid selected from Group 19

### Group 2

1) a C₁₋₆ alkoxy group, 2) a halogen atom, 3) a C₁₋₆ alkyl group, 4) a C₁₋₄ alkynyl group, 5) an amino group, 6) a hydroxy group, 7) a cyano group and 8) an amidino group

### Group 3

1) a C₁₋₆ alkyl group, 2) a C₃₋₈ cycloalkyl group and 3) a C₆₋₁₄ aryl group.

### Group 4

1) an aromatic monocyclic heterocyclic group selected from Group 5, 2) an aromatic condensed heterocyclic group selected from Group 6 and 3) a saturated or unsaturated non-aromatic heterocyclic group selected from Group 7

### Group 5.

furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl

### Group 6

benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl

### Group 7

oxylanyl, azetidinyl, oxetanyl, thietanyl, pyrrolidinyl, tetrahydrofuryl, thiolanyl, piperidinyl, tetrahydropyranyl, morpholinyl, thiomorpholinyl and piperazinyl

### Group 8

1) a C₁₋₆ alkyl, 2) a C₁₋₆ alkanoyl, 3) a C₇₋₁₃ arylcarbonyl, 4) an optionally halogenated C₂₋₆ alkoxycarbonyl, 5) a C₁₋₆ alkylimidoyl, 6) a formylimidoyl and 7) an amidino

### Group 9

1) 1-azetidinyl, 2) 1-pyrrolidinyl, 3) 1-piperidinyl, 4) 4-morpholinyl, 5) 1-piperazinyl and 6) 1-piperazinyl which may have a C₁₋₆ alkyl, a C₇₋₁₀ aralkyl and a C₆₋₁₀ aryl at 4-position

### Group 10

C₃₋₉ cycloalkyl, 1-indanyl, 2-indanyl, C₃₋₆ cycloalkenyl, C₄₋₆ cycloalkanedienyl and C₆₋₁₄ aryl

### Group 11

1) a C₁₋₆ alkyl group which may be substituted by member(s) selected from Group 12, 2) a C₃₋₆ cycloalkyl group which may be substituted by member(s) selected from Group 12, 3) a C₆₋₁₀ aryl group which may be substituted by member (s) selected from Group 12, 4) a C₇₋₁₀ aralkyl group which may be substituted by member(s) selected from Group 12, 5) a C₁₋₆ alkoxy group which may be substituted by member(s) selected from Group 12 and 6) a heterocyclic group selected from Group 13 which may be substituted by member(s) selected from Group 12

### Group 12

1) a hydroxy group, 2) an amino group, 3) an amino group which is mono or di-substituted by member(s) selected from Group 16, 4) a halogen atom, 5) a nitro group, 6) a cyano group, 7) a C₁₋₆ alkyl group which may be substituted by halogen atom(s) and 8) a C₁₋₆ alkoxy group which may be substituted by halogen atom(s)

### Group 13

1) an aromatic heterocyclic group selected from Group 5 and Group 6 and 2) a saturated or unsaturated non-aromatic heterocyclic group selected from Group 7, each of which contains at least one heteroatom(s) selected from the group consisting of an oxygen atom, a sulfur atom and a nitrogen atom as ring-constituting atom(s)

### Group 14

a C₁₋₆ alkyl group, a C₃₋₆ cycloalkyl group and a C₇₋₁₀ aralkyl group

### Group 15

1) a C₁₋₁₀ alkylsulfonyl which may be substituted by member(s) selected from Group 12, 2) a C₂₋₆ alkenylsulfonyl which may be substituted by member(s) selected from Group 12, 3) a C₂₋₆ alkynylsulfonyl which may be substituted by member(s) selected from Group 12, 4) a C₃₋₉ cycloalkylsulfonyl which may be substituted by member(s) selected from Group 12, 5) a C₃₋₉ cycloalkenylsulfonyl which may be substituted by member(s) selected from Group 12, 6) a C₆₋₁₄ arylsulfonyl which may be substituted by member (s) selected from Group 12 and 7) a C₇₋₁₀ aralkylsulfonyl which may be substituted by member(s) selected from Group 12

### Group 16

a C₁₋₆ alkyl group, a C₁₋₆ alkanoyl, a C₇₋₁₃ arylcarbonyl and a C₁₋₆ alkylsulfonyl

### Group 17

1-azetidinylcarbonyl, 1-pyrrolidinylcarbonyl, 1-piperidinylcarbonyl, 4-morpholinylcarbonyl and 1-piperazinylcarbonyl which may be substituted by member(s) selected from Group 18

### Group 18

a C₁₋₆ alkyl group, a C₇₋₁₀ aralkyl group and a C₆₋₁₀ aryl group

### Group 19

a C₁₋₁₀ alkylsulfonyl which may be substituted by member(s) selected from Group 12, a C₂₋₆ alkenylsulfonyl which may be substituted by member(s) selected from Group 12, a C₂₋₆ alkynylsulfonyl which may be substituted by member(s) selected from Group 12, a C₃₋₉ cycloalkylsulfonyl which may be substituted by member(s) selected from Group 12, a C₃₋₉ cycloalkenylsulfonyl which may be substituted by member(s) selected from Group 12, a C₆₋₁₄ arylsulfonyl which may be substituted by member(s) selected from Group 12, and a C₇₋₁₀ aralkylsulfonyl which may be substituted by member(s) selected from Group 12

### Group 20

1-azetidinylsulfonyl, 1-pyrrolidinylsulfonyl, 1-piperidinylsulfonyl, 4-morpholinylsulfonyl and 1-piperazinylsulfonyl which may be substituted by member(s) selected from Group 18

(II-12) the compound wherein R^{b1} is a C₃₋₈ cycloalkyl group which may be substituted by member(s) selected from Group 1 or a C₆₋₁₄ aryl group which may be substituted by member(s) selected from Group 1,

(II-13) the compound as shown in the above (II-12), wherein R^{b1} is 1) a C₆₋₁₄ aryl group which may be substituted by a halogen atom, a C₁₋₆ alkyl which may be substituted by halogen(s), a C₁₋₄ alkylthio, a nitro, a carbamoyl, a sulfamoyl or a C₁₋₆ alkylsulfonyl, 2) a C₁₋₆ alkyl group which may be substituted by (i) a C₂₋₆ alkoxycarbonyl group or (ii) a phenyl which may be substituted by C₁₋₆ alkyl(s) or 3) a C₃₋₈ cycloalkyl group which may be substituted by (i) a halogen atom, (ii) a C₁₋₆ alkyl(s) which may be substituted by halogen(s) or (iii) a C₁₋₆ alkoxy group which may be substituted by halogen(s);
R^{b2} is a phenyl group which may be substituted by a halogen atom, a C₁₋₆ alkyl, a C₁₋₄ alkoxy or a cyano, a C₃₋₈ cycloalkyl group or a pyridyl group;
R^{b3} is (i) a halogen atom, (ii) a carbamoyl group, (iii) a sulfamoyl group which may have one or two C₁₋₆ alkyl (s) or C₃₋₆ cycloalkyl (s) at N-atoms, a cyclic aminosulfonyl group selected from Group 20,a C₁₋₆ alkylsulfonyl group or C₃₋₆ cycloalkylsulfonyl group;
R^{b4} is a hydrogen atom;
nb is 0 or 1 and
pb is 0 or 1,

(II-14) the compound as shown in the above (II-12), wherein R^{b1} is 1) a phenyl which may be substituted by a halogen atom, a C₁₋₃ alkyl, trifluoromethyl, methoxy, trifluoromethoxy, methylthio or nitro, 2) a naphthyl, 3) a C₁₋₆ alkyl group which may be substituted by (i) a C₂₋₃ alkoxycarbonyl, (ii) phenyl or (iii) 3-isopropenylphenyl or 4) cyclohexyl group;
R^{b2} is a phenyl group which may be substituted by a halogen atom, methyl, methoxy or cyano, a cyclohexyl group or a 3-pyridyl group;
R^{b3} is (i) a halogen atom, (ii) a carbamoyl group, (iii) a 4-morpholinylsulfonyl group or (iv) a methylsulfonyl group,
R^{b4} is a hydrogen atom;
nb is 0 or 1; and
pb is 0 or 1,

(II-15) the compound as shown in the above (II-12), wherein R^{b1} is a phenyl group which may be substituted by a halogen atom or a C₁₋₃ alkyl; R^{b2} is a phenyl group which may be substituted by a halogen atom and methyl(s);
R^{b3} is (i) a halogen atom, (ii) a carbamoyl group, (iii) a sulfamoyl group which may be substituted by one or two members selected from the group consisting of C₁₋₆ alkyl and C₃₋₆ cycloalkyl at N-atoms, (iv)a cyclic aminosulfonyl group selected from Group 20, (v)a C₁₋₆ alkylsulfonyl group or (vi) a C₃₋₆ cycloalkyl sulfonyl group;
R^{b4} is a hydrogen atom;
nb is 0; and
pb is 0 or 1.

The hydrocarbon group represented by R^{c1} includes, for example, an aliphatic chain hydrocarbon group, an alicyclic hydrocarbon group, an aryl group and the like Preferably, it is an aliphatic chain hydrocarbon group or an alicyclic hydrocarbon group.

The aliphatic chain hydrocarbon group includes, for example, a straight or branched aliphatic hydrocarbon group such as alkyl group, alkenyl group, alkynyl group and the like, with preference given to alkyl group. Examples of the alkyl group include C₁₋₁₀ alkyl groups (preferably C₂₋₆ alkyl etc.), such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, 1-methylheptyl, 1-ethylhexyl, n-octyl, 1-methylheptyl, nonyl and the like. Examples of the alkenyl group include C₂₋₆ alkenyl groups, such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like. Examples of the alkynyl group include C₂₋₆ alkynyl groups, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. Examples of the alicyclic hydrocarbon group include saturated or unsaturated alicyclic hydrocarbon groups, such as cycloalkyl group, cycloalkenyl group, cycloalkanedienyl group and the like, with preference given to cycloalkyl group. Examples of the cycloalkyl group include C₃₋₉ cycloalkyl (preferably C₃₋₈ cycloalkyl etc.), such as cyclopropyl, cyclobutyl, cyclopentyl, cyclohexyl, cycloheptyl, cyclooctyl, cyclononyl and the like, and fused rings such as 1-indanyl, 2-indanyl and the like. Examples of the cycloalkenyl group include C₃₋₆ cycloalkenyl groups, such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl and the like. Examples of the cycloalkanedienyl group include C₄₋₆ cycloalkanedienyl groups, such as 2,4-cyclopentanedien-1-yl, 2,4-cyclohexanedien-1-yl, 2,5-cyclohexanedien-1-yl and the like.

Examples of the aryl group include monocyclic or fused polycyclic aromatic hydrocarbon groups, which are preferably C₆₋₁₄ aryl groups, such as phenyl, naphthyl, anthryl, phenanthryl, acenaphthylenyl, 4-indanyl, 5-indanyl etc., and the like, with particular preference given to phenyl, 1-naphthyl, 2-naphthyl and the like.

The hydrocarbon group having 2 or more carbon atoms represented by R^{c2} includes, for example, the hydrocarbon groups having 2 or more carbon atoms among those represented by R^{c1}. Of those recited with regard to R^{c1}, preferred are C₂₋₆ alkyl and C₃₋₈ cycloalkyl.

When R^{c1} and R^{c2} in combination form, together with an adjacent nitrogen atom, a ring optionally having a substituent or substituents, the ring may contain, besides one nitrogen atom, a different nitrogen atom, an oxygen atom and a sulfur atom. Examples thereof include monocyclic groups, such as 1-azetidinyl, 1-pyrrolidinyl, 1-piperidinyl, 1-homopiperidinyl, heptamethyleneimino, 1-piperazinyl, 1-homopiperazinyl, morpholino, thiomorpholino and the like, fused rings such as 2-isoindolinyl, 1,2,3,4-tetrahydro-2-isoquinolyl, 1,2,4,5-tetrahydro-3H-3-benzodiazepin-3-yl and the like, cyclic amino groups such as spiro ring and the like (e.g., indene-1-spiro-4'-piperidin-1'-yl etc.), said cyclic amino group optionally having 1 to 5, preferably 1 to 3, substituent (s) at a chemically permitted position on the ring.

Examples of the substituent include hydroxy group, cyano group, nitro group, oxo group, halogen atom (e.g., fluorine atom, chlorine atom, bromine atom, iodine atom etc.), a group of the formula: -Y^{c}R^{ca} (wherein R^{ca} is a hydrocarbon group optionally having a substituent or substituents or a heterocyclic group optionally having a substituent or substituents, Y^{c} is a bond (single bond), -CR^{cb}R^{cc}-, -COO-, -CO-, -CO-NR^{cb}-, -CS-NR^{cb}-, -CO-S-, -CS-S-, -CO-NR^{cb}-CO-NR^{cc}-, -C(=NH)-NR^{cb}-, -NR^{b}-, -NR^{cb}-CO-, -NR^{cb}-CS-, -NR^{cb}-CO-NR^{cc}-, -NR^{cb}-CS-NR^{cc}-, -NR^{cb}-CO-O-, -NR^{cb}-CS-O-, -NR^{cb}-CO-S-, -NR^{cb}-CS-S-, -NR^{cb}-C (=NH)-NR^{cc}-, -NR^{cb}-SO₂-, -NR^{cb}-NR^{cc}-, -O-, -O-CO-, -O-CS-, -O-CO-O, -O-CO-NR^{cb}-, -O-C (=NH)-NR^{cb}-, -S-, -SO-, -SO₂-, -SO₂-NR^{cb}-, -S-CO-, -S-CS-, -S-CO-NR^{cb}-, -S-CS-NR^{cb}-, -S-C (=NH)-NR^{cb}- and the like, wherein R^{cb} and R^{cc} are each a hydrogen atom, alkyl group optionally having a substituent or substituents, alkenyl group optionally having a substituent or substituents, alkynyl group optionally having a substituent or substituents, an aryl group optionally having a substituent or substituents, cycloalkyl group or cycloalkenyl group optionally having a substituent or substituents, a heterocyclic group optionally having a substituent or substituents, acyl group derived from carboxylic acid, alkylsulfonyl group optionally having a substituent or substituents, an aryl sulfonyl group optionally having a substituent or substituents, etc.), and the like.

The "hydrocarbon group" of the hydrocarbon group optionally having a substituent or substituents represented by R^{ca} include an aliphatic chain hydrocarbon group, alicyclic hydrocarbon group, aryl group and the like. Examples of these aliphatic chain hydrocarbon group, alicyclic hydrocarbon group and aryl group is the aliphatic chain hydrocarbon group, alicyclic hydrocarbon group and aryl group represented by R^{c1} respectively. Examples of the substituent of the hydrocarbon group include those mentioned as the substituents for the "hydrocarbon group optionally having a substituent or substituents" represented by R^{c3} to be mentioned later.

Examples of the "heterocyclic group optionally having a substituent or substituents" at the aforementioned R^{ca} include those mentioned as the "heterocyclic group optionally having a substituent or substituents" represented by R^{c3} to be mentioned later

Examples of the alkyl group optionally having a substituent or substituents, alkenyl group optionally having a substituent or substituents, alkynyl group optionally having a substituent or substituents, aryl group optionally having a substituent or substituents, cycloalkyl group or cycloalkenyl group optionally having a substituent or substituents, heterocyclic group optionally having a substituent or substituents, acyl group derived from carboxylic acid, alkyl sulfonyl group optionally having a substituent or substituents and arylsulfonyl group optionally having a substituent or substituents, represented by the aforementioned R^{cb} and R^{cc} include those mentioned as the substituent of the hydrocarbon group optionally having a substituent represented by R^{c3} to be mentioned later.

It is preferable that R^{c1} and R^{c2} in combination forms, together with an adjacent nitrogen atom, a heterocyclic ring optionally having a substituent or substituents. More preferably, NR^{c1}R^{c2} is a group of the formula: 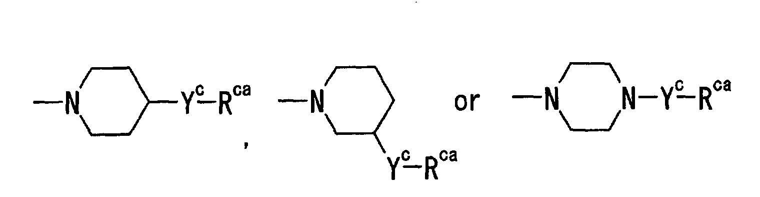 wherein Y^{c} and R^{ca} are as defined above. As used here, Y^{c} and R^{ca} are as defined above, and R^{ca} is particularly preferably an aryl group optionally having a substituent or substituents or a heterocyclic group optionally having a substituent or substituents.

Y^{c}R^{ca} is particularly preferably a benzyl group optionally having a substituent or substituents.

NR^{c1}R^{c2} is particularly preferably a 4-benzyl-1-piperidinyl group optionally having a substituent or substituents.

Examples of the hydrocarbon group of the hydrocarbon group optionally having a substituent or substituents represented by R^{c3} include those similar to the hydrocarbon groups represented by R^{c1}, with particular preference given to C₁₋₆ alkyl group, C₃₋₈ cycloalkyl group and aryl group These are exemplified by those recited for R^{c1}.

The heterocyclic group of the heterocyclic group optionally having a substituent or substituents represented by R^{c3} is, for example, an aromatic heterocyclic group, a saturated or unsaturated non-aromatic heterocyclic group (aliphatic heterocyclic group) and the like, containing, as an atom constituting the ring system, at least one (preferably 1 to 4, more preferably 1 or 2) of 1 to 3 kinds (preferably 1 or 2 kinds) of the hetero atom selected from an oxygen atom, a sulfur atom, a nitrogen atom and the like. Examples of the aromatic heterocyclic group include aromatic monocyclic heterocyclic group (e.g., 5- or 6-membered aromatic monocyclic heterocyclic group such as furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.); condensed aromatic heterocyclic group [e.g., 8 to 12-membered condensed aromatic heterocyclic group (preferably a heterocycle wherein the aforementioned 5- or 6-membered aromatic monocyclic heterocyclic group is condensed with a benzene ring or a heterocycle wherein the same or different two heterocycles of the aforementioned 5- or 6-membered aromatic monocyclic heterocyclic group are condensed), such as benzofuranyl, isobenzofuranyl, benzothienyl, indolyl, isoindolyl, 1H-indazolyl, benzindazolyl, benzooxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrro[1,2-b]pyridazinyl, pyrrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc.] and the like.

Examples of the non-aromatic heterocyclic group include 3 to 8-membered (preferably 5- or 6-membered) saturated or unsaturated (preferably saturated) non-aromatic heterocyclic group (aliphatic heterocyclic group), such as oxiranyl, azetidinyl, oxetanyl, thietanyl, pyrrolidinyl, tetrahydrofuryl, thioranyl, piperidinyl, tetrahydropyranyl, morpholinyl, thiomorpholinyl, piperazinyl etc., and the like.

Examples of the substituent of the hydrocarbon group optionally having a substituent or substituents as represented by R^{c3} and the substituent of the heterocyclic group optionally having a substituent or substituents as represented by R^{c3} include alkyl group optionally having a substituent or substituents, alkenyl group optionally having a substituent or substituents, alkynyl group optionally having a substituent or substituents, an aryl group optionally having a substituent or substituents, cycloalkyl group or cycloalkenyl group optionally having a substituent or substituents, a heterocyclic group optionally having a substituent or substituents, amino group optionally having a substituent or substituents, imidoyl group optionally having a substituent or substituents, amidino group optionally having a substituent or substituents, hydroxy group optionally having a substituent or substituents, thiol group optionally having a substituent or substituents, optionally esterified carboxyl group, carbamoyl group optionally having a substituent or substituents, thiocarbamoyl group optionally having a substituent or substituents, sulfamoyl group optionally having a substituent or substituents, halogen atom (e.g., fluorine, chlorine, bromine, iodine etc., preferably chlorine, bromine etc.), cyano group, nitro group, acyl group derived from carboxylic acid, alkyl sulfinyl group optionally having a substituent or substituents, alkyl sulfonyl group optionally having a substituent or substituents, arylsulfinyl group optionally having a substituent or substituents, arylsulfonyl group optionally having a substituent or substituents and the like, wherein 1 to 5 (preferably 1 to 3) of these optional substituents may be present at a substitutable position.

The aryl group of the "aryl group optionally having a substituent or substituents" as a substituent may be, for example, C₆₋₁₄ aryl group such as phenyl, naphthyl, anthryl, phenanthryl, acenaphthylenyl etc., and the like. Here, the substituent of the aryl group includes, for example, lower alkoxy group (e.g., C₁₋₆ alkoxy group such as methoxy, ethoxy, propoxy etc., and the like), halogen atom (e.g., fluorine, chlorine, bromine, iodine etc.), lower alkyl group (e.g., C₁₋₆ alkyl group such as methyl, ethyl, propyl etc., etc.), amino group, hydroxy group, cyano group, amidino group and the like, wherein one or two of these optional substituents may be present at a substitutable position.

The cycloalkyl group of the "cycloalkyl group optionally having a substituent or substituents" as a substituent may be, for example, C₃₋₇ cycloalkyl group, such as cyclopropyl, cyclobutyl, cyclopentyl, cyclohexyl, cycloheptyl etc., and the like. As used herein, examples of the substituent of the "cycloalkyl group" are similar in the kind and the number to those exemplified for the substituent of the aforementioned "aryl group optionally having a substituent or substituents".

The cycloalkenyl group of the "cycloalkenyl group optionally having substituents" as a substituent may be, for example, C₃₋₆ cycloalkenyl group such as cyclopropenyl, cyclobutenyl, cyclopentenyl, cyclohexenyl etc., and the like. As used herein, examples of the substituent of the "cycloalkenyl group optionally having a substituent or substituents" are similar in the kind and the number to those exemplified for the substituent of the aforementioned "aryl group optionally having a substituent or substituents".

The alkyl group of the "alkyl group optionally having a substituent or substituents" as a substituent may be, for example, C₁₋₆ alkyl such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl etc., and the like. As used herein, examples of the substituent of the alkyl group are similar in the kind and the number to those exemplified for the substituent of the aforementioned "aryl group optionally having a substituent or substituents". The alkenyl group of the "alkenyl group optionally having a substituent or substituents" as a substituent may be, for example, C₂₋₆ alkenyl group such as vinyl, allyl, isopropenyl, 2-methylallyl, 1-propenyl, 2-methyl-1-propenyl, 1-butenyl, 2-butenyl, 3-butenyl, 2-ethyl-3-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl etc., and the like. As used herein, examples of the substituent of the alkenyl group are similar in the kind and the number to those exemplified for the substituent of the aforementioned "aryl group optionally having a substituent or substituents".

The alkynyl group of the "alkynyl group optionally having a substituent or substituents" as a substituent may be, for example, C₂₋₆ alkynyl group, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. As used herein, examples of the substituent of the alkynyl group are similar in the kind and the number to those exemplified for the substituent of the aforementioned "aryl group optionally having a substituent or substituents". The heterocyclic group of the "heterocyclic group optionally having a substituent or substituents" as a substituent may be, for example, an aromatic heterocyclic group, a saturated or unsaturated non-aromatic heterocyclic group (aliphatic heterocyclic group) and the like, containing, as an atom (cyclic atom) constituting the ring system, at least one (preferably 1 to 4, more preferably 1 or 2) of 1 to 3 kinds (preferably 1 or 2 kinds) of the hetero atom selected from an oxygen atom, a sulfur atom and a nitrogen atom, and the like.

Examples of the "aromatic heterocyclic group" include aromatic monocyclic heterocyclic group (e.g., 5- or 6-membered aromatic monocyclic heterocyclic group, such as furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.) and condensed aromatic heterocyclic group [e.g., 8 to 12-membered condensed aromatic heterocycle (preferably a heterocycle wherein the aforementioned 5- or 6-membered aromatic monocyclic heterocyclic group is condensed with a benzene ring or a heterocycle wherein the same or different two heterocycle of the aforementioned 5- or 6-membered aromatic monocyclic heterocyclic group are condensed), such as benzofuranyl, isobenzofuranyl, benzothienyl, indolyl, isoindolyl, 1H-indazolyl, benzindazolyl, benzooxazolyl, 1,2-benzoisooxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrro[1,2-b]pyridazinyl, pyrrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc.] and the like.

Examples of the "non-aromatic heterocyclic group" include 3 to 8-membered (preferably 5- or 6-membered) saturated or unsaturated (preferably saturated) non-aromatic heterocyclic group (aliphatic heterocyclic group), such as oxiranyl, azetidinyl, oxetanyl, thietanyl, pyrrolidinyl, tetrahydrofuryl, thioranyl, piperidinyl, tetrahydropyranyl, morpholinyl, thiomorpholinyl, piperazinyl etc., and the like.

The substituent that the "heterocyclic group optionally having a substituent or substituents" as a substituent may have is exemplified by lower alkyl group (e.g., C₁₋₆ alkyl group, such as methyl, ethyl, propyl etc., and the like), acyl group (e.g., C₁₋₆ alkanoyl, such as formyl, acetyl, propionyl, pivaloyl etc., benzoyl etc.), and the like.

The substituent of the "amino group optionally having a substituent or substituents", "imidoyl group optionally having a substituent or substituents", "amidino group optionally having a substituent or substituents", "hydroxy group optionally having a substituent or substituents" and "thiol group optionally having a substituent or substituents" as a substituent may be, for example, lower alkyl group (e.g., C₁₋₆ alkyl group, such as methyl, ethyl, propyl, isopropyl, butyl, isobutyl, t-butyl, pentyl, hexyl etc., and the like), acyl group (e.g., C₁₋₆ alkanoyl (e.g., formyl, acetyl, propionyl, pivaloyl etc.), benzoyl etc.), C₁₋₆ alkyl sulfonyl (e.g., methanesulfonyl, ethanesulfonyl etc.), C₃₋₁₄ arylsulfonyl (e.g., benzenesulfonyl, p-toluenesulfonyl etc.), optionally halogenated C₁₋₆ alkoxycarbonyl (e.g., trifluoromethoxycarbonyl, 2,2,2-trifluoroethoxycarbonyl, trichloromethoxycarbonyl, 2,2,2-trichloroethoxycarbonyl etc.), and the like. The " amino group" of the "amino group optionally having a substituent or substituents" as the substituent may be substituted by imidoyl group optionally having a substituent or substituents (e.g., C₁₋₆ alkyl imidoyl, formylimidoyl, amidino etc.), and the like. In addition, two substituents may form a cyclic amino group together with a nitrogen atom. In this case, examples of the cyclic amino group include 3 to 8-membered (preferably 5- or 6-membered) cyclic amino, such as 1-azetidinyl, 1-pyrrolidinyl, 1-piperidinyl, morpholino, 1-piperazinyl and 1-piperazinyl optionally having, at the 4-position, lower alkyl group (e.g., C₁₋₆ alkyl group, such as methyl, ethyl, propyl, isopropyl, butyl, t-butyl, pentyl, hexyl etc., and the like), aralkyl group (e.g., C₇₋₁₀ aralkyl group, such as benzyl, phenethyl etc., and the like), aryl group (e.g., C₆₋₁₀ aryl group, such as phenyl, 1-naphthyl, 2-naphthyl etc., and the like), and the like.
Examples of the "carbamoyl group optionally having a substituent or substituents" include unsubstituted carbamoyl, N-monosubstituted carbamoyl group and N,N-disubstituted carbamoyl group.

The "N-monosubstituted carbamoyl group" is a carbamoyl group having one substituent on the nitrogen atom. Examples of the substituent include lower alkyl group (e.g., C₁₋₆ alkyl group, such as methyl, ethyl, propyl, isopropyl, butyl, isobutyl, t-butyl, pentyl, hexyl etc., and the like), cycloalkyl group (e.g., C₃₋₆ cycloalkyl group, such as cyclopropyl, cyclobutyl, cyclopentyl, cyclohexyl etc., and the like), aryl group (e.g., C₆₋₁₀ aryl group, such as phenyl, 1-naphthyl, 2-naphthyl etc., and the like), aralkyl group (e.g., C₇₋₁₀ aralkyl group, such as benzyl, phenethyl etc., preferably phenyl-C₁₋₄ alkyl group etc.), heterocyclic group (e.g., those exemplified as the "heterocyclic group" as a substituent of "hydrocarbon group optionally having a substituent or substituents" represented by R^{c3} and the like). The lower alkyl group, cycloalkyl group, aryl group, aralkyl group and heterocyclic group may have substituents, which substituents are, for example, hydroxy group, amino group optionally having a substituent or substituents [which amino group optionally having 1 or 2 from lower alkyl group (e.g., C₁₋₆ alkyl group such as methyl, ethyl, propyl, isopropyl, butyl, isobutyl, t-butyl, pentyl, hexyl etc., and the like), acyl group (e.g., C₁₋₆ alkanoyl such as formyl, acetyl, propionyl, pivaloyl etc., benzoyl, etc.), and the like as substituents], halogen atom (e.g., fluorine, chlorine, bromine, iodine etc.), nitro group, cyano group, lower alkyl group optionally having 1 to 5 halogen atoms as substituents (e.g., fluorine, chlorine, bromine, iodine etc.) lower alkoxy group optionally having 1 to 5 halogen atoms as substituents (e.g., fluorine, chlorine, bromine, iodine etc.), and the like. Examples of the lower alkyl group include C₁₋₆ alkyl group, such as methyl, ethyl, n-propyl, isopropyl, n-butyl, isobutyl, sec-butyl, tert-butyl, pentyl, hexyl etc., and the like, particularly preferably methyl, ethyl and the like. Examples of the lower alkoxy group include C₁₋₆ alkoxy group, such as methoxy, ethoxy, n-propoxy, isopropoxy, n-butoxy, isobutoxy, sec-butoxy, tert-butoxy etc., and the like, particularly preferably methoxy, ethoxy and the like. One, two or three of these (preferably 1 or 2) are the same or different and may be present.
The "N,N-disubstituted carbamoyl group" is a carbamoyl group having 2 substituents on a nitrogen atom. Examples of one of the substituents are those similar to the substituents of the aforementioned "N-monosubstituted carbamoyl group" and examples of the other include lower alkyl group (e.g., C₁₋₆ alkyl group, such as methyl, ethyl, propyl, isopropyl, butyl, t-butyl, pentyl, hexyl etc., and the like), C₃₋₆ cycloalkyl group (e.g., cyclopropyl, cyclobutyl, cyclopentyl, cyclohexyl etc.), C₇₋₁₀ aralkyl group (e.g., benzyl, phenethyl etc., preferably phenyl-C₁₋₄ alkyl group etc.) and the like. Two substituents may form a cyclic amino group together with a nitrogen atom. In this case, examples of the cyclic aminocarbamoyl group include 3 to 8-membered (preferably 5- or 6-membered) cyclic amino-carbomoyl such as 1-azetidinylcarbonyl, 1-pyrrolidinylcarbonyl, 1-piperidinylcarbonyl, morpholinocarbonyl, 1-piperazinylcarbonyl and 1-piperazinylcarbonyl optionally having, at the 4-position, lower alkyl group (e.g., C₁₋₆ alkyl group, such as methyl, ethyl, propyl, isopropyl, butyl, t-butyl, pentyl, hexyl etc., and the like), aralkyl group (e.g., C₇₋₁₀ aralkyl group, such as benzyl, phenethyl etc., and the like), aryl group (e.g., C₆₋₁₀ aryl group, such as phenyl, 1-naphthyl, 2-naphthyl etc., and the like), and the like.
Examples of the substituent of the "thiocarbamoyl group optionally having a substituent or substituents" are similar to those exemplified for the substituent of the aforementioned "carbamoyl group optionally having a substituent or substituents". Examples of the "sulfamoyl group optionally having a substituent or substituents" include unsubstituted sulfamoyl, N-monosubstituted sulfamoyl group and N,N-disubstituted sulfamoyl group.

The "N-monosubstituted sulfamoyl group" means sulfamoyl group having one substituent on a nitrogen atom. Examples of the substituent are those similar to the substituent of the "N-monosubstituted carbamoyl group".

The "N,N-disubstituted sulfamoyl group" means sulfamoyl group having 2 substituents on a nitrogen atom. Examples of the substituent are those similar to the substituent of the "N,N-disubstituted carbamoyl group".

Examples of the "optionally esterified carboxyl group" include, besides free carboxyl group, lower alkoxy carbonyl group, aryloxycarbonyl group, aralkyloxycarbonyl group and the like.

Examples of the "lower alkoxy carbonyl group" include C₁₋₆ alkoxy-carbonyl group, such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl etc., and the like. Of these, C₁₋₃ alkoxy-carbonyl group, such as methoxycarbonyl, ethoxycarbonyl, propoxycarbonyl etc., and the like are preferable.

Examples of the aryloxycarbonyl group" preferably include C₇₋₁₂ aryloxy-carbonyl group, such as phenoxycarbonyl, 1-naphthoxycarbonyl, 2-naphthoxycarbonyl etc., and the like.

Examples of the "aralkyloxycarbonyl group" preferably include C₇₋₁₀ aralkyloxy-carbonyl group (preferably C₆₋₁₀ aryl-C₁₋₄ alkoxy-carbonyl etc.) such as benzyloxycarbonyl, phenethyloxycarbonyl etc., and the like.

The "aryloxycarbonyl group" and "aralkyloxycarbonyl group" may have substituents. Examples of the substituent are similar in the kind and the number to those exemplified for the substituent of aryl group and aralkyl group as the substituents of the aforementioned N-monosubstituted carbamoyl group.

The "acyl group derived from carboxylic acid" as the substituent is exemplified by one wherein a hydrogen atom or the single substituent that the aforementioned "N-monosubstituted carbamoyl group" has on a nitrogen atom is bonded to carbonyl, and the like. Preferably C₁₋₆ alkanoyl such as formyl, acetyl, propionyl, pivaloyl etc. and acyl such as venzoyl etc., and the like.

The alkyl of the "alkyl sulfinyl group optionally having a substituent or substituents" and "alkyl sulfonyl group optionally having a substituent or substituents" as the substituent may be, for example, lower alkyl group such as C₁₋₆ alkyl group (e.g., methyl, ethyl, propyl, isopropyl, butyl, isobutyl, t-butyl, pentyl, hexyl etc.), and the like. The aryl of the "arylsulfinyl group optionally having a substituent or substituents" and "arylsulfonyl group optionally having a substituent or substituents" as the substituent may be, for example, C₆₋₁₄ aryl group, such as phenyl, naphthyl, anthryl, phenanthryl, acenaphthylenyl etc., and the like.

The substituent of these alkyl and aryl may be, for example, lower alkoxy group (e.g., C₁₋₆ alkoxy group, such as methoxy, ethoxy, propoxy etc., and the like), halogen atom (e.g., fluorine, chlorine, bromine, iodine etc.), lower alkyl group (e.g., C₁₋₆ alkyl group, such as methyl, ethyl, propyl etc., and the like), amino group, hydroxy group, cyano group, amidino group and the like, wherein one or two of these optional substituents may be present at a substitutable position.

The hydrocarbon group optionally having a substituent or substituents represented by R^{c4} is exemplified by those shown with regard to hydrocarbon group optionally having a substituent or substituents represented by R^{c3}, and the heterocyclic group optionally having a substituent or substituents represented by R^{c4} is exemplified by those shown with regard to the heterocyclic group optionally having a substituent or substituents represented by R^{c3}.

The divalent chain hydrocarbon group of the divalent chain hydrocarbon group optionally having a substituent or substituents other than oxo group, represented by E^{c}, is exemplified by C₁₋₆ alkylene, such as methylene, ethylene etc., C₂₋₆ alkenylene, such as ethenylene etc., C₂₋₆ alkynylene, such as ethynylene etc., and the like. Preferred is C₁₋₅ alkylene and more preferred is trimethylene.

The substituent of the divalent hydrocarbon group may be any as long as it is not an oxo group. Examples thereof include alkyl group optionally having a substituent or substituents, an aryl group optionally having a substituent or substituents, cycloalkyl group or cycloalkenyl group optionally having a substituent or substituents, optionally esterified carboxyl group, carbamoyl group or thiocarbamoyl group optionally having a substituent or substituents, amino group optionally having a substituent or substituents, hydroxy group optionally having a substituent or substituents, thiol (mercapto) group optionally having a substituent or substituents, acyl group derived from carboxylic acid, alkyl sulfonyl group optionally having a substituent or substituents, arylsulfonyl group optionally having a substituent or substituents, halogen (e.g., fluorine, chlorine, bromine etc.), nitro, cyano and the like. The number of the substituents may be 1 to 3. The alkyl group optionally having a substituent or substituents, the aryl group optionally having a substituent or substituents, the cycloalkyl group or cycloalkenyl group optionally having a substituent or substituents, the optionally esterified carboxyl group, the carbamoyl group or thiocarbamoyl group optionally having a substituent or substituents, the amino group optionally having a substituent or substituents, the hydroxy group optionally having a substituent or substituents, the thiol (mercapto) group optionally having a substituent or substituents, the acyl group derived from carboxylic acid, the alkyl sulfonyl group optionally having a substituent or substituents, the arylsulfonyl group optionally having a substituent or substituents are those similar to the substituent of the heterocyclic group optionally having a substituent or substituents represented by the aforementioned R^{c3}. Examples of the substituent of the methine group optionally having a substituent or substituents represented by J^{c} are those similar to the substituent of the heterocyclic group optionally having a substituent or substituents represented by the aforementioned R^{c3}.

The divalent chain C₁₋₃ hydrocarbon group of the divalent chain C₁₋₃ hydrocarbon group optionally having a substituent or substituents, represented by Q^{c} and R^{c}, is exemplified by one having 1 to 3 carbon atoms from the divalent chain hydrocarbon group of the divalent chain hydrocarbon group optionally having a substituent or substituents other than oxo group, represented by E^{c}.

The substituent of the divalent chain C₁₋₃ hydrocarbon group optionally having a substituent or substituents, represented by Q^{c} and R^{c}, is exemplified by those exemplified as the substituent of the divalent chain hydrocarbon group optionally having a substituent or substituents other than oxo group, represented by E^{c}. The salt of the carboxyl group or sulfonic acid group, as represented by R^{c5}, is exemplified by salts with alkali metal, such as sodium, potassium, lithium etc., salts with alkaline earth metal, such as calcium, magnesium, strontium etc., ammonium salt and the like.

Among the compounds represented by the formula (III) or salts thereof (hereinafter referred to as Compound (III)), the following compounds are preferable.

(III-I) The compound wherein R^{c1} is a C₁₋₆ alkyl group or a C₃₋₈ cycloalkyl group; R^{c2} is a C₂₋₆ alkyl group or a C₃-₈ cycloalkyl group, or R^{c1} and R^{c2} in combination form, together with an adjacent nitrogen atom, a ring optionally having a substituent or substituents; R^{c3} is a C₁₋₆ alkyl group optionally having a substituent or substituents, a C₃₋₈ cycloalkyl group optionally having a substituent or substituents, an aryl group optionally having a substituent or substituents or a heterocyclic group optionally having a substituent or substituents; R^{c4} is a hydrogen atom, alkyl group optionally having a substituent or substituents, a C₃₋₈ cycloalkyl group optionally having a substituent or substituents, an aryl group optionally having a substituent or substituents or a heterocyclic group optionally having a substituent or substituents; E^{c} is a C₂₋₅ alkylene group optionally having a substituent or substituents other than oxo group; G^{c} is CO or SO₂; J^{c} is a nitrogen atom or a methine group optionally having a substituent or substituents; and Q^{c} and R^{c} are each a bond or a C₁₋₃ alkylene group optionally having a substituent or substituents.

(III-2) The compound wherein R^{c1} and R^{c2} in combination form, together with an adjacent nitrogen atom, a ring optionally having a substituent or substituents.

(III-3) The compound as shown in the above (III-2), wherein the ring optionally having a substituent or substituents is a 1-piperidinyl group or a 1-piperazinyl group each optionally having a substituent or substituents.

(III-4) The compound as shown in the above (III-3), wherein the substituent of the 1-piperidinyl group or 1-piperazinyl group is (1) phenyl-C₁₋₄ alkyl optionally having halogen on a benzene ring, (2) diphenylmethyl optionally having hydroxy, (3) benzoyl optionally having halogen on a benzene ring, (4) 2-phenylethen-1-yl, (5) phenyl optionally having halogen, (6) hydroxy, (7) phenoxy or (8) benzyloxy.

(III-5) The compound as shown in the above [III-2], wherein the ring optionally having a substituent or substituents is a 1-piperidinyl group optionally having a substituent or substituents.

(III-6) The compound as shown in the above [III-5], wherein the substituent of the 1-piperidinyl group is a benzyl group optionally having halogen on a benzene ring.

(III-7) The compound wherein R^{c3} is (1) a C₁₋₆ alkyl group, (2) a C₃₋₈ cycloalkyl group, (3) a benzyl group optionally having a hydroxy group, (4) a naphthylmethyl group, (5) a phenyl group optionally having, as a substituent, (a) C₁₋₄ alkyl optionally having halogen, (b) C₁₋₄ alkoxy optionally having halogen, (c) phenyl, (d) cyano, (e) benzyloxy or (f) a halogen atom, (6) a naphthyl group, (7) an indanyl group or (8) a tetrahydronaphthyl group.

(III-8) The compound wherein R^{c3} is a phenyl group optionally having, as a substituent, C₁₋₄ alkyl or halogen.

(III-9) The compound wherein E^{c} is C₂₋₆ polymethylene optionally having hydroxy.

(III-10) The compound wherein R^{c4} is (1) a hydrogen atom, (2) C₁₋₆ alkyl optionally having (a) halogen, (b) pyridyl, (c) morpholino, (d) furyl, (e) ethynyl or (f) C₃-₈ cycloalkyl, (3) phenyl-C₁₋₄ alkyl optionally having (a) halogen, (b) C₁₋₄ alkyl, (c) halogeno-C₁₋₄ alkyl or (d) C₁₋₄ alkoxy on a benzene ring, or (4) C₃₋₈ cycloalkyl.

(III-11) The compound wherein R^{c4} is (a) C₁₋₄ alkyl group optionally having, as a substituent, halogen or furyl or (b) a benzyl group optionally having halogen on a benzene ring.

(III-12) The compound wherein -N(R^{c1})R^{c2} is a 1-piperidinyl group optionally having a substituent or substituents, E^{c} is a trimethylene group, R^{c3} is a phenyl group optionally having a substituent or substituents, G^{c} is CO, J^{c} is CH, and Q^{c} and R^{c} are each a methylene group In the above formulas, examples of the C₁₋₆ alkyl group shown by R^{da} in groups -NR^{da}-SO₂- and -NR^{da}-CO-, which are represented by B^{d} include e.g. methyl, ethyl, propyl, isopropyl, butyl, isobutyl, t-butyl, pentyl, hexyl, etc., examples of the C₂₋₆ alkenyl group include e.g. vinyl, allyl, 1-propeny, isopropeny, 2-butenyl, 3-butenyl, 2-hexenyl, etc., and examples of the C₃₋₈ cycloalkyl group include e.g. cyclopropyl, cyclobutyl, cyclopentyl, cyclohexyl, cyclooctyl, etc.

Examples of the halogen atom represented by R^{d1} include e.g. fluorine, chlorine, bromine, iodine, etc., examples of the C₁₋₆ alkyl group include e.g. methyl, ethyl, propyl, isopropyl, butyl, isobutyl, t-butyl, pentyl, hexyl, etc., examples of the C₂₋₄ alkenyl group include e.g. vinyl, 1-propeny, 2-propeny, isopropeny, butenyl, isobutenyl, etc., example of C₁₋₄ alkanoyl group include e.g. formyl, acetyl, propionyl, butyryl, etc., and example of C₁₋₄ alkoxy group include e.g. methoxy, ethoxy, propoxy, etc.

Examples of halogen represented by R^{d2} include e.g.fluorine, chlorine, bromine, iodine, etc.

Examples of the C₁₋₆ alkyl in the "C₁₋₆ alkyl which may be substituted by a halogen or C₁₋₄ alkoxy" represented by R^{d2} include e.g. methyl, ethyl, propyl, isopropyl, butyl, isobutyl, t-butyl, pentyl, hexyl, etc., examples of the halogen as the substituent includes e.g. fluorine, chlorine, bromine, iodine, etc., and examples of C₁₋₄ alkoxy as the substituent include e.g. methoxy, ethoxy, n-propoxy, isopropoxy, n-butoxy, isobutoxy, sec-butoxy, tert-butoxy, etc.

Examples of the C₁₋₄ alkoxy group in the " C₁₋₄ alkoxy which may be substituted by a halogen or C₁₋₄ alkoxy" represented by R^{d2} include e.g. methoxy, ethoxy, n-propoxy, isopropoxy, n-butoxy, isobutoxy, sec-butoxy, tert-butoxy, etc., examples of the halogen as the substituent includes e.g. fluorine, chlorine, bromine, iodine, etc., and examples of C₁₋₄ alkoxy as the substituent include e.g. methoxy, ethoxy, n-propoxy, isopropoxy, n-butoxy, isobutoxy, sec-butoxy, tert-butoxy, etc.

Examples of C₁₋₄ alkanoylamino represented by R^{d2} include e.g. formylamino, acetylamino, propionylamino, butyrylamino, etc.

In the groups SO₂NR^{db}R^{dc}, CONR^{db}R^{dc} and NR^{db}R^{dc} each of which is represented by R^{d2}, examples of the "C₁₋₆ alkyl group which may be substituted by a halogen or C₁₋₄ alkoxy" represented by R^{db} and R^{dc} include those mentioned for the "C₁₋₆ alkyl which may be substituted by a halogen or C₁₋₄ alkoxy" represented by R^{d2}; examples of the "C₃₋₈ cycloalkyl group which may be substituted by a halogen or C₁₋₄ alkoxy" include e.g. cyclopropyl, cyclobutyl, cyclopentyl, cyclohexyl, cyclooctyl, etc.; the halogen which is the substituent include e.g. fluorine, chlorine, bromine, iodine, etc., and the C₁₋₄ alkoxy which is the substituent include e.g. methoxy, ethoxy, n-propoxy, isopropoxy, n-butoxy, isobutoxy, sec-butoxy, tert-butoxy, etc.

In the groups SO₂NR^{db}R^{dc}, CONR^{db}R^{dc} and NR^{db}R^{dc} each of which is represented by R^{dc}, examples of the cyclic amino group which forms by combining R^{db} and R^{dc} together with the nitrogen atom include e.g. 1) 1-azetidinyl, 2) 1-pyrrolidinyl, 3) 1-piperidinyl, 4) 4-morpholinyl, 5) 1-piperazinyl and 6) 1- piperazynyl which may be substituted by a C₁₋₆ alkyl (e.g.methyl, ethyl, propyl, isopropyl, butyl, isobutyl, t-butyl, pentyl, hexyl, etc.), a C₇₋₁₀ aralkyl (e.g. benzyl, phenethyl, etc) or a C₆₋₁₀ aryl (e.g. phenyl, 1-naphthyl, 2-naphthyl, etc.) at 4-position. In the group NR^{da}-SO₂R^{dd} represented by R^{d2}, the definition of R^{da} is the same as that of R^{da} in groups -NR^{da}-SO₂- and -NR^{da}-CO- each of which represented by B^{d}.
In the groups SO₂R^{dd} and -NR^{da}-SO₂R^{dd}represented by R^{d2}, examples of the C₁₋₆alkyl group represented by R^{dd} include e.g. methyl, ethyl, propyl, isopropyl, butyl, isobutyl, t-butyl, pentyl, hexyl, etc. and examples of the C₃₋₈cycloalkyl group include, e.g. cyclopropyl, cyclobutyl, cyclopentyl, cyclohexyl, cyclooctyl, etc.

In the formula (IV), (IIId) and (IVd), examples of the hydrocarbon group in thehydrocarbon group which may be substituted represented by R^{d3} in the groups shown by the formulas (d1), (d2), (d3), (d4), (d5) and (d6) includes e.g. a C₁₋₆alkyl (e.g. methyl, ethyl, propyl, isopropyl, butyl, isobutyl, t-butyl, pentyl, hexyl, etc.), a C₂₋₆alkenyl (e.g. vinyl, allyl, 1-propeny, isopropeny, 2-butenyl, 3-butenyl, 2-hexenyl, etc.), a C₂₋₆alkynyl (e.g. ethynyl, 2-propynyl, 2-butynyl, 5-hexynyl, etc.), a C₃₋₈Cycloalkyl (e. g. cyclopropyl, cyclobutyl, cyclopentyl, cyclohexyl, cyclooctyl, etc.), a C₆₋₁₀aryl (e.g. phenyl, naphthyl, etc.), etc.

Examples of the substituent of the hydrocarbon group include e.g. a halogen (e.g. fluorine, chlorine, bromine, iodine, etc), a C₁₋₄alkoxy( e.g. methoxy, ethoxy, n-propoxy, isopropoxy, n-butoxy, isobutoxy, sec-butoxy, tert-butoxy, etc.), phenyl, a C₁₋₆alkyl (e.g. methyl, ethyl, n-propyl, isopropyl, n-butyl, isobutyl, sec-butyl, tert-butyl, pentyl, hexyl, etc.), nitro, cyano, hydroxy, a C₁₋₄alkanoylamino (e.g. formylamino, acetylamino, propionylamino, etc.), carbamoyl, sulfamoyl, etc.

Among them, when the hydrocarbon group is a C₁₋₆alkyl, a C₂₋₆alkenyl or C₂₋₆alkynyl, preferable examples of the substituent include a halogen, a C₁₋₄alkoxy and phenyl; when the hydrocarbon group is a C₃₋₈cycloalkyl, preferable examples of the substituent include a halogen, a C₁₋₆alkyl and a C₁₋₄alkoxy; when the hydrocarbon group is a C₆₋₁₀aryl, preferable example include a halogen, a C₁₋₆alkyl, a C₁₋₄alkoxy, nitro, cyano, hydroxy, a C₁₋₄alkanoylamino, carbamoyl and sulfamoyl.

Examples of the C₁₋₄alkoxy in C₁₋₄alkoxy which may be substituent represented by R^{d3} include e.g. methoxy, ethoxy, propoxy, etc, and example of the substituent in C₁₋₄alkoxy which may be substituent represented by R^{d3} include e.g. a halogen (e.g. fluorine, chlorine, bromine, iodine, etc.), phenyl, etc.

Examples of the amino group which may be substituted represented by R^{d3} include not only an unsubstituted amino group but also, e.g. a C₁₋₆alkylamino (e.g. methylamino, ethylamino, propylamino, isopropylamino, butylamino, isobutylamino, t-butylamino, pentylamino, hexylamino, etc.), a di (C₁₋₆alkyl) amino (e.g. dimethylamino, diethylamino, dipropylamino, dibutylamino, etc.), a C₁₋₄alkoxyamino (e.g. methoxyamino, ethoxyamino, n-propoxyamino, isopropoxyamino, n-butoxyamino, isobutoxyamino, sec-butoxyamino, tert-butoxyamino, etc.), etc.

Examples of the C₁₋₆ alkyl group represented by R^{d4} and R^{d5} include e.g. methyl, ethyl, propyl, isopropyl, butyl, isobutyl, t-butyl, pentyl, hexyl, etc.

Examples of the C₁₋₆ alkyl group represented by R^{d6} include e.g. methyl, ethyl, propyl, isopropyl, butyl, isobutyl, t-butyl, pentyl, hexyl, etc. and examples of the C₂₋₆ alkenyl group include e.g. vinyl, allyl, 1-propeny, isopropeny, 2-butenyl, 3-butenyl, 2-hexenyl, etc.

Among the compounds of the formula (IV) or salts thereof (hereinafter referred to as Compound (IV)), the following compounds are preferable.

(IV-1) a compound wherein R^{d3} is 1) C₁₋₆ alkyl group which may be substituted by a halogen, a C₁₋₄ alkoxy or phenyl, 2) a C₂₋₆alkenyl group, 3) a C₂₋₆alkynyl group, 4) aC₃₋₈cycloalkylgroup which may be substituted by a halogen, a C₁₋₆alkyl or a C₁₋₄alkoxy, 5) a C₆₋₁₀aryl group which may be substituted by a halogen, a C₁₋₆alkyl, a C₁₋₄alkoxy, nitro, cyano, hydroxy, a C₁₋₄alkanoylamino, carbamoyl or sulfamoyl, 6) a C₁₋₄alkoxy group which may be substituted by a halogen or phenyl, or 7) an amino group which may be substituted by one or two groups selected from the group consisting of a C₁₋₆alkyl and a C₁₋₄alkoxy;

(IV-2) a compound wherein R^{d3} is 1) C₁₋₆ alkyl group, 2) a C₂₋₆alkenyl group, 3) a C₃₋₈cycloalkylgroup, 4) a C₁₋₄alkoxy group or 5) an amino group which may be substituted by a C₁₋₆alkyl or a C₁₋₄alkoxy, X^{d} is -SO₂- or -CO-, nd is 1 or 2, md is 0, 1 or 2, R^{d4} and R^{d5} are the same or different, and each of which is a hydrogen atom or a methyl group, R^{d6} is a hydroxy group, methyl group or a C₂₋₆alkenyl group, and rd is 3.

(IV-3) a compound wherein A^{d} is a group of the formula: 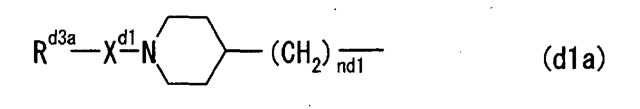 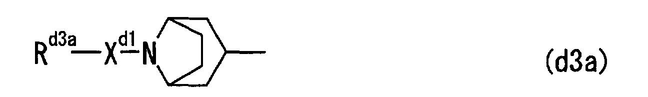 or 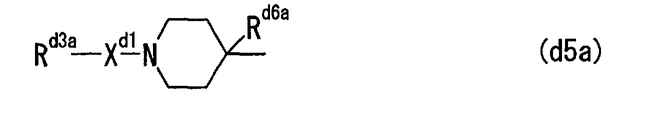 wherein R^{d3a} is a C₁₋₆alkyl group, X^{d1} is -SO₂- or -CO-, nd1 is 1 or 2, R^{d6a} is a hydroxy group or a methyl group; rd is 3, B^{d} is -CH₂-, each of pd and qd is 0, 1 or 2, R^{d1} is a halogen atom, a methylgroup, R^{d2} is a halogen, a C₁₋₄alkoxy, nitro, a C₁₋₄alkanoylamino, SO₂NR^{db}R^{dc}, SO₂R^{dd}, CONR^{db}R^{dc}, NR^{db}R^{dc} or NR^{da}-SO₂R^{dd} (wherein R^{da} is a hydrogen atom, a C₁₋₆alkyl group, a C₂₋₆alkenyl group or a C₃₋₈cycloalkyl group, R^{db} and R^{dc} areare the same or different, and each of which is a hydrogen atom, a C₁-₆alkyl group or a C₃₋₈cycloalkyl group, or R^{db} and R^{dc} may combine each other together with the nitrogen atom to form a cyclic amino group, R^{dd} is a C₁₋₆alkylgroup or a C₃₋₈cycloalkyl group).

"A 5- to 6-membered cyclic ring" of "a 5- to 6-membered cyclic ring group which may be substituted", which is represented by R^{e1} in the above-mentioned formula (eI), is exemplified by a group which is formed by removing one hydrogen atom from a 6-membered aromatic hydrocarbon such as benzene or the like, a 5- to 6-membered aliphatic hydrocarbon such as cyclopentane, cyclohexane, cyclopentene, cyclohexene, cyclopentadiene, cyclohexadiene or the like, a 5- to 6-membered aromatic heterocyclic ring, which contains 1 to 4 heteroatoms of 1 to 2 kinds selected from the nitrogen atom, the sulfur atom and the oxygen atom, such as furan, thiophene, pyrrole, imidazole, pyrazole, thiazole, oxazole, isothiazole, isoxazole, tetrazole, pyridine, pyrazine, pyrimidine, pyridazine, triazole or the like, a 5- to 6-membered, non-aromatic heterocyclic ring, which contains 1 to 4 heteroatoms of 1 to 2 kinds selected from the nitrogen atom, the sulfur atom and the oxygen atom, such as tetrahydrofuran, tetrahydrothiophene, dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran or the like, or the like, where as for "a 5- to 6-membered cyclic ring" is preferably benzene, furan, thiophene, pyridine, cyclopehtane, cyclohexane, pyrrolidine, piperidine, piperazine, morpholine, thiomorpholine, tetrahydropyran (preferably, a 6-membered cyclic ring) or the like, particularly benzene.

Examples of "a substituent" which may be possessed by "a 5- to 6-membered cyclic ring" of "a 5- to 6-membered cyclic ring group which may be substituted", which is represented by R^{e1}, to be used include a halogen atom, nitro, cyano, an alkyl which may be substituted, a cycloalkyl which may be substituted, the hydroxyl group which may be substituted, the thiol group which may be substituted (the sulfur atom may be oxidized to form a sulfinyl group which may be substituted or a sulfonyl group which may be substituted), an amino group which may be substituted, an acyl which may be substituted, the carboxyl group which may be esterified, an aromatic group which may be substituted and the like.

A halogen as a substituent of R^{e1} is exemplified by fluorine, chlorine, bromine, iodine or the like, where fluorine and chlorine are particularly preferable.

An alkyl for an alkyl which may be substituted as a substituent of R^{e1} is exemplified by a straight-chain or branched alkyl having a carbon number of 1 to 10, for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl. A substituent in said alkyl which may be substituted is exemplified by a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxyl which may be halogenated (for example, methoxy, ethoxy, propoxy, butoxy, trifluoromethoxy, trifluoroethoxy or the like), a C₁₋₄ alkoxy-C₁₋₄ alkoxyl which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like) or the like, where the number of the substituents is preferably 1 to 3.

The cycloalkyl for a cycloalkyl which may be substituted as a substituent of R^{e1} is exemplified by, for example, a C₃₋₇ cycloalkyl such as cyclopropyl, cyclobutyl, cyclopentyl, cyclohexyl, cycloheptyl or the like. The substituent in said cycloalkyl which may be substituted is exemplified by a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxyl which may be halogenated (for example, methoxy, ethoxy, propoxy, butoxy, trifluoromethoxy, trifluoroethoxy or the like), a C₁₋₄ alkoxy-C₁₋₄ alkoxyl which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like) or the like, where the number of the substituents is preferably 1 to 3.

The substituent for the hydroxyl group which may be substituted as a substituent of R^{e1} is exemplified by a substituent such as (1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, etc.);
(2) a cycloalkyl which may be substituted and may contain heteroatoms (for example, a C₃₋₇ cycloalkyl such as cyclopropyl, cyclobutyl, cyclopentyl, cyclohexyl, cycloheptyl or the like; a saturated, 5- to 6-membered heterocyclic ring group containing 1 to 2 heteroatoms such as tetrahydrofuranyl, tetrahydrothienyl, pyrrolidinyl, pyrazolidinyl, piperidyl, piperazinyl, morpholinyl, thiomorpholinyl, tetrahydropyranyl, tetrahydrothiopyranyl or the like (preferably, tetrahydropyranyl or the like); or the like is exemplified);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl 2-cyclopentenylmethyl, 2-cyclohexenylmethyl or the like, or the like is exemplified);
(5) an aralkyl which may be substituted (for example, a phenyl-C₁₋₄ alkyl (for example, benzyl, phenethyl or the like) or the like is exemplified);
(6) formyl or an acyl which may be substituted (for example, an alkanoyl which has the carbon number of 2 to 4 (for example, acetyl, propionyl, butyryl, isobutyryl or the like), an alkylsulfonyl which has the carbon number of 1 to 4 (for example, methanesulfonyl, ethanesulfonyl or the like) or the like is exemplified); and
(7) an aryl which may be substituted (for example, phenyl, naphthyl or the like is exemplified),
   where examples of the substituents which may be possessed by (1) an alkyl which may be substituted, (2) a cycloalkyl which may be substituted, (3) an alkenyl which may be substituted, (4) a cycloalkenyl which may be substituted, (5) an aralkyl which may be substituted, (6) an acyl which may be substituted and (7) an aryl which may be substituted include a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₆ alkoxyl which may be halogenated (for example, methoxy, ethoxy, propoxy, butoxy, trifluoromethoxy, trifluoroethoxy or the like; preferably, a C₁₋₄ alkoxyl which may be halogenated), formyl, a C₂₋₄ alkanoyl (for example, acetyl, propionyl or the like), a C₁₋₄ alkylsulfonyl (for example, methanesulfonyl, ethanesulfonyl or the like), a 5- to 6-membered, aromatic heterocyclic ring which may be substituted [for example, an aromatic heterocyclic ring, which contains 1 to 4 heteroatoms of 1 to 2 kinds selected from the nitrogen atom, the sulfur atom and the oxygen atom, such as furan, thiophene, pyrrole, imidazole, pyrazole, thiazole, oxazole, isothiazole, isoxazole, tetrazole, pyridine, pyrazine, pyrimidine, pyridazine, triazole or the like; the substituent which may be possessed by said heteroaromatic ring is exemplified by a halogen (for example, fluorine, chlorine, bromine, iodine or the like), nitro, cyano, the hydroxyl group, the thiol group, an amino group, the carboxyl group, a C₁₋₄ alkyl which may be halogenated (for example, trifluoromethyl, methyl, ethyl or the like), a C₁₋₄ alkoxyl which may be halogenated (for example, methoxy, ethoxy, propoxy, butoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.], or the like, where the number of the substituents is preferably 1 to 3.

The substituent for the thiol group which may be substituted as a substituent of R^{e1} is exemplified by a substituent similar to "a substituent for the hydroxyl group which may be substituted as a substituent of R^{e1}", among which preferable is
(1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like is exemplified);
(3) an aralkyl which may be substituted (for example, a phenyl-C₁₋₄ alkyl (for example, benzyl, phenethyl or the like) or the like is exemplified); and
(4) an aryl which may be substituted (for example, phenyl, naphthyl or the like is exemplified),
   where examples of the substituents which may be possessed by (1) an alkyl which may be substituted, (2) a cycloalkyl which may be substituted, (3) an aralkyl which may be substituted and (4) an aryl which may be substituted, which are described above, include a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxyl which may be halogenated (for example, methoxy, ethoxy, propoxy, butoxy, trifluoromethoxy, trifluoroethoxy or the like), a C₁₋₄ alkoxy-C₁₋₄ alcoxy which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

A substituent for an amino group which may be substituted as a substituent of R^{e1} is exemplified by an amino group which may have 1 to 2 substituents similar to "a substituent for the hydroxyl group which may be substituted as a substituent of R^{e1}", among which preferable is (1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified) ;
(2) a cycloalkyl which may be substituted (for example, a C₃₋₇ cycloalkyl such as cyclopropyl, cyclobutyl, cyclopentyl, cyclohexyl, cycloheptyl or the like);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified);
(5) formyl, or an acyl which may be substituted (for example, an alkanoyl which has the carbon number of 2 to 4 (for example, acetyl, propionyl, butyryl, isobutyryl or the like), an alkylsulfonyl which has the carbon number of 1 to 4 (for example, methanesulfonyl, ethanesulfonyl or the like) or the like is exemplified); and
(6) an aryl which may be substituted (for example, phenyl, naphthyl or the like is exemplified),
   where examples of the substituents which may be possessed by (1) an alkyl which may be substituted, (2) a cycloalkyl which may be substituted, (3) an alkenyl which may be substituted, (4) a cycloalkenyl which may be substituted, (5) an acyl which may be substituted and (6) an aryl which may be substituted, which are described above, include a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxy which may be halogenated (for example, methoxy, ethoxy, propoxy, butoxy, trifluoromethoxy, trifluoroethoxy or the like), C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy or the like) formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

Also, as for the substituents for an amino group which may be substituted as a substituent of R^{e1}, the substituents for the amino group may bind together to form a cyclic ring amino group (for example, a cyclic ring amino group which is formed by removing one hydrogen from the nitrogen atom constituting the ring of a 5- to 6-membered cyclic ring such as tetrahydropyrrole, piperazine, piperidine, morpholine, thiomorpholine, pyrrole, imidazole or the like, and has a bond on the nitrogen atom). Said cyclic ring amino group may have substituents, and such substituents are exemplified by a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁-₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxy which may be halogenated (for example, methoxy, ethoxy, propoxy, butoxy, trifluoromethoxy, trifluoroethoxy or the like), a C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

The acyl which may be substituted as a substituent of R^{e1} is exemplified by a group, wherein the carbonyl group or the sulfonyl group is bonded with
(1) hydrogen,
(2) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(3) a cycloalkyl which may be substituted (for example, a C₃₋₇ cycloalkyl such as cyclopropyl, cyclobutyl, cyclopentyl, cyclohexyl, cycloheptyl or the like);
(4) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(5) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified); or
(6) a 5- to 6-membered, monocyclic ring aromatic group which may be substituted (for example, phenyl, pyridyl or the like is exemplified), which is bonded with a carbonyl group or a sulfonyl group, (for example, acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl or the like), where examples of the substituents which may be possessed by (2) the alkyl which may be substituted, (3) the cycloalkyl which may be substituted, (4) the alkenyl which may be substituted, (5) the cycloalkenyl which may be substituted and (6) the 5- to 6-membered, monocyclic ring aromatic group which may be substituted, which are described above, include a halogen (for example, fluorine, chlorine, bromine, iodine or the like), nitro, cyano, a hydroxyl group, a thiol group which may be substituted (for example, thiol, a C₁-₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), a carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxy which may be halogenated (for example, methoxy, ethoxy, propoxy, butoxy, trifluoromethoxy, trifluoroethoxy or the like), a C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

The carboxyl group which may be esterified as a substituent of R^{e1} is exemplified by a group, wherein the carbonyloxy group is bonded with
(1) hydrogen,
(2) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(3) a cycloalkyl which may be substituted and may contain heteroatoms (for example, a C₃₋₇ cycloalkyl such as cyclopropyl, cyclobutyl, cyclopentyl, cyclohexyl, cycloheptyl or the like);
(4) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(5) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified); or
(6) an aryl which may be substituted (for example, phenyl, naphthyl or the like is exemplified), preferably carboxyl, a lower (C₁₋₆) alkoxycarbonyl and an aryloxycarbonyl (for example, methoxycarbonyl, ethoxycarbonyl, propoxycarbonyl, phenoxycarbonyl, naphthoxycarbonyl or the like), where examples of the substituents which may be possessed by (2) an alkyl which may be substituted, (3) a cycloalkyl which may be substituted, (4) an alkenyl which may be substituted, (5) a cycloalkenyl which may be substituted and (6) an aryl which may be substituted, which are described above, include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkoxy which may be halogenated (for example, methoxy, ethoxy, propoxy, butoxy, trifluoromethoxy, trifluoroethoxy or the like), a C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

The aromatic group for an aromatic group which may be substituted as a substituent of R^{e1} is exemplified by a 5-to 6-membered, same element or heterocyclic ringaromatic group such as phenyl, pyridyl, furyl, thienyl, pyrrolyl, imidazolyl, pyrazolyl, thiazolyl, oxazolyl, isothiazolyl, isooxazolyl, tetrazolyl, pirazinyl, pyrimidinyl, pyridazinyl, triazolyl or the like, a condensedheterocyclic ring aromatic group such as benzofuran, indole, benzothiophene, benzoxazole, benzthiazole, indazole, benzimidazole, quinoline, isoquinoline, quinoxaline, phthalazine, quinazoline, cinnoline or the like, or the like. Examples of the substituents for these aromatic groups include a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, propoxy, butoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like), where the number of the substituents is preferably 1 to 3.

One to four (preferably one or two) of these substituents of R^{e1} may be the same or different and present at any position. Also, in the case where "a 5- to 6-membered cyclic ring" of "a 5- to 6-membered cyclic ring which may be substituted", which is represented by R^{e1}, has 2 or more substituents, 2 substituents of them may be bonded together to form, for example, a lower (C₁₋₆) alkylene (for example, trimethylene, tetramethylene or the like), a lower (C₁₋₆) alkyleneoxy (for example, -CH₂-O-CH₂-, -O-CH₂-CH₂-, -O-CH₂-CH₂-CH₂-, -O-CH₂-CH₂-CH₂-CH₂-, -O-C(CH₃)(CH₃)-CH₂-CH₂- or the like), a lower (C₁₋₆) alkylenethio (for example, -CH₂-S-CH₂-, -S-CH₂-CH₂-, -S-CH₂-CH₂-CH₂-, -S-CH₂-CH₂-CH₂-CH₂-, -S-C(CH₃)(CH₃)-CH₂-CH₂- or the like), a lower (C₁₋₆) alkylenedioxy (for example, -O-CH₂-O-, -O-CH₂-CH₂-O-, -O-CH₂-CH₂-CH₂-O- or the like), a lower (C₁₋₆) alkylenedithio (for example, -S-CH₂-S-, -S-CH₂-CH₂-S-, -S-CH₂-CH₂-CH₂-S- or the like), an oxy-lower (C₁₋₆) alkylenamino (for example, -O-CH₂-NH-, -O-CH₂-CH₂-NH- or the like), an oxy-lower (C₁₋₆) alkylenethio (for example, -O-CH₂-S-, -O-CH₂-CH₂-S- or the like), a lower (C₁₋₆) alkylenamino (for example, -NH-CH₂-CH₂-, -NH-CH₂-CH₂-CH₂- or the like), a lower (C₁₋₆) alkylenediamino (for example, -NH-CH₂-NH-, -NH-CH₂-CH₂-NH- or the like), a thia-lower (C₁₋₆) alkylenamino (for example, -S-CH₂-NH-, -S-CH₂-CH₂-NH- or the like), a lower (C₂₋₆) alkenylene (for example, -CH₂-CH=CH-, -CH₂-CH₂-CH=CH-, -CH₂-CH=CH-CH₂-CH₂- or the like), a lower (C₄₋₆) alkadienylene (for example, -CH=CH-CH=CH- or the like) or the like.

Furthermore, the bivalent group which is formed by binding each other 2 substituents of R^{e1} may have 1 to 3 substituents similar to "the substituent" which may be possessed by "a 5- to 6-membered cyclic ring" of "a 5- to 6-membered cyclic ring which may be substituted", which is represented by R^{e1}, (a halogen atom, nitro, cyano, an alkyl which may be substituted, a cycloalkyl which may be substituted, the hydroxyl group which may be substituted, the thiol group which may be substituted (the sulfur atom may be oxidized to form a sulfinyl group which may be substituted or a sulfonyl group which may be substituted), an amino group which may be substituted, an acyl which may be substituted, the carboxyl group which may be esterified or amidated, an aromatic group which may be substituted and the like).

"The substituent" which may be possessed by "a 5- to 6-membered cyclic ring" of "a 5- to 6-membered cyclic ring which may be substituted", which is represented by R^{e1}, is exemplified particularly by a lower (C₁₋₄) alkyl which may be halogenated or alkoxylated with a lower (C₁₋₄) alkyl (for example, methyl, ethyl, t-butyl, trifluoromethyl, methoxymethyl, ethoxymethyl, propoxymethyl, butoxymethyl, methoxyethyl, ethoxyethyl, propoxyethyl, butoxyethyl, or the like), a lower (C₁₋₄) alkoxyl which may be halogenated or alkoxylated with a lower (C₁₋₄) alkyl (for example, methoxy, ethoxy, propoxy, butoxy, t-butoxy, trifluoromethoxy, methoxymethoxy, ethoxymethoxy, propoxymethoxy, butoxymethoxy, methoxyethoxy, ethoxymethoxy, propoxyethoxy, butoxyethoxy, methoxypropoxy, ethoxypropoxy, propoxypropoxy, butoxypropoxy or the like), a halogen (for example, fluorine, chlorine or the like), nitro, cyano, an amino group which may be substituted with 1 to 2 of a lower (C₁₋₄) alkyl, formyl or a lower (C₂₋₄) alkanoyl (for example, amino, methylamino, dimethylamino, formylamino, acetylamino or the like), a 5- to 6-membered cyclic ring amino group (for example, 1-pyrrolidinyl, 1-piperazinyl, 1-piperidinyl, 4-morpholino, 4-thiomorpholino, 1-imidazolyl, 4-tetrahydropyranyl or the like) or the like.

"A bivalent group, in which the number of atoms constituting the straight-chain portion is 1 to 4", represented by X^{e1} and X^{e2}, is exemplified by, for example, -(CH₂) _{ea'}- [ea' represents an integer of 1 to 4 (preferably, an integer of 1 to 2) ] , -(CH2) eb--Xe3- [eb' represents an integer of 0 to 3 (preferably, an integer of 0 to 1) and X^{e3} represents an imino group which may be substituted (for example, an imino group which may be substituted with a lower (C₁₋₆) lower alkyl, a lower (C₃₋₇) cycloalkyl, formyl, a lower (C₂₋₇) lower alkanoyl, a lower (C₁₋₆) lower alkoxycarbonyl or the like), the carbonyl group, the oxygen atom, the sulfur atom which may be oxidized by the oxygen atom (for example, -S (O) ₑₘ- (em represents an integer of 0 to 2) or the like)], -CH=CH-, -C≡C-, -CO-NH-, -SO₂-NH- or the like. Although these groups may be bonded with W^{e} through either of the left or right bond, it is preferable to be bonded with W^{e} through the right hand in the case of X^{e1}, whereas it is preferable to be bonded with W^{e} through the left hand in the case of X^{e2}.

As for X^{e1}, a bond, -(CH₂)_{eb'}-O- [eb' is an integer of 0, 1 or 2 (preferably, an integer of 0 to 1), -C≡C- or the like is preferable, and a bond is more preferable.

As for X^{e2}, -(CH₂) _{ea"}- [ea" is an integer of 1 to 2], -(CH₂) _{eb"}-X^{e3}- [eb" is an integer of 0 to 1 and X^{e3} is an imino group which may be substituted, the carbonyl group, the oxygen atom, the sulfur atom which may be oxidized by the oxygen atom], -CH=CH-, -CO-NH-, -SO₂-NH- or the like is preferable, and -CO-NH- is more preferable.

In the above-mentioned formula (eI), a bivalent group which is represented by formula representd by W^{e} 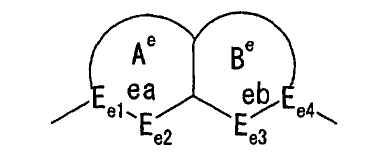 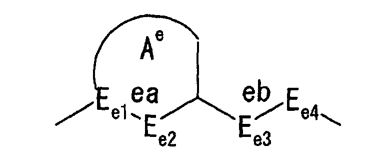 and 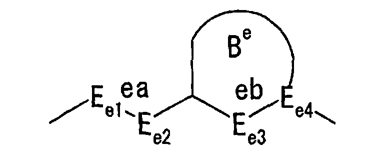 (wherein each of ring A^{e} and ring B^{e} is a 5- to 7-membered cyclic ring group which may be substituted, each of Eₑ₁ and Eₑ₄ represents the carbon atom which may be substituted or the nitrogen atom which may be substituted, each of Eₑ₂ and Eₑ₃ represents the carbon atom which may be substituted or the nitrogen atom which may be substituted, the sulfur atom which may be oxidized (for example, -S(O)ₑₘ- (em represents an integer of 0 to 2) or the like) or the oxygen atom and each of ea and eb represents to be a single bond or a double bond) represents which the bonding with adjacent X^{e1} and X^{e2} is made in a mode which is shown respectively by 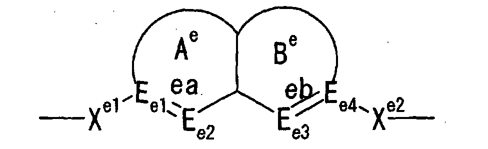 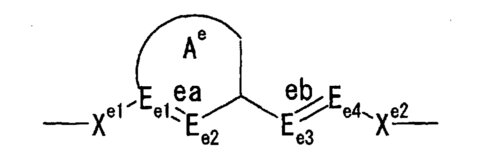 and 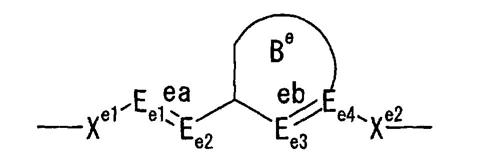 (wherein each of the symbols has the meaning defined above).

In the above-mentioned formula (eI), "a 5- to 7-membered cyclic ring" of "a 5- to 7-membered cyclic ring which may be substituted", which is represented by A^{e}, is exemplified by a 5- to 7-membered (preferably, 5- to 6-membered, saturated or unsaturated alicyclic hydrocarbon such as a C₅₋₇ cycloalkane (for example, cyclopentane, cyclohexane, cycloheptane or the like), a C₅₋₇ cycloalkene (for example, 1-cyclopentene, 2-cyclopentene, 3-cyclopentene, 2-cyclohexene, 3-cyclohexene or the like), a C₅₋₆ cycloalkadiene (for example, 2,4-cyclopentadiene, 2,4-cyclohexadiene, 2,5-cyclohexadiene or the like); a 6-membered aromatic hydrocarbon such as benzene; a 5- to 7-membered, aromatic heterocyclic ring, a saturated or unsaturated non-aromatic heterocyclic ring (aliphatic heterocyclic ring) or the like, which contains at least one (preferably, 1 to 4, more preferably 1 to 2) of heteroatoms of 1 to 3 kinds (preferably, 1 to 2 kinds) selected from the oxygen atom, the sulfur atom and the nitrogen atom; or the like.

Herein, "an aromatic heterocyclic ring" is exemplified by a 5- to 7-membered, aromatic monocyclic heterocyclic ring (for example, furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazane, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine or the like) or the like, and "a non-aromatic heterocyclic ring" is exemplified by, for example, a 5- to 7-membered (preferably, 5- to 6-membered), saturated or unsaturated non-aromatic heterocyclic ring (aliphatic heterocyclic ring) such as pyrrolidine, tetrahydrofuran, thiolan, piperidine, tetrahydropyran, morpholine, thiomorpholine, piperazine, pyran, oxepine, thiepin, azepine or the like, or a 5- to 6-membered, non-aromatic heterocyclic ring, where a part or all of the double bonds in the above-mentioned aromatic monocyclic heterocyclic ring are saturated, or the like.

As for "a 5- to 7-membered cyclic ring" of "a 5- to 7-membered cyclic ring which may be substituted", which is represented by A^{e}, a 5- to 6-membered, aromatic ring is preferable, and further benzene, furan, thiophene, pyrrole, pyridine (preferably, 6-membered) or the like is preferable, where benzene is particularly preferable.

"The substituent" which may be possessed by "a 5- to 7-membered cyclic ring" of "a 5- to 7-membered cyclic ring which may be substituted", which is represented by A^{e}, is exemplified by a group similar to "the substituent" which may be possessed by "a 5- to 6-membered cyclic ring" of "a 5- to 6-membered cyclic ring which may be substituted", which is represented by R^{e1}. Also, 1 to 4 (preferably, 1 to 2) of the substituent for A^{e} may be present at any positions of the same or different rings, and the substituent may be possessed at any substitutable position, whether it is any of the positions which are representd by Eₑ₁ and Eₑ₂ or any of other positions.

In the above-mentioned formula (eI), "a 5- to 7-membered cyclic ring" of "a 5- to 7-membered cyclic ring which may be substituted", which is represented by B, is exemplified by a 5- to 7-membered cyclic ring which may have substituents at optional, substitutable positions, which is represented by formula 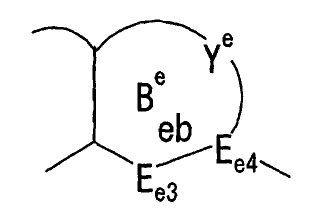 or the like.

In the above-mentioned formula (eI), the bivalent group which is represented by Y^{e} is a bivalent group, with which ring B^{e} forms a 5- to 7-membered cyclic ring which may be substituted, and is exemplified by a bivalent group such as, for example,
(1) -(CH₂)ₑₐ₁-O-(CH₂)ₑₐ₂- (each of ea1 and ea2 represents 0, 1 or 2 in the same or different manner. Hereupon, the sum of ea1 and ea2 is 2 or less), -O-(CH=CH)-, -(CH=CH)-O-,
(2) -(CH₂) _{eb1}-S (O) ₑₘ-(CH₂), _{eb2}- (em represents an integer of 0 to 2, and each of eb1 and eb2 represents 0, 1 or 2 in the same or different manner. Hereupon, the sum of b1 and b2 is 2 or less), -S (O) ₑₘ-(CH=CH)-, -(CH=CH)-S (O) ₑₘ-,
(3) -(CH₂) _{ed1}- (de1 represents 1, 2 or 3), -CH₂-(CH=CH)-, -(CH=CH)-CH₂-, -CH=CH-, -CH=,
(4) -(CH₂)ₑ₁-NH-(CH₂)ₑ₂- (each of e1 and e2 represents 0, 1 or 2 in the same or different manner. Hereupon, the sum of e1 and e2 is 2 or less), -NH-(CH=CH)-, -(CH=CH)-NH-, -(CH₂)ₑ₆-(N=CH)-(CH₂)ₑ₇-, -(CH₂)ₑ₇-(CH=N)-(CH₂)ₑ₆- (either of e6 and e7 represents 0, and the other represents 0 or 1), -(CH₂)ₑ₈-(N=N)-(CH₂)ₑ₉- (either of e8 and e9 represents 0, and the other represents 0 or 1) or the like. Specifically, the bivalent group is exemplified by, for example, a bivalent group such as -O-, -O-CH₂-, -O-CH₂-CH₂-, -O-CH=CH-, -S(O) em- (em represents an integer of 0 to 2), -S(O) em-CH₂- (em represents an integer of 0 to 2), -S(O) em-CH₂-CH₂- (em represents an integer of 0 to 2), -S(O) em-(CH=CH)-, (em represents an integer of 0 to 2), -CH₂-, -(CH₂)₂-, -(CH₂)₃-, - CH=, -CH=CH-, -CH=CH-CH₂-, -CH₂-CH=CH-, -NH-, -N=CH-, -CH=N-, -N=N- (respectively, the bonding represented starts from ring A) or the like.

In addition, said bivalent group may have a substituent, and said substituent is exemplified by a group similar to "the substituent" which may be possessed by "a 5- to 6-membered cyclic ring" of "a 5- to 6-membered cyclic ring which may be substituted", which is represented by R^{e1}, oxo and the like, where a lower (C₁₋₃) alkyl (for example, methyl, ethyl, propyl or the like), phenyl, oxo, a hydroxyl group or the like is particularly preferable. Furthermore, said bivalent group may be -O-C(O)- (the bonding represented starts from ring A) or the like. The substituent for such a bivalent group may be present at any same or different positions of 1 to 4 (preferably, 1 to 2) The substituent position may be any position which is substitutable to said bivalent group.

As the bivalent group which is represented by Y^{e}, a group such as -Y^{e}'-(CH₂)ₑₘ'-(Y^{e}' is -S(O)ₑₘ- (em is an integer of 0 to 2), -O-, -NH- or -CH₂-, and em' is an integer of 0 to 2), -CH=, -CH=CH-, -N=CH-, -(CH₂)ₑₘ' -Y^{e}'- (Y^{e}' is -S (O) ₑₘ- (em is an integer of 0 to 2), -O-, -NH- or -CH₂-, and em' is an integer of 0 to 2), -CH=N- or the like, with starting the bonding representd from ring A^{e}, is preferable, where a group such as -Y^{e}' -(CH₂) _{em'}- (Y^{e}' is -S(O)ₑₘ- (em is an integer of 0 to 2), -O-, -NH- or -CH₂-, and em' is an integer of 0 to 2), -CH=, -CH=CH-, -N=CH- or the like, with starting the bonding representd from ring A^{e}, is more preferable, and a group (ring Be is a 5- to 7-membered ring which may be substituted) such as -Y^{e}'-(CH₂)₂- (Y^{e}' represents -S(O)ₑₘ- (em represents an integer of 0 to 2), -O-, -NH- or -CH₂-), with starting the bonding representd from ring A^{e}, is most preferable.

"The substituent" which may be possessed by "a 5- to 7-membered cyclic ring" of "a 5- to 7-membered cyclic ring which may be substituted", which is represented by Be, is exemplified by a group similar to "the substituent" which may be possessed by "a 5- to 6-membered cyclic ring" of "a 5- to 6-membered cyclic ring which may be substituted", which is represented by R^{e1}. Also, such a substituent for Be may be present at any positions of 1 to 4 (preferably, 1 to 2) of the same or different rings, but it is preferable that the position of Eₑ₃ is unsubstituted.

In the above-mentioned formula (eI), it is preferable that each of Eₑ₃ and Eₑ₄ is the carbon atom which may be substituted (preferably, the carbon atom which is not substituted), and eb is a double bond.

In the above-mentioned formula (eI), "a bivalent cyclic ring group" representd by Z^{e1} is exemplified by a group which is formed by removing two hydrogen atoms from a group similar to "a 5- to 6-membered cyclic ring" of "a 5-to 6-membered cyclic ring which may be substituted", or the like, where a bivalent cyclic ring group, which is formed by removing two hydrogen atoms from benzene, furan, thiophene, pyridine, cyclopentane, cyclohexane, pyrrolidine, piperidine, piperazine, morpholine, thiomorpholine, tetrahydropyran or the like, is more preferable, and a bivalent cyclic ring group, which is formed by removing two hydrogen atoms from benzene, cyclohexane or piperidine (preferably, benzene) is most preferably used.

"A bivalent cyclic ring group" representd by Z^{e1} may have a substituent similar to "a substituent" which is possessed by "a 5- to 6-membered cyclic ring" of "a 5- to 6-membered cyclic ring which may be substituted", but it is preferable not to have a substituent other than X^{e2} and Z^{e2}, and also it is preferable that the substitution position for Z^{e2} is para to X^{e2} in the case where Z^{e1} is a 6-membered, bivalent cyclic ring group (preferably, phenylene).

In the above-mentioned formula (eI), "a bivalent group, in which the number of hydrogen atoms constituting the straight-chain portion is 1 to 4", which is represented by Z^{e2}, is exemplified by a bivalent group which has a hydrocarbon chain having the carbon number of 1 to 4, which may be substituted (for example, a C₁₋₄ alkylene, a C₂₋₄ alkenylene or the like, preferably a C₁₋₃ alkylene, more preferably methylene), or the like.

A bivalent group, which is represented by Z^{e2}, may be any group having a bivalent chain, in which the number of atoms constituting the straight-chain portion is 1 to 4, and is exemplified by, for example, an alkylene chain represented by -(CH₂)ₑₖ₁- (ek1 is an integer of 1 to 4), an alkenylene chain represented by -(CH₂)ₑₖ₂-(CH=CH)-(CH₂)ₑₖ₃- (each of ek2 and ek3 represents an integer of 0, 1 or 2 in the same or different manner. Hereupon, the sum of ek2 and ek3 is 2 or less) or the like.

A bivalent group, which is represented by X^{e1}, X^{e2} and Z^{e2}, may have a substituent at an optional position (preferably, on the carbon atom), where such a substituent may be any one that is capable of being bonded to a bivalent chain constituting the straight-chain portion, is exemplified by, for example, a lower (C₁₋₆) alkyl (for example, methyl, ethyl, propyl, isopropyl, butyl, isobutyl, sec-butyl, tert-butyl, pentyl, isopentyl, neopentyl, hexyl or the like), a lower (C₃₋₇) cycloalkyl (for example, cyclopropyl, cyclobutyl, cyclopentyl, cyclohexyl, cycloheptyl or the like), formyl, a lower (C₂₋₇) alkanoyl (for example, acetyl, propionyl, butyryl or the like), a phosphono group which may be esterified, the carboxyl group which may be esterified, the hydroxyl group, oxo or the like, where preferably a lower alkyl having the carbon number of 1 to 6 (preferably a C₁₋₃ alkyl), the hydroxyl group, oxo or the like is exemplified.

Said phosphono group which may be esterified is exemplified by a group represented by -P(O)(OR^{e7})(OR^{e8}) [wherein each of R^{e7} and R^{e8} is hydrogen, an alkyl group having the carbon number of 1 to 6 or a cycloalkyl group having the carbon number of 3 to 7, and may be bonded together to form a 5- to 7-membered cyclic ring].

In the above-mentioned formula, an alkyl group having the carbon number of 1 to 6, which is represented by R^{e7} and R^{e8}, is exemplified by methyl, ethyl, propyl, isopropyl, butyl, isobutyl, see-butyl, tert-butyl, pentyl, isopentyl, neopentyl, hexyl or the like, and a cycloalkyl having the carbon number of 3 to 7 is exemplified by cyclopropyl, cyclobutyl, cyclopentyl, cyclohexyl, cycloheptyl or the like), where a lower alkyl having the carbon number of 1 to 6 is exemplified preferably, and a lower alkyl having the carbon number of 1 to 3 is exemplified more preferably R^{e7} and R^{e8} may be the same or different, but it is preferable to be the same. Also, in the case where R^{e7} and R^{e8} are bonded together to form a 5- to 7-membered cyclic ring, R^{e7} and R^{e8} are bonded together to form a straight-chained C₂₋₄ alkylene side chain represented by -(CH₂)₂-, -(CH₂)₃-, -(CH₂)₄-. Said side chain may have substituents, and such a substituent is exemplified by, for example, the hydroxyl group, a halogen or the like.

The carboxyl group, which is esterified for the carboxyl group which may be esterified, is exemplified by a group, wherein the carboxyl group is bonded with an alkyl group having the carbon number of 1 to 6 or a cycloalkyl group having the carbon number of 3 to 7, such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl or the like.

A bivalent group, which is represented by Z^{e2}, is exemplified by a C₁₋₃ alkylene which may be substituted, where a C₁₋₃ alkylene which may be substituted with a C₁₋₃ alkyl, the hydroxyl group or oxo is more preferable.

Furthermore, as for a bivalent group, which is represented by Z^{e2}, a group represented by -Z^{e}'-(CH₂)en- or -(CH₂) en-Z^{e}' - (Z^{e}' is -CH (OH)-, -C (O)- or -CH₂-, en is an integer of 0 to 2 and each methylene group may have 1 to 2 groups of the same or different substituent), with starting from the benzene ring, is preferable, a group represented by -Z^{e}' -(CH₂) en- (Z^{e}' represents -CH (OH)-, -C (O) - or -CH₂-, en represents an integer of 0 to 2 (preferably, en is 0) and each methylene group may have 1 to 2 groups of the same or different substituents), with starting from the benzene ring, is more preferable and methylene is most preferable.

In the above-mentioned formula (eI), "an amino group" of "an amino group which may be substituted, where the nitrogen atom may be converted into a quaternary ammonium or the N-oxide" represented by R^{e2}, is exemplified by an amino group which may have 1 to 2 substituents, an amino group which has 3 substituents, where the nitrogen atom may be converted into a quaternary ammonium, or the like. In the case where the substituents on the nitrogen atom are 2 or more, these substituents may be the same or different, and in the case where the substituents on the nitrogen atom are 3, the amino group may be of any type of -N⁺(R^{e})₃, -N⁺(R^{e})₂R^{e'}, -N⁺R^{e}R^{e}'R^{e}" (each of R^{e}, R^{e'} and R^{e"} is hydrogen or a substituent in a different manner). In addition, a counter anion for the amino group, in which the nitrogen atom is converted into a quaternary ammonium, is exemplified by, in addition to an anion of a halogen atom (for example, Cl⁻, Br⁻, I⁻ or the like) or the like, an anion derived from an inorganic acid such as hydrochloric acid, hydrobromic acid, nitric acid, sulfuric acid, phosphoric acid and the like, an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like and an anion derived from an acidic amino acid such as aspartic acid, glutamic acid and the like, where Cl⁻, Br⁻, I⁻ or the like is more preferable.

Examples of substituents for said amino group include substituents such as,
(1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl or the like is exemplified);
(2) a cycloalkyl which may be substituted (for example, a C₃₋₈ cycloalkyl such as cyclopropyl, cyclobutyl, cyclopentyl, cyclohexyl, cycloheptyl, cianooctyl or the like is exemplified);
   (2-1) said cycloalkyl contains one heteroatom selected from the sulfur atom, the oxygen atom and the nitrogen atom, and may form oxirane, thiolan, aziridine, tetrahydrofuran, tetrahydrothiophene, pyrrolidine, tetrahydropyran, tetrahydrothiopyran, tetrahydrothiopyran 1-oxide, piperidine or the like (preferably, a 6-membred ring such as tetrahydropyran, tetrahydrothiopyran, piperidine or the like), where the binding position with the amino group is preferably position 3 or position 4 (preferably, position 4) ;
   (2-2) also, said cycloalkyl may condensed with the benzene ring to form an indane (for example, indan-1-yl, indan-2-yl or the like), tetrahydronaphthalene, (for example, tetrahydronaphthalen-5-yl, tetrahydronaphthalen-6-yl or the like) or the like (preferably, indane, etc.);
   (2-3) furthermore, said cycloalkyl may be crosslinked via a straight-chained, atom chain having the carbon number of 1 to 2 to form a crosslinked, cyclic hydrocarbon residue such as bicyclo[2.2.1]heptyl, bicyclo[2.2.2]octyl, bicyclo[3.2.1]octyl, bicyclo[3.2.2]nonyl or the like (preferably, a cycloalkyl having a crosslink via a straight-chained, atom chain having the carbon number of 1 to 2, or the like, more preferably bicyclo [2.2.1] heptyl or the like);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified);
(5) an aralkyl which may be substituted (for example, a phenyl-C₁₋₄ alkyl (for example, benzyl, phenethyl or the like) or the like is exemplified);
(6) formyl or an acyl which may be substituted (for example, an alkanoyl which has the carbon number of 2 to 4 (for example, acetyl, propionyl, butyryl, isobutyryl or the like), an alkylsulfonyl which has the carbon number of 1 to 4 (for example, methanesulfonyl, ethanesulfonyl or the like), an alkoxycarbonyl which has the carbon number of 1 to 4 (methoxycarbonyl, ethoxycarbonyl, tert-butoxycarbonyl or the like), an aralkyloxycarbonyl which has the carbon number of 7 to 10 (for example, benzyloxycarbonyl or the like) or the like is exemplified);
(7) an aryl which may be substituted (for example, phenyl, naphthyl or the like is exemplified); and
(8) a heterocyclic ring group which may be substituted (a group which is formed by removing one hydrogen atom from a 5- to 6-membered, aromatic heterocyclic ring, which contains 1 to 4 heteroatoms of 1 to 2 kinds selected from the nitrogen atom, the sulfur atom and the oxygen atom, such as furan, thiophene, pyrrole, imidazole, pyrazole, thiazole, oxazole, isothiazole, isoxazole, tetrazole, pyridine, pyrazine, pyrimidine, pyridazine, triazole or the like, a group which is formed by removing one hydrogen atom from a 5- to 6-membered, non-aromatic heterocyclic ring, which contains 1 to 4 heteroatoms of 1 to 2 kinds selected from the nitrogen atom, the sulfur atom and the oxygen atom, such as tetrahydrofuran, tetrahydrothiophene, dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran or the like, or the like; preferably, a group, which is formed by removing one hydrogen atom from a 5- to 6-membered, non-aromatic heterocyclic ring, or the like; and more preferably, a group, which is formed by removing one hydrogen atom from a 5- to 6-membered, non-aromatic heterocyclic ring, which has one heteroatom, such as tetrahydrofuran, piperidine, tetrahydropyran, tetrahydrothiopyran or the like) and the like. In addition, the substituents of said amino group may be bonded each other to form a 5- to 7-membered, cyclic amino such as piperidine, piperazine, morpholine, thiomorpholine or the like.

Examples of the substituents which may be possessed by (1) an alkyl which may be substituted, (2) a cycloalkyl which may be substituted, (3) an alkenyl which may be substituted, (4) a cycloalkenyl which may be substituted, (5) an aralkyl which may be substituted, (6) an acyl which may be substituted, (7) an aryl which may be substituted and (8) a heterocyclic ring group which may be substituted, which are described above, include a halogen (for example, fluorine, chlorine, bromine, iodine or the like), a lower (C₁₋₄) alkyl which may be halogenated, a C₁₋₄ alkoxyl which may be halogenated (for example, methoxy, ethoxy, propoxy, butoxy, trifluoromethoxy, trifluoroethoxy or the like), a C₁₋₄ alkylenedioxy (for example, -O-CH₂-O-, -O-CH₂-CH₂-O- or the like), formyl, a C₂₋₄ alkanoyl (for example, acetyl, propionyl or the like), a C₁₋₄ alkylsulfonyl (for example, methanesulfonyl, ethanesulfonyl or the like), a phenyl-lower (C₁₋₄) alkyl, a C₃₋₇ cycloalkyl, cyano, nitro, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a lower (C₁₋₄) alkoxycarbonyl, a lower (C₇₋₁₀) aralkyloxycarbonyl, the oxo group (preferably, a halogen, a lower (C₁₋₄) alkyl.which may be halogenated, a C₁₋₄ alkoxyl which may be halogenated, a phenyl-lower (C₁₋₄) alkyl, a C₃₋₇ cycloalkyl, cyano, a hydroxyl group or the like) and the like, where the number of the substituents is preferably 1 to 3.

In the above-mentioned formula (eI), "an amino group which may be substituted, where the nitrogen atom may be converted into a quaternary ammonium or the N-oxide" representd by R^{e2}, is preferably an amino group which may have 1 to 3 substituents selected from,
(1) a straight-chained or branched, lower (C₁₋₆) alkyl which may have 1 to 3 groups of a halogen, cyano, a hydroxyl group or a C₃₋₇ cycloalkyl;
(2) a C₅₋₈ cycloalkyl which may have 1 to 3 groups of a halogen, a lower (C₁₋₄) alkyl which may be halogenated or a phenyl-lower (C₁₋₄) alkyl, may contain one heteroatom selected from the sulfur atom, the oxygen atom and the nitrogen atom, may be condensed with the benzene ring and may be crosslinked via a straight-chained, atom chain having the carbon number of 1 to 2, (for example, cyclopentyl, cyclohexyl, cycloheptyl cyclooctyl, tetrahydropyranyl, tetrahydrothiapyranyl, piperidinyl, indanyl, tetrahydronaphthalenyl, bicyclo[2.2.1]heptyl or the like);
(3) a phenyl-lower (C₁₋₄) alkyl which may have 1 to 3 groups of a halogen, a lower (C₁₋₄) alkyl which may be halogenated or a C₁₋₄ alkoxyl which may be halogenated;
(4) a phenyl which may have 1 to 3 groups of a halogen, a lower (C₁-₄) alkyl which may be halogenated or a C₁₋₄ alkoxyl which may be halogenated; and
(5) a 5- to 6-membered, aromatic heterocyclic ring which may have 1 to 3 groups of a halogen, a lower (C₁₋₄) alkyl which may be halogenated, a C₁₋₄ alkoxy which may be halogenated, a lower (C₁₋₄) alkoxy-(C₁₋₄) lower alkoxy which may be halogenated, a phenyl-lower (C₁₋₄) alkyl, cyano or the hydroxyl (for example, a group which is formed by removing one hydrogen atom from furan, thiophene, pyrrole, pyridine or the like).

In the above-mentioned formula (eI), "a nitrogen-containing, heterocyclic ring" of "a nitrogen-containing, heterocyclic group which may be substituted and may contain the sulfur atom or the oxygen atom as a ring-constituting atom, where the nitrogen atom may be converted into a quaternary ammonium or the N-oxide" is exemplified by a 5-to 6-membered, aromatic heterocyclic ring, which may contain, in addition to one nitrogen atom, 1 to 3 heteroatoms of 1 to 2 kinds selected from the nitrogen atom, the sulfur atom and the oxygen atom, such as pyrrole, imidazole, pyrazole, thiazole, oxazole, isothiazole, isoxazole, tetrazole, pyridine, pyrazine, pyrimidine, pyridazine, triazole or the like, a 5- to 8-membered, non-aromatic heterocyclic ring, which may contain, in addition to one nitrogen atom, 1 to 3 hetero atoms of 1 to 2 kinds selected from the nitrogen atom, the sulfur atom and the oxygen atom, such as pyrrolidine, pyrroline, imidazolidine, imidazoline, pyrazolidine, pyrazoline, piperidine, piperazine, oxazine, oxadiazine, thiazine, thiadiazine, morpholine, thiomorpholine, azacycloheptane, azacyclooctane (azokane) or the like, or the like, where each of these nitrogen-containing, heterocyclic rings may be crosslinked via a straight-chained, atom chain having the carbon number of 1 to 2 and may form a crosslinked, nitrogen-containing, heterocyclic ring such as azabicyclo[2.2.1]heptane, azabicyclo[2.2.2]octane (quinuclidine) or the like (preferably, piperidine, which may have a crosslink via a straight-chained, atom chain having the carbon number of 1 to 2, or the like).

Among the specific examples of the above-mentioned nitrogen-containingheterocyclic ring, pyridine, imidazole, pyrrolidine, piperidine, piperazine, morpholine, thiomorpholine and azabicyclo [2.2.2] octane (preferably, 6-membered, cyclic rings) are preferable.

The nitrogen atom of said "nitrogen-containingheterocyclic ring" may be converted into a quaternary ammonium, or may be oxidized In the case where the nitrogen atom of said "nitrogen-containingheterocyclic ring" is converted into a quaternary ammonium, a counter anion for "the nitrogen-containingheterocyclic ring group, in which the nitrogen atom is converted into a quaternary ammonium", is exemplified by, in addition to an anion of a halogen atom (for example, Cl⁻, Br⁻, I⁻ or the like) or the like, an anion derived from an inorganic acid such as hydrochloric acid, hydrobromic acid, nitric acid, sulfuric acid, phosphoric acid and the like, an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like and an anion derived from an acidic amino acid such as aspartic acid, glutamic acid and the like, where Cl⁻, Br⁻, I⁻ or the like is more preferable.

Said "nitrogen-containingheterocyclic ring" may be bonded with a bivalent group, which is represented by Z^{e2}, via either of the carbon atom or the nitrogen atom and may be bonded on the carbon atom constituting the cyclic ring, as in the case of 2-pyridyl, 3-pyridyl, 2-piperidyl or the like, whereas a bonding as in the case of 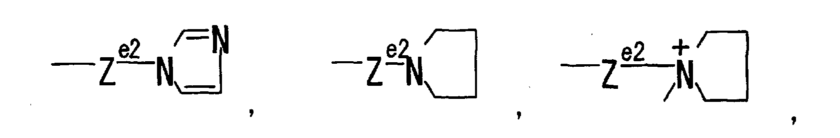 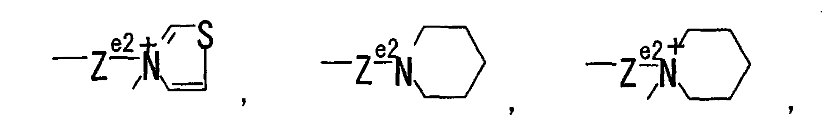 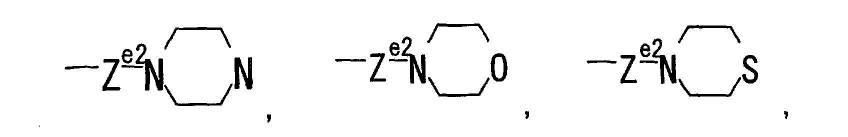 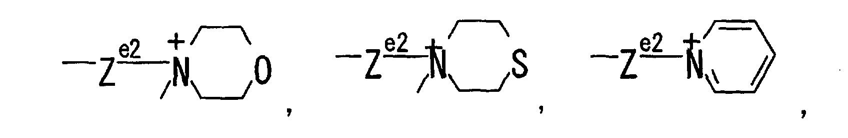 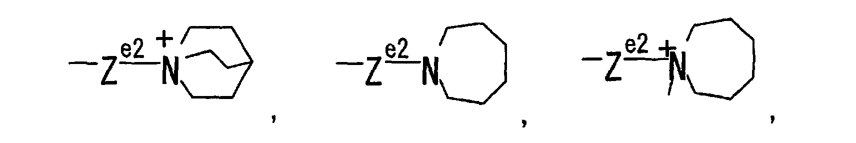 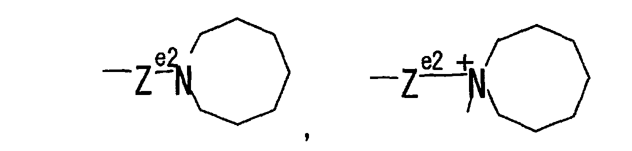 or the like is preferable.

Examples of substituents of said "nitrogen-containingheterocyclic ring" include a halogen (for example, fluorine, chlorine, bromine, iodine or the like), a lower (C₁₋₄) alkyl which may be substituted, a lower (C₁₋₄) alkoxyl which may be substituted, a phenyl which may be substituted, a mono- or diphenyl-lower (C₁₋₄) alkyl which may be substituted, a C₃₋₇ cycloalkyl which may be substituted, cyano, nitro, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a lower (C₁₋₄) alkoxycarbonyl, formyl, a (C₂₋₄) alkanoyl, a lower (C₁₋₄) alkylsulfonyl, a heterocyclic ring group which may be substituted (e.g. a group which is formed by removing one hydrogen atom from a 5- to 6-membered, aromatic heterocyclic ring, which contains 1 to 4 heteroatoms of 1 to 2 kinds selected from the nitrogen atom, the sulfur atom and the oxygen atom, such as furan, thiophene, pyrrole, imidazole, pyrazole, thiazole, oxazole, isothiazole, isoxazole, tetrazole, pyridine, pyrazine, pyrimidine, pyridazine, triazole or the like, a group which is formed by removing one hydrogen atom from a 5- to 6-membered, non-aromatic heterocyclic ring, which contains 1 to 4 heteroatoms of 1 to 2 kinds selected from the nitrogen atom, the sulfur atom and the oxygen atom, such as tetrahydrofuran, tetrahydrothiophene, dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran or the like, or the like, where the number of the substituents is preferably 1 to 3. Also, the nitrogen in said "nitrogen-containingheterocyclic ring" may be oxidized.

Examples of the substituents, which may be possessed by "a lower (C₁₋₄)alkyl which may be substituted", "a lower (C₁₋₄) alkoxyl which may be substituted", "a phenyl which may be substituted", "a mono- or diphenyl-lower (C₁₋₄) alkyl which may be substituted", "a C₃₋₇ cycloalkyl which may be substituted" and "a heterocyclic ring group which may be substituted", as the substituents which may be possessed by said "nitrogen-containingheterocyclic ring", include, for example, a halogen (for example, fluorine, chlorine, bromine, iodine or the like), a lower (C₁₋₄) alkyl which may be halogenated, a lower (C₃₋₁₀) cycloalkyl, a lower (C₃₋₁₀) cycloalkenyl, a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like), a C₁₋₃ alkylenedioxy (for example, methylenedioxy, ethylenedioxy or the like), cyano, nitro, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁-₄ alkylcarbamoyl, a di-C₁-₄ alkylcarbamoyl or the like), a lower (C₁₋₄) alkoxycarbonyl and the like, where the number, of the substituents is preferably 1 to 3.

In the above-mentioned formula (eI), the substituent, which may be possessed by "a nitrogen-containingheterocyclic ring" of "a nitrogen-containing heterocyclic ring group which may be substituted and may contain the sulfur atom or the oxygen atom as a ring-constituting atom, where the nitrogen atom may be converted into a quaternary ammonium or the N-oxide" is exemplified preferably by (1) a halogen, (2) cyano, (3) a hydroxyl group, (4) a carboxyl group, (5) a lower (C₁₋₄) alkoxycarbonyl, (6) a lower (C₁₋₄) alkyl which may be substituted with a halogen, a hydroxyl group or a lower (C₁₋₄) alkoxy, (7) a lower (C₁₋₄) alkoxy which may be substituted with a halogen, the hydroxyl group or a lower (C₁₋₄) alkoxy, (8) a phenyl which may be substituted with a halogen, a lower (C₁₋₄) alkyl, a hydroxyl group, a lower (C₁₋₄) alkoxy or a C₁₋₃ alkylenedioxy, (9) a mono- or diphenyl-lower (C₁₋₄) alkyl which may be substituted with a halogen, a lower (C₁₋₄) alkyl, the hydroxyl group, a lower (C₁₋₄) alkoxy or a C₁₋₃ alkylenedioxy, (10) a group which is formed by removing one hydrogen atom from a 5- to 6-membered aromatic heterocyclic ring, such as furan, thiophene, pyrrole, pyridine or the like, or the like.

In the above-mentioned formula (eI), "a group which is bonded via the sulfur atom", which is represented by R^{e2}, is exemplified by a group represented by -S(O)em-R^{eS} (wherein em represents an integer of 0 to 2 and R^{eS} is a substituent) In the above-mentioned formula, examples of the substituent representd by R^{eS} include, for example,
(1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like is exemplified);
(3) an aralkyl which may be substituted (for example, a phenyl-C₁₋₄ alkyl (for example, benzyl, phenethyl or the like) or the like is exemplified); and
(4) an aryl which may be substituted (for example, phenyl, naphthyl or the like is exemplified), where examples of the substituents which may be possessed by (1) an alkyl which may be substituted, (2) a cycloalkyl which may be substituted, (3) an aralkyl which may be substituted and
(4) an aryl which may be substituted, which are described above, include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like) or the like,
where the number of the substituents is preferably 1 to 3.

In the above-mentioned formula (eI), "the hydrocarbon group" for the hydrocarbon group which may be substituted, which is represented by R^{e5}' and R^{e6}', in "a group represented by formula 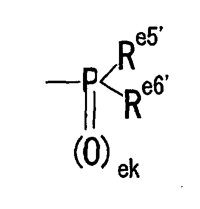 (wherein ek represents 0 or 1, the phosphorus atom may form a phosphonium salt when ek is 0, and each of R^{e5'} and R^{e6'} is a hydrocarbon group which may be substituted, a hydroxyl group which may be substituted or an amino group which may be substituted (preferably, the hydrocarbon group which may be substituted or an amino group which may be substituted; more preferably the hydrocarbon group which may be substituted) and R^{e5}' and R^{e6'} may bind each other to form a cyclic ring group together with the adjacent phosphorus atom)", which is represented by R^{e2}, is exemplified by,
(1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified);
(5) an alkinyl which may be substituted (for example, an alkinyl which has the carbon number of 2 to 10 such as ethinyl, 1-propinyl, 2-propinyl, 1-butinyl, 2-pentinyl, 3-hexinyl or the like, preferably a lower (C₂₋₆) alkinyl, or the like is exemplified);
(6) an aralkyl which may be substituted (for example, a phenyl-C₁₋₄ alkyl (for example, benzyl, phenethyl or the like) or the like is exemplified); and
(7) an aryl which may be substituted (for example, phenyl, naphthyl or the like is exemplified), where examples of the substituents which may be possessed by (1) an alkyl which may be substituted, (2) a cycloalkyl which may be substituted, (3) an alkenyl which may be substituted, (4) a cycloalkenyl which may be substituted, (5) an alkinyl which may be substituted, (6) an aralkyl which may be substituted and (7) an aryl which may be substituted, which are described above, include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like) or the like,
where the number of the substituents is preferably 1 to 3.

The hydroxyl group which may be substituted, which is represented by R^{e5}' and R^{e6}', is exemplified by a hydroxyl group which may be substituted by, for example, (1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified);
(5) an aralkyl which may be substituted (for example, a phenyl-C₁₋₄ alkyl (for example, benzyl, phenethyl or the like) or the like is exemplified);
(6) formyl or an acyl which may be substituted (for example, an alkanoyl which has the carbon number of 2 to 4 (for example, acetyl, propionyl, butyryl, isobutyryl or the like), an alkylsulfonyl which has the carbon number of 1 to 4 (for example, methanesulfonyl, ethanesulfonyl or the like) or the like is exemplified); and
(7) an aryl which may be substituted (for example, phenyl, naphthyl or the like is exemplified).

Examples of the substituents which may be possessed by (1) an alkyl which may be substituted, (2) a cycloalkyl which may be substituted, (3) an alkenyl which may be substituted, (4) a cycloalkenyl which may be substituted, (5) an aralkyl which may be substituted, (6) an acyl which may be substituted and (7) an aryl which may be substituted, which are described above, include a halogen (for example, fluorine, chlorine, bromine, iodine or the like), nitro, cyano, the hydroxyl group, the thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like) and the like, where the number of the substituents is preferably 1 to 3.

Also, in the above-mentioned formula, R^{e5'} and R^{e6'} may bind each other to form a cyclic ring group together with the adjacent phosphorus atom (preferably, a 5- to 7-membered cyclic ring). Such a cyclic ring group may have substituents and examples of said substituents include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic amino such as tetrahydropyrrole, piperazine, piperidine, morpholine, thiomorpholine, pyrrole, imidazole or the like, or the like), the carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like) and the like, where the number of the substituents is preferably 1 to 3.

In the above-mentioned formula (eI), a counter anion in the case where the phosphorus atom forms a phosphonium salt is exemplified by, in addition to an anion of a halogen atom (for example, Cl⁻, Br⁻, I⁻ or the like) or the like, an anion derived from an inorganic acid such as hydrochloric acid, hydrobromic acid, nitric acid, sulfuric acid, phosphoric acid and the like, an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like and an anion derived from an acidic amino acid such as aspartic acid, glutamic acid and the like, where Cl⁻, Br⁻, I⁻ or the like is more preferable.

An amino group which may be substituted, which is represented by R^{e5}', and R^{e6}', is exemplified by an amino group which may have 1 to 2 groups such as,
(1) an alkyl which may be substituted (for example, a C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl or the like, preferably a lower (C₁₋₆) alkyl, or the like is exemplified);
(2) a cycloalkyl which may be substituted (for example, a C₃₋₇ cycloalkyl such as cyclopropyl, cyclobutyl, cyclopentyl, cyclohexyl, cycloheptyl or the like);
(3) an alkenyl which may be substituted (for example, an alkenyl which has the carbon number of 2 to 10 such as allyl, crotyl, 2-pentenyl, 3-hexenyl or the like, preferably a lower (C₂₋₆) alkenyl, or the like is exemplified);
(4) a cycloalkenyl which may be substituted (for example, an cycloalkenyl which has the carbon number of 3 to 7 such as 2-cyclopentenyl, 2-cyclohexenyl, 2-cyclopentenylmethyl, 2-cyclohexenylmethyl or the like, or the like is exemplified);
(5) formyl or an acyl which may be substituted (for example, an alkanoyl which has the carbon number of 2 to 4 (for example, acetyl, propionyl, butyryl, isobutyryl or the like), an alkylsulfonyl which has the carbon number of 1 to 4 (for example, methanesulfonyl, ethanesulfonyl or the like) or the like is exemplified); and
(6) an aryl which may be substituted (for example, phenyl, naphthyl or the like is exemplified).

Examples of the substituents which may be possessed by (1) an alkyl which may be substituted, (2) a cycloalkyl which may be substituted, (3) an alkenyl which may be substituted, (4) a cycloalkenyl which may be substituted, (5) an acyl which may be substituted and (6) an aryl which may be substituted, which are described above, include a halogen (for example, fluorine, chlorine, bromine, iodine or the like), nitro, cyano, a hydroxyl group, a thiol group which may be substituted (for example, thiol, a C₁₋₄ alkylthio or the like), an amino group which may be substituted (for example, amino, a mono-C₁₋₄ alkylamino, a di-C₁₋₄ alkylamino, a 5- to 6-membered cyclic ring amino such as tetrahydropyrrole, piperazine, piperidine, morpholine, thiomorpholine, pyrrole, imidazole or the like, or the like), a carboxyl group which may be esterified or amidated (for example, carboxyl, a C₁₋₄ alkoxycarbonyl, carbamoyl, a mono-C₁₋₄ alkylcarbamoyl, a di-C₁₋₄ alkylcarbamoyl or the like), a C₁₋₄ alkyl which may be halogenated (for example, trifluoromethyl, methyl, ethyl or the like), a C₁₋₄ alkoxy which may be halogenated (for example, methoxy, ethoxy, trifluoromethoxy, trifluoroethoxy or the like), formyl, a C₂₋₄ alkanoyl (for example, acetyl, propionyl or the like), a C₁₋₄ alkylsulfonyl (for example, methanesulfonyl, ethanesulfonyl or the like) and the like, where the number of the substituents is preferably 1 to 3.

The substituents for "an amidino group which may be substituted" and "a guanidino group which may be substituted" each of which is represented by R^{e2} are exemplified by substituents similar to those for "an amino group which may be substituted, where the nitrogen atom may be converted into a quaternary ammonium or the N-oxide" which is represented by R^{e2}.

It is preferable that R^{e2} is (1) an amino group which may be substituted, where the nitrogen atom may be converted into a quaternary ammonium or the N-oxide, (2) a nitrogen-containing heterocyclic ring group which may be substituted and may contain the sulfur atom or the oxygen atom as a ring-constituting atom, where the nitrogen atom may be converted into a quaternary ammonium or the N-oxide, (3) an amidino group which may be substituted or (4) a guanidino group which may be substituted and it is more preferable that R^{e2} is an amino group which may be substituted, where the nitrogen atom may be converted into a quaternary ammonium or the N-oxide, or the like Also, R^{e2} may be an amidino group which may be substituted or a guanidino group which may be substituted.

It is more preferable that R^{e2} is a group represented by -NR^{e}R^{e"} or -N⁺R^{e}R^{e'} R^{e"} (wherein each of R^{e}, R^{e'} and R^{e"} represents an aliphatic hydrocarbon group (an aliphatic-chain hydrocarbon group and an aliphatic, cyclic hydrocarbon group) which may be substituted or an alicyclic (non-aromatic) heterocyclic ring group which may be substituted).

In the above-mentioned formula, "an aliphatic hydrocarbon group which may be substituted" and "an alicyclic heterocyclic ring group which may be substituted", which are representd by R^{e}, R^{e}' and R^{e}", are exemplified by substituents similar to "an alicyclic hydrocarbon group which may be substituted (for example, an alkyl, a cycloalkyl, an alkenyl, a cycloalkenyl or the like, each of which may be substituted)" and "an alicyclic, heterocyclic ring group which may be substituted (for example, a 5- to 6-membered non-aromatic heterocyclic ring which may be substituted or the like)", which are exemplified by as the substituents which may be possessed by "an amino group which may be substituted" representd by R^{e2}.

Especially, as for R^{e} and R^{e}', an aliphatic hydrocarbon group which may be substituted (for example, an alkyl, an alkenyl or the like, each of which may be substituted) is preferable, a C₁₋₆ alkyl group which may be substituted is more preferable and the methyl group which may be substituted is particularly preferable.

It is preferable that R^{e}" is an alicyclic hydrocarbon group which may be substituted (preferably, a C₃₋₈ cycloalkyl group which may be substituted; more preferably, a cyclohexyl which may be substituted) or an alicyclic heterocyclic ring group which may be substituted (preferably, a saturated alicyclic heterocyclic ring group which may be substituted (preferably, a 6-membered cyclic ring group); more preferably, tetrahydropyranyl which may be substituted, tetrahydrothiopyranyl which may be substituted or piperidyl which may be substituted; and particularly preferably, tetrahydropyranyl which may be substituted).

In the above formula (eIa), R^{e1} and Z^{e2} have the same meanings given above.

In the above formula (eIa), a group of the formula: 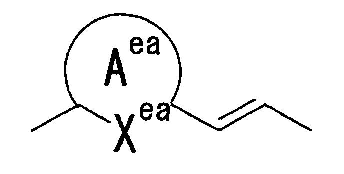 or 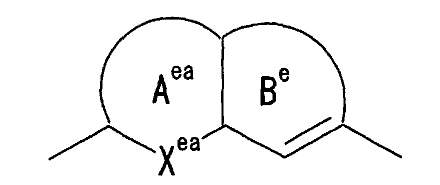 represented by W^{ea} binds to adjacent groups in the following manner: 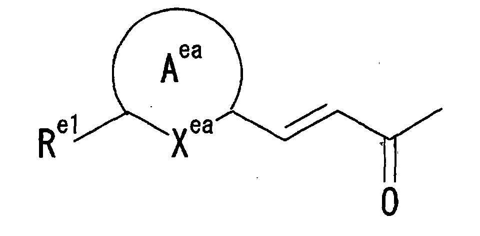 or

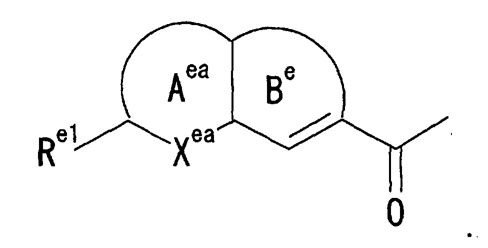

In the above formula, examples of the "5- to 6-membered aromatic ring" in the "optionally substituted 5-to 6-membered aromatic ring" represented by A^{ea} include 6-membered aromatic hydrocarbon such as benzene, etc.; 5- to 6-membered aromatic heterocyclic ring containing 1 to 3 hetero-atoms consisting of 1 to 2 kinds of hetero-atoms selected from oxygen atom, sulfur atom and nitrogen atom such as furan, thiophene, pyrrole, imidazole, pyrazole, thiazole, oxazole, isothiazole, isoxazole, pyridine, pyrazine, pyrimidine, pyridazine, triazole, etc.; etc. Among others, benzene, furan, thiophene, pyridine (preferably, 6-membered ring) etc. are preferable, and in particular benzene is preferable.

Examples of the "substituents", which the "5- to 6-membered aromatic ring" in the "optionally substituted 5-to 6-membered aromatic ring" represented by A^{ea} may have, are similar to the "substituents" which the "5- to 6-membered ring" in the "optionally substituted 5- to 6-membered ring" represented by R^{e1} may have. The number of said substituents for the ring A^{ea} is 1-4 (preferably 1-2), and they may be same or different and present at any possible position (e.g. the position of the group X^{ea} and the other positions) on the ring represented by A^{ea}.

In the above formula, examples of the "5- to 7-membered ring" in the "optionally substituted 5- to 7-membered ring" represented by B^{e} include a 5- to 7-membered ring group of the formula: 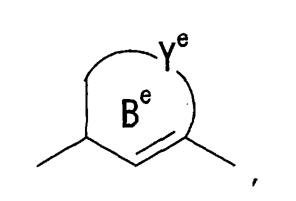 which may have a substituent at optional, substitutable positions, etc.

In the above formula, Y^{e} has the meaning given above. One to four (preferably one or two) of these substituents for A^{ea} may be the same or different from each other and present at any positions in the ring, and it is preferable that the carbon atom at the position ea in the group: 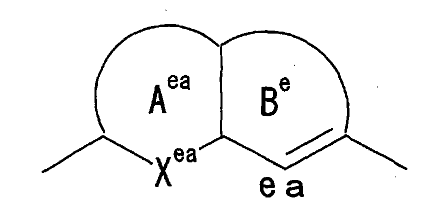 represented by W^{ea} is not substituted.

In the above formula (eIa), the amino group that may be substituted, where the nitrogen atom may be converted into a quaternary ammonium, represented by R^{e2a} include those mentioned as the amino group that may be substituted, where the nitrogen atom may be converted into a quaternary ammonium, represented by R^{e2}.

In the above formula (eIa), the group represented by the formula represented by R^{e2a}: 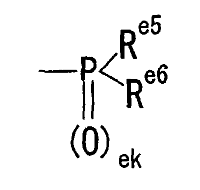 (wherein ek represents 0 or 1, the phosphorus atom may form a phosphonium salt when ek is 0, and each of R^{e5} and R^{e6} represents the hydrocarbon atom that may be substituted or an amino group that may be substituted and R^{e5} and R^{e6} may bind each other to form a cyclic ring group together with the adjacent phosphorus atom), "hydrocarbon atom that may be substituted" or "an amino group that may be substituted" represented by R^{e5} or R^{e6} and a cyclic group in case of forming a cyclic ring group by combining R^{e5} and R^{e6} together with the adjacent phosphorus atom include those mentioned as examples for the above R^{e5'} and R^{e6'}.
In the above formula, a counter anion in case that the phosphorus atom is converted into a quaternary ammonium, is exemplified by, in addition to an anion of a halogen atom (for example, Cl⁻, Br⁻, I⁻ or the like) or the like, an anion derived from an inorganic acid such as hydrochloric acid, hydrobromic acid, nitric acid, sulfuric acid, phosphoric acid and the like, an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like and an anion derived from an acidic amino acid such as aspartic acid, glutamic acid and the like, where Cl⁻, Br⁻, I⁻ or the like is more preferable.

As the R^{e2a}, "an amino group that may be substituted, where the nitrogen atom may be converted into a quaternary ammonium and the group represented by the formula:

- N+R^{e}R^{e}'R^{e}"

wherein each of R^{e}, R^{e}' and R^{e}" is an aliphatic hydrocarbon group which may be substituted or an alicyclic heterocyclic group which may be substituted are more preferable.

As an compound of the formula (eIa), a compound of the formula: 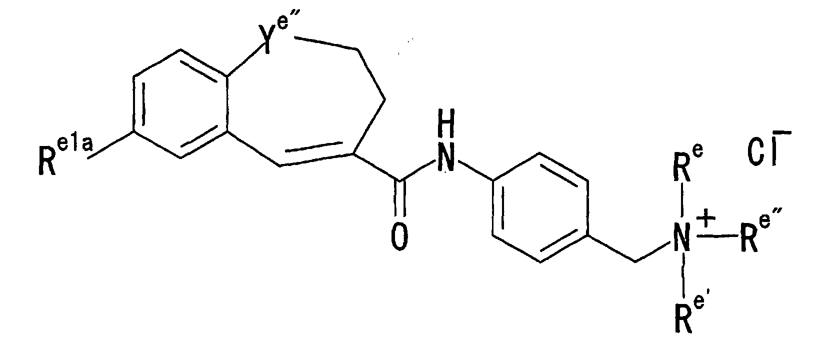 wherein R^{e1a} is an optionally substituted phenyl group or an optionally substituted thienyl group; Y^{e}" is -CH₂-, -O-, or - S-; and R^{e}, R^{e}' and R^{e}" are independently an optionally substituted aliphatic hydrocarbon group or an optionally substituted alicyclic heterocyclic ring group, is preferable.

In the above formula, the "substituent" which may be possessed by the "phenyl group" in the "optionally substituted phenyl group" and the "thienyl group" in the "potionally substituted thienyl group", each of which is represented by R^{e1a}, include those for the optionally substituted 5- or 6- membered cyclic ring group represented by the above R^{e1}.

Examples of the "optionally substituted aliphatic hydrocarbon group" and the "optionally substituted alicyclic heterocyclic ring group" represented by R^{e}, R^{e}' or R^{e}" include those exemplified by the substituents for the "optionally substituted amino group" represented by R^{e2a}. Among them, as the group R^{e} or R^{e'}, an optionally substituted chain hydrocarbon group is preferable, an optionally substituted C₁-₆ alkyl group is more preferable, and an optionally substituted methyl group is most preferable; and as the group R", an optionally substituted alicyclic hydrocarbon group (more preferably, an optionally substituted C₃₋₈ cycloalkyl group; more preferably, an optionally substituted cyclohexyl) or an optionally substituted alicyclic heterocyclic ring group (preferably, an optionally substituted saturated alicyclic heterocyclic ring group (preferably 6-membered ring group); more preferably, an optionally substituted tetrahydropyranyl, an optionally substituted tetrahydrothiopyranyl or an optionally substituted piperidyl; most preferably, an optionally substituted tetrahydropyranyl) is preferable. Furthermore, a compound of the formula: 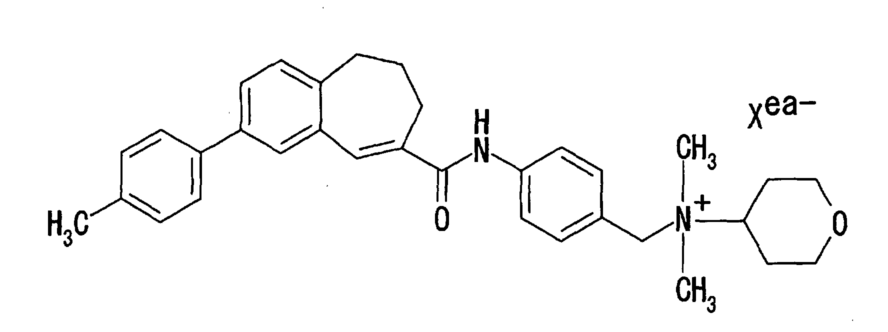 wherein X^{ea-} is an anion is preferable.

In the above formula, examples of the anion represented by X^{ea-} include an anion of a halogen atom; an anion derived from an inorganic acid such as hydrochloric acid, hydrobromic acid, nitric acid, sulfuric acid, phosphoric acid, etc.; an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; an anion derived from an acidic amino acid such as aspartic acid, glutamic acid, etc.; etc. Among them, an anion of a halogen atom is preferable.
In the above formula (eIb), the substituents of "phenyl group" in the "phenyl group which may be substituted" and the substituents of "thienyl group" in the "thienyl group which may be substituted" shown by R^{e1b} include those mentioned as the substituents in "5- to 6-membered cyclic ring group" shown by R^{e1}.

In the above formula (eIb), Y^{eb} is -CH₂-, -O- or -S-; and preferably -CH₂- or -O-.

In the above formula (eIb), R^{e2b}, R^{e3b} and R^{e4b} are independently an "optionally substituted aliphatic hydrocarbon group" or an "optionally substituted alicyclic heterocyclic ring group".

Examples of the "aliphatic hydrocarbon group" in the "optionally substituted aliphatic hydrocarbon group" represented by R^{e2b}, R^{e3b} and R^{e4b} include
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably C₁₋₆ alkyl, etc.);
(2) an optionally substituted cycloalkyl (e.g. C₃₋₈ cycloalkyl, etc. such as cyclopropyl, cyclobutyl, cyclopentyl, cyclohexyl, cycloheptyl, cyclooctyl, etc.);
(3) an optionally substituted alkenyl (e.g. alkenyl which has a carbon number of 2 to 10 such as allyl, crotyl, 2-pentenyl, 3-hexenyl, etc., preferably C₂₋₆alkenyl, etc.);
(4) an optionally substituted cycloalkenyl (e.g. cycloalkenyl which has a carbon number 3 to 7, such as 2-cyclopentenyl, 2-cyclohexenyl, 2-cyclopentenylmethyl, 2-cyclohexenylmethyl, etc.); etc.

Examples of the "alicyclic heterocyclic ring group" in the "optionally substituted alicyclic heterocyclic ring group" represented by R^{e2b}, R^{e3b} and R^{e4b} include group formed by removing a hydrogen atom from a 5- to 6-membered non-aromatic heterocyclic ring containing 1 to 4 hetero-atoms consisting of 1 to 2 kinds of hetero-atoms selected from nitrogen atom, sulfur atom and oxygen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, etc.; preferably a group formed by removing a hydrogen atom from a 5- to 6-membered saturated heterocyclic ring, containing 1 hetero-atom such as tetrahydrofuran, piperidine, tetrahydropyran, tetrahydrothiopyran, etc.); etc.

Examples of the substituents, which the "aliphatic hydrocarbon group" and the "alicyclic heterocyclic ring group" in the "optionally substituted aliphatic hydrocarbon group" and the "optionally substituted alicyclic heterocyclic ring group" represented by R^{e2b}, R^{e3b} and R^{e4b} may have, include halogen (e.g. fluorine, chlorine, bromine, iodine, etc.), an optionally halogenated C₁₋₄ alkyl, an optionally halogenated C₁-₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), phenyl, phenyl-C₁₋₄ alkyl, C₃₋₇ cycloalkyl, cyano, nitro, oxo, hydroxy, mercapto, amino, carboxyl, C₁₋₄ alkoxy-carbonyl (preferably, halogen, an optionally halogenated C₁₋₄ alkyl, an optionally halogenated C₁₋₄ alkoxy, phenyl-C₁₋₄ alkyl, C₃₋₇ cycloalkyl, cyano, oxo, hydroxy group, etc.), etc., and the number of the substituents are preferably 1 to 3.

In the above formula (eIb), as the group R^{e2b} or R^{e3b}, an optionally substituted chain hydrocarbon group is preferable, and an optionally substituted alkyl group is more preferable. In particular, the groups R^{e2b} and R^{e3b} are preferably the same and more preferably both of the groups R^{e2b} and R^{e3b} are methyl.

In the above formula (eIb), as the group R^{e4b}, an optionally substituted alicyclic hydrocarbon group or an optionally substituted alicyclic heterocyclic group is preferable and an optionally substituted cycloalkyl group or an optionally substituted saturated alicyclic heterocyclic ring group is more preferable. In particular, R^{e4b} is preferably an optionally substituted cyclohexyl or an optionally substituted 6-membered saturated alicyclic heterocyclic ring group and more preferably an optionally substituted cycloalkyl, an optionally substituted tetrahydropyranyl, an optionally substituted tetrahydrothiopyranyl or an optionally substituted piperidyl.
In the formula (eIc), R^{e1} has the same meaning given above.

In the above formula (eIc), the substituent of the 6-to 7-membered ring in a "6- to 7-membered ring which may be substituted" represented by A^{ec} include those which the "5-to 6-membered ring" in the "optionally substituted 5- to 6-membered ring" represented by R^{e1} may have. One to three (preferably one to two) of the substituents of A^{ec} are the same or different each other and may be present at any position on the ring.

In the group of the formula: 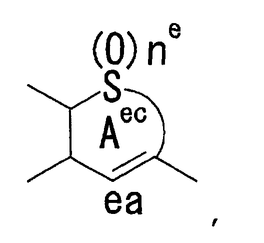 a carbon atom at the position ea is preferably unsubstituted.

Examples of the "optionally substituted 6- to 7-membered ring" represented by A^{ec} include a 6- to 7-membered ring group of the formula: 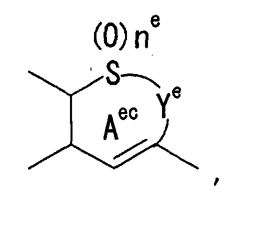 which may have a substituent at any possible position, etc.

In the above formula, Y^{e} has the same meaning given above.

Examples of the "substituents", which the "benzene ring" in the "optionally substituted benzene ring" represented by B^{ec} may have, include those for the "5- to 6-membered ring" in the "optionally substituted 5- to 6-membered ring" represented by R^{e1}, etc. Among them, halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc. are preferable and in particular, halogen, an optionally halogenated C₁₋₄ alkyl, an optionally halogenated C₁₋₄ alkoxy, etc. are preferable. The number of the substituents are preferably 1 to 3.

In the above formula (eIc), n is an integer of 1 or 2 (preferably, 2).

In the above formula (eIc), the divalent group represented by Z^{e2} has the same meaning given above. In the above formula (eIc), examples of (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium, (3) a group binding through a sulfur atom and (4) a group of the formula: 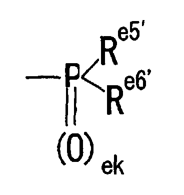 wherein each symbols has the same meanings given above, represented by R^{e2c} are the same as those of R^{e2} mentioned above.

In the above formula (eId), examples of the "5- to 6-membered aromatic ring" of the "5- to 6-membered aromatic ring which has a group of the formula: R^{ed}-Z^{e1d}-X^{ed}-Z^{e2d}- wherein R^{ed} is a hydrogen atom or an optionally substituted hydrocarbon group, X^{ed} is an optionally substituted alkylene chain, and Z^{e1d} and Z^{e2d} are respectively hetero-atoms, include a 6-membered aromatic hydrocarbon such as benzene, etc.; 5- to 6-membered aromatic heterocyclic ring containing 1 to 4 hetero-atoms consisting of 1 to 2 kinds of hetero-atoms selected from nitrogen atom, sulfur atom and oxygen atom such as furan, thiophene, pyrrole, imidazole, pyrazole, thiazole, oxazole, isothiazole, isoxazole, tetrazole, pyridine, pyrazine, pyrimidine, pyridazine, triazole, etc.; etc. Among them, benzene, furan, thiophene, pyridine, etc. are preferable, benzene, furan or thiophene is more preferable, and in particular, benzene is preferable.

Examples of the "hydrocarbon group" in the "optionally substituted hydrocarbon group" represented by R^{ed} include
(1) alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc., more preferably lower (C₁₋₄) alkyl, etc.);
(2) cycloalkyl (e.g. C₃₋₇ cycloalkyl, etc. such as cyclopropyl, cyclobutyl, cyclopentyl, cyclohexyl, cycloheptyl, etc.);
(3) alkenyl (e.g. alkenyl which has a carbon number of 2 to 10 such as allyl, crotyl, 2-pentenyl, 3-hexenyl, etc., preferably lower (C₂₋₆) alkenyl, etc.);
(4) cycloalkenyl (e.g. cycloalkenyl which has a carbon number of 3 to 7, etc. such as 2-cyclopentenyl, 2-cyclohexenyl, 2-cyclopentenylmethyl, 2-cyclohexenylmethyl, etc.);
(5) alkynyl (e.g. alkynyl which has a carbon number of 2 to 10 such as ethynyl, 1-propynyl, 2-propynyl, 1-butynyl, 2-pentynyl, 3-hexynyl, etc., preferably lower (C₂₋₆) alkynyl, etc.);
(6) aralkyl (e.g. phenyl-C₁₋₄ alkyl (e.g. benzyl, phenethyl, etc.), etc.);
(7) aryl (e.g. phenyl, naphthyl, etc.);
(8) cycloalkyl -alkyl (e.g. C₃₋₇ cycloalkyl-C₁₋₄ alkyl such as cyclopropylmethyl, cyclobutylmethyl, cyclopentylmethyl, cyclohexylmethyl, cycloheptylmethyl;), etc.

Examples of the substituents, which the above-mentioned (1) alkyl, (2) cycloalkyl, (3) alkenyl, (4) cycloalkenyl, (5) alkynyl, (6) aralkyl, (7) aryl and (8) cycloalkyl-alkyl may have, include halogen (e.g. fluorine, chlorine, bromine, iodine, etc.), nitro, cyano, hydroxy group, an optionally substituted thiol group (e.g., thiol, C₁₋₄ alkylthio, etc.), an optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), C₁₋₄ alkylenedioxy (e.g. -O-CH₂-O-, -O-CH₂-CH₂-O-, etc.), an optionally substituted sulfonamide [a group formed by combining an optionally substituted amino group (e.g. amino, a mono-C₁₋₄ alkyklamino, di-C₁₋₄ alkylamino, 5- to 6-membered cyclic amino such as tetrahydropyrrole, piperazine, piperidine, morpholine, thiomorpholine, pyrrole, imidazole, etc.) with -SO₂-, etc.], formyl, C₂₋₄ alkanoyl (e.g. acetyl, propionyl, etc.), C₁₋₄ alkylsulfonyl (e.g. methanesulfonyl, ethanesulfonyl, etc.), an optionally substituted heterocyclic group etc., and the number of the substituents are preferably 1 to 3.

The "heterocyclic group" in the "optionally substituted heterocyclic group" which is the substituent for the "optionally substituted hydrocarbon group" represented by R^{ed} is exemplified by a group which is formed by removing one hydrogen atom from an aromatic heterocyclic ring or non-aromatic heterocyclic ring. The aromatic heterocyclic ring include a 5- to 6-membered aromatic heterocyclic ring, which contains 1 to 4 hetero atoms of 1 to 2 kinds selected from the nitrogen atom, the sulfur atom and the oxygen atom, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole or the like, and the non-aromatic heterocyclic ring includes a 5- to 6-membered non-aromatic heterocyclic ring, which contains 1 to 4 heteroatoms of 1 to 2 kinds selected from the nitrogen atom, the sulfur atom and the oxygen atom, such as tetrahydrofuran, tetrahydrothiophene, dioxolan dithiolan, oxathiolan, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran or the like and non-aromatic heterocyclic ring group formed by being saturated all or a part of the bond of the ring (preferably, aromatic heterocyclic ring such as pyrazole, thiazole, oxazole tetrazole or the like) ., ..

Examples of "heterocyclic ring" of "heterocyclic group which may be substituted" which is the substituent of the "optionally substituted hydrocarbon group" represented by R^{ed}, may optionally have 1 to 3 substituents at any substitutable position. The substituent include a halogen atom (e.g. fluorine, chlorine, bromine, iodine, et.), nitro, cyano, hydroxyl, a thiol group which may be substituted (e.g. thiol, a C₁₋₄ alkylthio, etc.), an amino group which may be substituted (e.g. amino, a mono-C₁₋₄ alkylamino, a di-C₁₋₄ alkylamino, a 5- to 6- membered cyclic amino such as tetrahydropyrrole, piperazine, piperidine, morpholine, thiomorpholine, pyrrole, imidazole, etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), C₁₋₄ alkylenedioxy (e.g. -O-CH₂-O-, -O-CH₂-CH₂-O-, etc.), an optionally substituted sulfonamide [a group formed by combining an optionally substituted amino group (e.g. amino, a monoC₁₋₄ alkyklamino, diC₁₋₄ alkylamino, 5- to 6-membered cyclic amino such as tetrahydropyrrole, piperazine, piperidine, morpholine, thiomorpholine, pyrrole, imidazole, etc. ) with -SO₂-, etc.], formyl, C₂₋₄ alkanoyl (e.g. acetyl, propionyl, etc.), C₁₋₄ alkylsulfonyl (e.g. methanesulfonyl, ethanesulfonyl, etc.), (preferably C₁₋₄ alkyl etc.)

When the group of the formula: R^{ed}-Z^{e1d}-X^{ed}-Z^{e2d}- (wherein each symbol has the same meaning given above) is a monovalent group, (it does not bind to the 5- to 6-membered aromatic ring to form a ring), an optionally substituted alkyl group is preferable as the group R^{ed}, an optionally halogenated lower alkyl group is more preferable, and in particular, an optionally halogenated C₁₋₄ alkyl group is preferable.

In the above formula (eId), examples of the "optionally substituted alkylene chain" represented by X^{ed} include an optionally substituted straight or branched C₁₋₆ alkylene, etc. In said alkylene chain, a straight portion is preferably constituted by 1-4 carbon atoms, and in particular, an optionally substituted straight C₁₋₄ alkylene (preferably ethylene or propylene) is preferable as X^{ed}.

Examples of the substituent, which the "alkylene chain" in the "optionally substituted alkylene chain" represented by X^{ed} may have, include any one of which can bind to a divalent chain constituting the straight portion, for example, lower alkyl which has a carbon number of 1 to 6 (e.g. methyl, ethyl, propyl, isopropyl, butyl, isobutyl, sec-butyl, tert-butyl, pentyl, isopentyl, neopentyl, hexyl, etc.), lower (C₃₋₇) cycloalkyl (e.g. cyclopropyl, cyclobutyl, cyclopentyl, cyclohexyl, cycloheptyl, etc.), formyl, lower (C₂₋₇) alkanoyl (e.g. acetyl, propionyl, butyryl, etc.), an optionally esterified phosphono group, an optionally esterified carboxyl group, hydroxy group, oxo, etc., and more preferably lower alkyl which has a carbon number of 1 to 6 (preferably C₁₋₃ alkyl), hydroxy group, oxo, etc.

Examples of the optionally esterified phosphono group include a group of the formula: P(O)(OR^{e7d})(OR^{e8d}) wherein R^{e7d} and R^{e8d} are independently hydrogen, a alkyl group which has a carbon number of 1 to 6 or a cycloalkyl group which has a carbon number of 3 to 7, and R^{e7d} and R^{e8d} may bind to each other to form a 5- to 7-membered ring.

In the above formula, examples of the alkyl group which has a carbon number of 1 to 6 represented by R^{e7d} and R^{e8d} include methyl, ethyl, propyl, isopropyl, butyl, isobutyl, sec-butyl, tert-butyl, pentyl, isopentyl, neopentyl, hexyl, etc., and examples of the cycloalkyl which has a carbon number of 3 to 7 include cyclopropyl, cyclobutyl, cyclopentyl, cyclohexyl, cycloheptyl, etc. Among them, a straight-chain lower alkyl which has a carbon number of 1 to 6 is preferable and lower alkyl which has a carbon number of 1 to 3 is more preferable. The groups R^{e7d} and R^{e8d} may be same or different, and preferably the groups R^{e7d} and R^{e8d} are same. When R^{e7d} and R^{e8d} may bind to each other to form a 5- to 7-membered ring, the groups R^{e7d} and R^{e8d} bind to each other to represent a straight-chain C₂₋₄ alkylene side chain of the formula: -(CH₂)₂-, -(CH₂)₃-, -(CH₂)₄-, etc. Said side chain may have a substituent, and examples of the substituent include hydroxy group, halogen, etc.

Examples of the optionally esterified carboxyl group include an ester group formed by binding a carboxyl group to an alkyl group which has a carbon number of 1 to 6 or a cycloalkyl group which has a carbon number of 3 to 7 (e.g. methoxycarbonyl, ethoxycarbonyl, propoxycarbonyl, isopropoxycarbonyl, butoxycarbonyl, isobutoxycarbonyl, sec-butoxycarbonyl, tert-butoxy-carbonyl, pentyloxycarbonyl, hexyloxycarbonyl, etc.).

As the group X^{ed}, an optionally substituted C₁₋₄ alkylene is preferable, C₁₋₄ alkylene which may be substituted with C₁₋₃ alkyl, hydroxy group or oxo is more preferable, and in particular, a group of the formula: -(CH₂)ₑₙ- (en is an integer of 1-4) is preferable.

Examples of the hetero-atom represented by Z^{e1d} and Z^{e2d} include -O-, -S(O)ₑₘ- (em is an integer of 0 to 2), -N(R^{e4d})- (R^{e4d} is a hydrogen atom or an optionally substituted lower alkyl group), etc. As the group Z^{e1d}, -O- or -S(O)ₑₘ- (em is an integer of 0 to 2) is preferable, and -O- is more preferable. As the group Z^{e2d}, -O- or -N(R^{e4d})- (R^{e4d} is a hydrogen atom or an optionally substituted lower alkyl group) is preferable, and -O- is more preferable.

Examples of the optionally substituted lower alkyl group represented by R^{e4d} are similar to the above "optionally substituted lower alkyl group" exemplified in the "optionally substituted hydrocarbon group" represented by R^{ed} .

Examples of the further substituent, which the "5- to 6-membered ring" in the "5- to 6- membered aromatic ring which has a group of the formula: R^{ed}-Z^{e1d}-X^{ed}-Z^{e2d}- wherein each symbol is the same meaning given above, and which may have a further substituent" represented by R^{e1d} may have, in addition to the group of the formula: R^{ed}-Z^{e1d}-X^{ed}-Z^{e2d}-, include
a halogen atom, nitro, cyano, an optionally substituted alkyl, an optionally substituted cycloalkyl, an optionally substituted hydroxy group, an optionally substituted thiol group (wherein a sulfur atom may be oxidized to form an optionally substituted sulfinyl group or an optionally substituted sulfonyl group), an optionally substituted amino group, an optionally substituted acyl group, an optionally esterified or amidated carboxyl group, an optionally substituted aromatic group.

Examples of the halogen as the substituents for R^{e1d} include fluorine, chlorine, bromine, iodine, etc. Among them, fluorine and chlorine are preferable.

Examples of the alkyl in the optionally substituted alkyl as the substituents for R^{e1d} include a straight or branched alkyl which has a carbon number of 1 to 10 such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl.

Examples of the substituents in the optionally substituted alkyl include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkoxy (e.g. methoxy, ethoxy, propoxy, butoxy, trifluoromethoxy, trifluoroethoxy, etc.), an optionally halogenated C₁₋₄ alkoxy-C₁₋₄ alkoxy (e.g. methoxymethoxy, methoxyethoxy, ethoxyethoxy, trifluoromethoxyethoxy, trifluoroethoxyethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the cycloalkyl in the optionally substituted cycloalkyl as the substituents for R^{e1d} include C₃₋₇ cycloalkyl, etc. such as cyclopropyl, cyclobutyl, cyclopentyl, cyclohexyl, cycloheptyl, etc.

Examples of the substituents in the optionally substituted cycloalkyl include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁-₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), C formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the substituents in the optionally substituted hydroxy group as the substituents for R¹ include
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl which may contain a hetero-atom (e.g: C₃₋₇ cycloalkyl such as cyclopropyl, cyclobutyl, cyclopentyl, cyclohexyl, cycloheptyl, etc.; a saturated 5- to 6-membered heterocyclic ring group containing 1 to 2 hetero-atoms such as tetrahydrofuranyl, tetrahydrothienyl, pyrrolidinyl, pyrazolidinyl, piperidyl, piperazinyl, morpholinyl, thiomorpholinyl, tetrahydropyranyl, tetrahydrothiopyranyl, etc. (preferably, tetrahydropyranyl, etc.).; etc.);
(3) an optionally substituted alkenyl (e.g. alkenyl which has a carbon number of 2 to 10 such as allyl, crotyl, 2-pentenyl, 3-hexenyl, etc., preferably lower (C₂₋₆)alkenyl, etc.);
(4) an optionally substituted cycloalkenyl (e.g. cycloalkenyl which has a carbon number of 3 to 7, etc. such as 2-cyclopentenyl, 2-cyclohexenyl, 2-cyclopentenylmethyl, 2-cyclohexenylmethyl, etc.);
(5) an optionally substituted aralkyl (e.g. phenyl-C₁₋₄ alkyl (e.g. benzyl, phenethyl, etc.), etc.);
(6) formyl or an optionally substituted acyl (e.g. alkanoyl which has a carbon number of 2 to 4 (e.g, acetyl, propionyl, butyryl, isobutyryl, etc.), alkylsulfonyl which has a carbon number of 1 to 4 (e.g. methanesulfonyl, ethanesulfonyl, etc.), etc.);
(7) an optionally substituted aryl (e.g. phenyl, naphthyl, etc.); etc.

Examples of the substituents which the above-mentioned (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl and (7) optionally substituted aryl may have include halogen (e.g., fluorine, chlorine, bromine, iodine, etc.), nitro, cyano, hydroxy group, an optionally substituted thiol group (e.g., thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₆ alkoxy (e.g. methoxy, ethoxy, propoxy, butoxy, trifluoromethoxy, trifluoroethoxy, etc.; preferably an optionally halogenated C₁₋₄ alkoxy), formyl, C₂₋₄ alkanoyl (e.g. acetyl, propionyl, etc.), C₁₋₄ alkylsulfonyl (e.g. methanesulfonyl, ethanesulfonyl, etc.), an optionally substituted 5- to 6-membered aromatic heterocyclic ring [e.g. 5- to 6-membered aromatic heterocyclic ring containing 1 to 4 hetero-atoms consisting of 1 to 2 kinds of hetero-atoms selected from nitrogen atom, sulfur atom and oxygen atom such as furan, thiophene, pyrrole, imidazole, pyrazole, thiazole, oxazole, isothiazole, isoxazole, tetrazole, pyridine, pyrazine, pyrimidine, pyridazine, triazole, etc.; Examples of the substituents which said heterocyclic ring may have include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3], etc., and the number of the substituents are preferably 1 to 3.

Examples of the substituents in the optionally substituted thiol group as the substituents for R^{e1d} are similar to the above-described substituents in the optionally substituted hydroxy group as the substituents for R^{e1d}, and among them,
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₇ cycloalkyl, etc. such as cyclopropyl, cyclobutyl, cyclopentyl, cyclohexyl, cycloheptyl, etc.);
(3) an optionally substituted aralkyl (e.g. phenyl-C₁₋₄ alkyl (e.g. benzyl, phenethyl, etc.), etc.);
(4) an optionally substituted aryl (e.g. phenyl, naphthyl, etc.); etc. are preferable.

Examples of the substituents which the above-mentioned (1) optionally substituted alkyl, (2) optionally substituted cycloalkyl, (3) optionally substituted aralkyl and (4) optionally substituted aryl may have include halogen (e.g., fluorine, chlorine, bromine, iodine, etc.), nitro, cyano, hydroxy group, an optionally substituted thiol group (e.g., thio, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the substituents in the optionally substituted amino group as the substituents for R^{e1d} include an amino group which may have one to two substituents similar to the above-described substituents in the optionally substituted hydroxy group as the substituents for R^{e1d}, etc. Among them,
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₇ cycloalkyl, etc. such as cyclopropyl, cyclobutyl, cyclopentyl, cyclohexyl, cycloheptyl, etc.);
(3) an optionally substituted alkenyl (e.g. alkenyl which has a carbon number of 2 to 10 such as allyl, crotyl, 2-pentenyl, 3-hexenyl, etc., preferably lower (C₂₋₆) alkenyl, etc.);
(4) an optionally substituted cycloalkenyl (e.g. cycloalkenyl which has a carbon number of 3 to 7, etc. such as 2-cyclopentenyl, 2-cyclohexenyl, 2-cyclopentenylmethyl, 2-cyclohexenylmethyl, etc.);
(5) formyl or an optionally substituted acyl (e.g. alkanoyl which has a carbon number of 2 to 4 (e.g. acetyl, propionyl, butyryl, isobutyryl, etc.), which has a carbon number of 1 to 4 alkylsulfonyl (e.g. methanesulfonyl, ethanesulfonyl, etc.), etc.);
(6) an optionally substituted aryl (e.g. phenyl, naphthyl, etc.); etc. are preferable.

Examples of the substituents, which each of the above-described (1) optionally substituted alkyl, (2) optionally substituted cycloalkyl, (3) optionally substituted alkenyl, (4) optionally substituted cycloalkenyl, (5) optionally substituted acyl and (6) optionally substituted aryl may have, include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₉ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents is preferably 1 to 3.

The substituents in the optionally substituted amino group as the substituents for R^{e1d} may bind to each other to form a cyclic amino group (e.g. 5- to 6-membered cyclic amino, etc. such as tetrahydropyrrole, piperazine, piperidine, morpholine, thiomorpholine, pyrrole, imidazole, etc.). Said cyclic amino group may have a substituent, and examples of the substituents include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents is preferably 1 to 3.

Examples of the optionally substituted acyl as the substituents for R^{e1d} include a carbonyl group or a sulfonyl group binding to
(1) hydrogen;
(2) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(3) an optionally substituted cycloalkyl (e.g. C₃₋₇ cycloalkyl, etc. such as cyclopropyl, cyclobutyl, cyclopentyl, cyclohexyl, cycloheptyl, etc.);
(4) an optionally substituted alkenyl (e.g. alkenyl which has a carbon number of 2 to 10 such as allyl, crotyl, 2-pentenyl, 3-hexenyl, etc., preferably lower (C₂₋₆) alkenyl, etc.);
(5) an optionally substituted cycloalkenyl (e.g. cycloalkenyl which has a carbon number of 3 to 7, etc. such as 2-cyclopentenyl, 2-cyclohexenyl, 2-cyclopentenylmethyl, 2-cyclohexenylmethyl, etc.);
(6) an optionally substituted 5- to 6-membered monocyclic aromatic group (e.g. phenyl, etc., 5- to 6-membeed aromatic heterocyclic group containing 1 to 4 hetero-atoms of 1 to 2 kinds selected from nitrogen atom, sulfur atom and oxygen atom, such as furyl, thienyl, pyrrolyl, imidazole, pyrazolyl, thiazolyl, oxazolyl, isothiazolyl, isoxazolyl, tetrazolyl, pyridyl, pyrazyl, pyrimidyl, pyridazinyl, triazolyl, etc., preferably, pyridyl, thienyl, etc.)
(7) an optionally substituted 5- to 6-membered monocyclic non-aromatic heterocyclic group ( a group formed by removing one hydrogen atom from a 5- to 6-membered monocyclic non-aromatic heterocyclic ring which contains 1 to 4 heteroatoms of 1 to 2 kinds selected from nitrogen atom, sulfur atom and oxygen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, etc., preferably dioxolanyl, etc.) with carbonyl or sulfonyl group (e.g. acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl, etc.).

Examples of the substituents, which the above-mentioned (2) optionally substituted alkyl, (3) optionally substituted cycloalkyl, (4) optionally substituted alkenyl, (5) optionally substituted cycloalkenyl, (6) optionally substituted 5- to 6-membered monocyclic aromatic group and (7) optionally substituted 5- to 6-membered non-aromatic heterocyclic group may have, include a halogen (e.g. fluorine, chlorine, bromine, iodine, etc.), nitro, cyano, hydroxyl group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, a 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), C₁₋₄ alkylenedioxy (e.g. -O-CH₂-O-, -O-CH₂-CH₂-O-, etc.), an optionally substituted sulfonamide [a group formed by combining an optionally substituted amino group (e.g. amino, a monoC₁₋₄ alkyklamino, diC₁₋₄ alkylamino, 5- to 6-membered cyclic amino such as tetrahydropyrrole, piperazine, piperidine, morpholine, thiomorpholine, pyrrole, imidazole, etc. ) with -SO₂-, etc.], formyl, a C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the optionally esterified carboxyl group as the substituents for R^{e1d} include a carbonyloxy group binding to
(1) hydrogen;
(2) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(3) an optionally substituted cycloalkyl (e.g. C₃₋₇ cycloalkyl, etc. such as cyclopropyl, cyclobutyl, cyclopentyl, cyclohexyl, cycloheptyl, etc.);
(4) an optionally substituted alkenyl (e.g. alkenyl which has a carbon number of 2 to 10 such as allyl, crotyl, 2-pentenyl,3-hexenyl, etc., preferably lower (C₂₋₆) alkenyl, etc.);
(5) an optionally substituted cycloalkenyl (e.g. cycloalkenyl which has a carbon number of 2 to 1, etc. such as 2-cyclopentenyl, 2-cyclohexenyl, 2-cyclopentenylmethyl, 2-cyclohexenylmethyl, etc.);
(6) an optionally substituted aryl (e.g. phenyl, naphthyl, etc.); etc., and preferably carboxyl, lower (C₁₋₆) alkoxycarbonyl, aryloxycarbonyl (e.g. methoxycarbonyl, ethoxycarbonyl, propoxycarbonyl, phenoxycarbonyl, naphthoxycarbonyl, etc.), etc.

Examples of the substituents, which the above-mentioned (2) optionally substituted alkyl, (3) optionally substituted cycloalkyl, (4) optionally substituted alkenyl, (5) optionally substituted cycloalkenyl and (6) optionally substituted aryl may have, include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the optionally amidated carboxyl group as the substituents for R^{e1d} include an carbonyl group binding to an optionally substituted amino group, etc. which is similar to the above-described "optionally substituted amino group as the substituents for R^{e1d} ", and among others, carbamoyl, mono-C₁₋₆ alkylcarbamoyl, di-C₁₋₆ alkylcarbamoyl, etc. are preferable.

Examples of the aromatic group in the optionally substituted aromatic group as the substituents for R^{e1d} include 5- to 6-membered aromatic homocyclic or heterocyclic ring such as phenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl, triazolyl, oxadiazolyl, thiadiazolyl, etc.; fused aromatic heterocyclic ring such as benzofuran, indole, benzothiophene, benzoxazole, benzothiazole, indazole, benzimidazole, quinoline, isoquinoline, quinoxaline, phthalazine, quinazoline, cinnoline, etc.; etc.

Examples of the substituents for these aromatic groups include halogen (e.g. fluorine, chlorine, bromine, iodine, etc.), nitro, cyano, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), an optionally halogenated C₁₋₄ alkyl (e.g. trifluoromethyl, methyl, ethyl, etc.), an optionally halogenated C₁₋₄ alkoxy (e.g. methoxy, ethoxy, propoxy, butoxy, trifluoromethoxy, trifluoroethoxy, etc.), formyl, C₂₋₄ alkanoyl (e.g. acetyl, propionyl, etc.), C₁₋₄ alkylsulfonyl (e.g. methanesulfonyl, ethanesulfonyl, etc.), etc., and the number of the substituents is preferably 1 to 3.

The number of the above-mentioned substituents for R1 is 1-4 (preferably 1 to 2) and they may be same or different and present at any possible position on the ring.

When the group represented by R^{ed} binds to the 5 to 6 membered aromatic ring to form a ring, the group of the formula: R^{ed}-Z^{e1d}-X^{ed}-Z^{e2d}- wherein each symbol is as defined above (as the group R^{ed} is preferably a hydrogen atom). forms a divalent group such as lower (C₁₋₆) alkylenedioxy (e.g., -O-CH₂-O-, -O-CH₂-CH₂-O-, -O-CH₂-CH₂-CH₂-O-, etc.), oxy-lower (C₁₋₆) alkylene-amino (e.g., -O-CH₂-NH-, -O-CH₂-CH₂-NH-, etc.), oxy-lower (C₁₋₆) alkylenethio (e.g., -O-CH₂-S-, -O-CH₂-CH₂-S-, etc.), lower (C₁₋₆) alkylene-diamino (e.g., -NH-CH₂-NH-, -NH-CH₂-CH₂-NH-, etc.), thia-lower (C₁₋₆) alkylene-amino (e.g., -S-CH₂-NH-, -S-CH₂-CH₂-NH-, etc.), etc. Examples of the further substituent, which the "5 to 6 membered ring" in the "5 to 6 membered aromatic ring which has a group of the formula: R^{ea}-Z^{eld}-X^{ed}-Z^{e2d}- wherein each symbol is as defined above, and which may have a further substituent" represented by R^{e1d} may have, in addition to the group of the formula: R^{ed}-Z^{e1d}-X^{ed}-Z^{e2d}-, include a lower (C₁₋₄) alkyl optionally substituted with a halogen or a lower (C₁₋₄) alkoxy (e.g. methyl, ethyl, t-butyl, trifluoromethyl, methoxymethyl, ethoxymethyl, propoxymethyl, butoxymethyl, methoxyethyl, ethoxyethyl, propoxyethyl, butoxyethyl, etc.), a lower (C₁₋₄) alkoxy optionally substituted with a halogen or a lower (C₁₋₄) alkoxy (e.g. methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy, etc.), halogen (e.g. fluorine, chlorine, etc.), nitro, cyano, an amino optionally substituted with 1-2 lower (C₁₋₄) alkyl, formyl or lower (C₂₋₄) alkanoyl (e.g. amino, methylamino, dimethylamino, formylamino, acetylamino, etc.), 5- to 6-membered cyclic amino (e.g. 1-pyrrolidinyl, 1-piperazinyl, 1-piperidinyl, 4-morpholino, 4-thiomorpholino, 1-imidazolyl, 4-tetrahydropyranyl, etc.), etc.

When R^{e1d} is a benzene, the "group of the formula: R^{ed}-Z^{e1d}-X^{ed}-Z^{e2d}-" is preferably present at para position and the further substituent, which the "5 to 6 membered aromatic ring which may have, in addition to the group of the formula: R^{ed}-Z^{e1d}-X^{ed}-Z^{e2d}-, is preferably present at meta position.

In the above formula, examples of the "optionally substituted imino group" represented by Y^{ed} include a divalent group of the formula: -N (R^{e5d})- wherein R^{e5d} is a hydrogen atom or a substituent, etc.

As R^{e5d}, a hydrogen atom, an optionally substituted hydrocarbon group,
an optionally substituted heterocyclic group, an optionally substituted hydroxyl group, an optionally substituted thiol group (which may be oxidized, and form an optionally substituted sulfinyl group or an optionally substituted sulfonyl group), an optionally substituted amino group, an optionally esterified or amidated carboxyl group, and an optionally substituted acyl group etc., are preferable, and a hydrogen atom, an optionally substituted hydrocarbon group, an optionally substituted heterocyclic group, an optionally substituted acyl group, etc. are more preferable. Preferable examples of R^{e5d} include a hydrogen atom, an optionally substituted hydrocarbon group, an optionally substituted acyl group, etc., and C₁₋₄ alkyl, C₁₋₄ alkylsulfonyl, formyl, C₂₋₅ alkanoyl, etc. are more preferable, C₁₋₄ alkyl, formyl, C₂₋₅ alkanoyl, etc. are further more preferable, and in. particular, formyl or ethyl is preferable. As examples of preferable embodiment of R^{ed}, a group represented by the formula: -(CH₂)ₑₖ-R^{e6d}, wherein ek is 0 or 1, and R^{e6d} represents an optionally substituted 5-to 6- membered monocyclic aromatic group (for example, those similar to "(6) an optionally substituted 5- to 6-membered monocyclic aromatic group" mentioned in the item "an optionally substituted acyl group" as the substitutent of R^{ed} are mentioned, and phenyl, pyrazolyl, thiazolyl, oxazolyl, tetrazolyl, each of which may be substituted by a halogen, an optionally halogenated C₁₋₄ alkyl, an optionally halogenated C₁₋₄ alkoxy, etc., etc. are preferable.) .

Specific examples of the "optionally substituted hydrocarbon group" as R^{e5d} are similar to the "optionally substituted hydrocarbon group" as R^{ed}. Specific examples of the "optionally substituted heterocyclic group" as R^{ed} are similar to the "optionally substituted heterocyclic group" which is a substituent of the "optionally substituted hydrocarbon group" represented by R^{ed}. Specific examples of the "optionally substituted hydroxyl group", the "optionally substituted thiol group, the "optionally substituted amino group", the "optionally esterified or amidated carboxyl group" and the "optionally substituted acyl group", each of which is represented by R^{e5d}, are similar to the "optionally substituted hydroxyl group", the "optionally substituted thiol group", the "optionally substituted amino group", the "optionally esterified or amidated carboxyl group" and the "optionally substituted acyl group", each of which is the substituent of R^{e1d}, respectively.

In the above formula (eId), examples of the "optionally substituted aliphatic hydrocarbon group" (aliphatic chain hydrocarbon group and aliphatic cyclic hydrocarbon group) represented by R^{e2d} and R^{e3d} include
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₈ cycloalkyl, etc. such as cyclopropyl, cyclobutyl, cyclopentyl, cyclohexyl, cycloheptyl, cyclooctyl, etc.), provided that
   (2-1) said cycloalkyl may contain one hetero-atom selected from a sulfur atom, an oxygen atom and a nitrogen atom to form oxirane, thiorane, aziridine, tetrahydrofuran, tetrahydrothiophene, pyrrolidine, tetrahydropyran, tetrahydrothiopyran, tetrahydrothiopyran 1-oxide, piperidine, etc. (preferably, 6-membered ring such as tetrahydropyran, tetrahydrothiopyran, piperidine, etc.); that
   (2-2) said cycloalkyl may be fused with a benzene ring to form indane, tetrahydronaphthalene, etc. (preferably, indane, etc.); and that
   (2-3) said cycloalkyl may be clrosslinked via straight-chained, atom chain having a carbon number of 1 to 2, and may form a crosslinked cyclic hydrocarbon residue such as bicyclo[2.2.1]heptyl, bicyclo[2.2.2]octyl, bicyclo[3.2.1]octyl, bicyclo[3.2.2]nonyl, etc., preferably, a cyclohexyl group, etc. having a bridging comprising a straight chain constituted by 1-2 carbon atoms, and more preferably bicyclo[2.2.1]heptyl, etc.;
(3) an optionally substituted alkenyl (e.g. C₂₋₁₀ alkenyl such as allyl, crotyl, 2-pentenyl,3-hexenyl, etc., preferably lower (C₂₋₆)alkenyl, etc.);
(4) an optionally substituted cycloalkenyl (e.g. C₃₋₇ cycloalkenyl, etc. such as 2-cyclopentenyl, 2-cyclohexenyl, 2-cyclopentenylmethyl, 2-cyclohexenylmethyl, etc.); etc.

Examples of the substituents, which the above-mentioned (1) optionally substituted alkyl, (2) optionally substituted cycloalkyl, (3) optionally substituted alkenyl and (4) optionally substituted cycloalkenyl may have, include halogen (e.g. fluorine, chlorine, bromine, iodine, etc.), an optionally halogenated lower (C₁₋₄) alkyl, an optionally halogenated C₁₋₄ alkoxy (e.g., methoxy, ethoxy, propoxy, butoxy, trifluoromethoxy, trifluoroethoxy, etc.), C₁₋₄ alkylenedioxy (e.g., -O-CH₂-O-, -O-CH₂-CH₂-O-, etc.), formyl, C₂₋₄ alkanoyl (e.g., acetyl, propionyl, etc.), C₁₋₄ alkylsulfonyl (e.g., methanesulfonyl, ethanesulfonyl, etc.), phenyl-lower (C₁₋₄) alkyl, C₃₋₇ cycloalkyl, cyano, nitro, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), a lower (C₁₋₄) alkoxy-carbonyl, oxo group (preferably, halogen, an optionally halogenated lower (C₁₋₄) alkyl, an optionally halogenated lower (C₁₋₄) alkoxy, phenyl-lower (C₁₋₄) alkyl, C₃₋₇ cycloalkyl, cyano, hydroxy group, etc.), etc., and the number of the substituents are preferably 1 to 3.

examples of the "optionally substituted aliphatic hydrocarbon group" represented by R^{e2d} and R^{e3d} include
(1) a lower (C₁₋₆) straight or branched alkyl which may have 1-3 substituents selected from the class consisting of halogen, cyano, hydroxy group and C₃₋₇ cycloalkyl;
(2) C₅₋₈ cycloalkyl which may be substituted with 1-3 substituents selected from the class consisting of a halogen, an optionally halogenated lower (C₁₋₄) alkyl and a phenyl-lower (C₁₋₄) alkyl, which may contain a hetero-atom selected from the class consisting of a sulfur atom, an oxygen atom and a nitrogen atom, which may be fused with a benzene ring and which may be crosslinked via straight-chained, atom chain having the carbon number of 1 to 2 (e.g., cyclopentyl, cyclohexyl, cycloheptyl, cyclooctyl, tetrahydropyranyl, tetrahydrothiapyranyl, piperidinyl, indanyl, tetrahydronaphthalenyl, bicyclo[2.2.1]heptyl, etc., each of which may be substituted); etc.

In the above formula (eId), examples of the "optionally substituted alicyclic (non-aromatic) heterocyclic group" represented by Re^{2d} and R^{e3d} include 5-to 6-membered non-aromatic heterocyclic ring containing 1 to 4 hetero-atoms consisting of 1 to 2 kinds of hetero-atoms selected from nitrogen atom, sulfur atom and oxygen atom such as tetrahydrofuran, tetrahydrothiophene, dioxolane, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, etc., preferably, a 5- to 6-membered non-aromatic heterocyclic ring containing 1 hetero-atom such as tetrahydrofuran, piperidine, tetrahydropyran, etc. or the like.

Examples of the substituent, which the "alicyclic heterocyclic group" in the "optionally substituted alicyclic heterocyclic group" represented by R^{e2d} and R^{e3d} may have, include halogen (e.g. fluorine, chlorine, bromine, iodine, etc.), an optionally halogenated lower (C₁₋₄) alkyl, an optionally halogenated C₁₋₄ alkoxy (e.g., methoxy, ethoxy, propoxy, butoxy, trifluoromethoxy, trifluoroethoxy, etc.), C₁₋₄ alkylenedioxy (e.g., -O-CH₂-O-, -O-CH₂-CH₂-O-, etc.), formyl, C₂₋₄ alkanoyl (e.g., acetyl, propionyl, etc.), C₁₋₄ alkylsulfonyl (e.g., methanesulfonyl, ethanesulfonyl, etc.), phenyl-lower (C₁₋₄) alkyl, C₃₋₇ cycloalkyl, cyano, nitro, hydroxy group, an optionally substituted thiol group (e.g. thiol, C₁₋₄ alkylthio, etc.), an optionally substituted amino group (e.g. amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, etc., etc.), an optionally esterified or amidated carboxyl group (e.g. carboxyl, C₁₋₄ alkoxy-carbonyl, carbamoyl, mono-C₁₋₄ alkylcarbamoyl, di-C₁₋₄ alkylcarbamoyl, etc.), a lower (C₁₋₄) alkoxy-carbonyl, oxo group (preferably, halogen, an optionally halogenated lower (C₁₋₄) alkyl, an optionally halogenated lower (C₁₋₄) alkoxy, phenyl-lower (C₁₋₄) alkyl, C₃₋₇ cycloalkyl, cyano, hydroxy group, etc.), etc., and the number of the substituents are preferably 1 to 3.

Among them, as R^{e2d}, an optionally substituted acyclic hydrocarbon group (e.g., alkyl, alkenyl, etc., each of which may be substituted) is preferable, an optionally substituted lower C₁₋₆ alkyl group is more preferable, and in particular, an optionally substituted methyl group is preferable.

As R^{e3d}, an optionally substituted alicyclic hydrocarbon group (e.g., cycloalkyl, cycloalkenyl, etc., each of which may be substituted; preferably, an optionally substituted lower C₃₋₈ cycloalkyl group; and more preferably, an optionally substituted cyclohexyl) or an optionally substituted alicyclic heterocyclic group (preferably, an optionally substituted saturated alicyclic heterocyclic group (preferably, 6 membered ring group)); more preferably, an optionally substituted tetrahydropyranyl, an optionally substituted tetrahydrothiopyranyl or an optionally substituted piperidyl; and in particular, an optionally substituted tetrahydropyranyl) is preferable.

The compound represented by the formula (IV) may be hydrated, and the compound represented by the formula (IV), the salt thereof and the hydrate thereof are hereinafter referred to as Compound (IV).

Compound (IV) can be produced, for example, by the following manner.

### Production Method 1

As shown by the following formula, Compound (IV) can be produced by reacting a compound of the formula (IId) or a salt thereof (hereinafter referred to as Compound (IId)) with a compound represented by the formula (IIId), a salt thereof or a reactive derivative thereof at the carboxyl group hereinafter referred to as Compound (IIId). 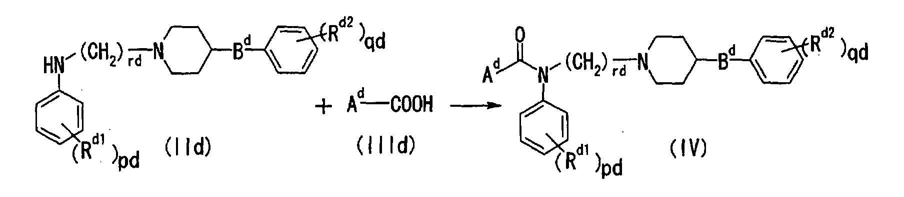 (wherein each symbol has the same meaning given above)

Examples of the reactive derivative at the carboxyl group of a compound represented by the formula (IIId) which can be used in an acylating reaction include, for example, an acid halide, an acid azide, an acid anhydride, a mixed acid anhydride, an active amide, an active ester, an active thio ester, an isocyanate, etc. Examples of the acid halide include, for example, an acid chloride, an acid bromide, etc.; examples of the mixed acid anhydrides include a mono-C₁₋₆alkyl-carbonic acid mixed acid anhydride (e.g. a mixed acid anhydride of free acid and monomethylcarbonic acid, monoethylcarbonic acid, mono-isopropylcarbonic acid, mono-isobutylcarbonic acid, mono-tert-butylcarbonic acid, mono-benzylcarbonic acid,.mono-(p-nitrobenzyl)carbonic acid, mono-allylcarbonic acid, etc.), a C₁₋₆ aliphatic carboxylic acid mixed acid anhydride (e.g. a mixed acid anhydride of free acid and acetic acid, trichloroacetic acid, cyanoacetic acid, propionic acid, butyric acid, isobutyric acid, valeric acid, isovaleric acid, pivalic acid, trifluoroacetic acid, trichloroacetic acid, acetoacetic acid, etc.), a C₇₋₁₂ aromatic carboxylic acid mixed acid anhydride (e.g. a mixed acid anhydride of free acid and benzoic acid, p-toluic acid, p-chloro benzoic acid, etc.), an organic sulfonic acid mixed acid anhydride (e.g. mixed acid anhydride of free acid and methanesulfonic acid, ethanesulfonic acid, benzenesulfonic acid, p-toluenesulfonic acid, etc.) etc.; examples of the active amide include an amide with a nitrogen-containing heterocyclic compound (an acid amide of a free acid and, for example, pyrazole, imidazole, benzotriazole, etc., these nitrogen-containing heterocyclic compound may be substituted with a C₁₋₆alkyl group (e.g., methyl, ethyl, etc.), a C₁₋₆alkoxy group (e.g., methoxy, ethoxy, etc.), a halogen atom (e.g., fluorine, chlorine, bromine, etc.), an oxo group, a thioxo group, a C₁₋₆alkylthio group (e.g., methylthio, ethylthio, etc.), etc.), etc.

As an active ester, all the active esters used in the field of the synthesis of β-lactam and peptide may be used. Examples of the active ester include, for example, an organic phosphoric acid ester (e.g. diethoxyphosphoric acid ester, diphenoxyphosphoric acid ester, etc.), p-nitrophenyl ester, 2,4-dinitrophenyl ester, cyanomethyl ester, pentachlorophenyl ester, N-hydroxysuccinimide ester, N-hydroxyphthalimide ester, 1-hydroxybenzotriazole ester, 6-chloro-1-hydroxybenzotriazole ester, 1-hydroxy-1H-2-pyridone ester, etc. Examples of the active thio ester include an ester of the acid with an aromatic heterocyclic thiol compound (e.g. 2-pyridylthiol ester, 2-benzothiazolylthiol ester, etc., which heterocyclics may be substituted with a C₁₋₆alkyl group (e.g. methyl, ethyl, etc.), a C₁₋₆alkoxy group (e.g., methoxy, ethoxy, etc.), a halogen atom (e.g., fluorine, chlorine, bromine, etc.), a C₁-₆alkyl-thio group (e.g., methylthio, ethylthio, etc.), etc.).

Examples of the leaving group represented by L include, for example, a halogen atom (e.g., a chlorine atom, a bromine atom, an iodine atom, etc.), an alkyl- or arylsulfonyloxy group (e.g., methanesulfonyloxy, trifluoromethanesulfonyloxy, ethanesulfonyloxy, benzenesulfonyloxy, p-toluenesulfonyloxy, etc.), etc. The reaction is usually carried out in a solvent inert to the reaction. Examples of the use for the solvent include an ether solvent (e.g., ethyl ether, diisoprpyl ether, dimethoxy ethane, tetrahydrofuran, dioxane, etc.), a halogenated solvent (e.g., dichloromethane, dicholoroethane, chloroform, etc.), an aromatic solvent (e.g., toluene, chlorobenzene, xylene, etc.), acetonitrile, N,N-dimethylformamide (DMF), acetone, methylethyl ketone, dimethylsulfoxide (DMSO), water, etc., or a mixed solvent thereof. Among them, acetonitrile, dichloromethane, chloroform, etc. are preferable. The reaction is usually carried out by using 1 to 5 equivalent, preferably 1 to 3 equivalents of Compound (IIId) relative to 1 equivalent of Compound (IId). The reaction temperature ranges from -20 °C to 50°C, preferably 0 °C to room temperature, and reaction time is usually 5 minutes to 100 hours. The reaction may smoothly proceed by using a base. As the base, an inorganic base and an organic base can be used effectively. Examples of the inorganic base include a hydroxide, a hydride, a carbonate, a bicarbonate of alkaline metal or alkaline earth metal. Among them, potassium carbonate, sodium carbonate, sodium hydroxide, potassium hydroxide, sodium hydrogencarbonate, potassium hydrogencarbonate are preferable. Examples of the organic base preferably include a tertiary amine such as triethylamine. Examples of the reactive derivative at the carboxyl group of the formula (IIId) are as mentioned above, and among them, an acid halide is preferable. The used amount of the base is usually 1 to 10 equivalents, preferably 1 to 3 equivalents relative to 1 equivalent of Compound IId).

The acylation reaction in which a carboxylic acid is used is carried out in an inert solvent (e.g., a halogenated solvent, acetonitrile) by reacting one equivalent of Compound (IId) with 1 to 1.5 equivalent of carboxylic acid (A^{d}-CO₂H) in the presence of 1 to 1.5 equivalent of dehydrating condensation agent such as dicyclohexyl carbodiimide (DCC), etc. The reaction is usually carried out at room temperature, and the reaction time is 0.5 to 24 hours.

Compound (IIId) used as the starting material in the reaction can be produced by a conventional manner using a compound described in Heterocycles, 43(10) 2131-2138 (1996) etc. as a starting compound. Compound (IId) used in the method can be produced by a manner described in Chem. Pharm. Bull. 47(1) 28-36 (1999) or Japanese unexamined patent publication No. 53654/1981 or a similar manner thereof.

Compound (IId) wherein rd is 3 can be produced, for example, by a method described in Synthetic Comm., 1991,20,3167-3180.

That is, the above compound can be produced by the following method by applying an addition reaction of amines or amides to unsaturated bond. 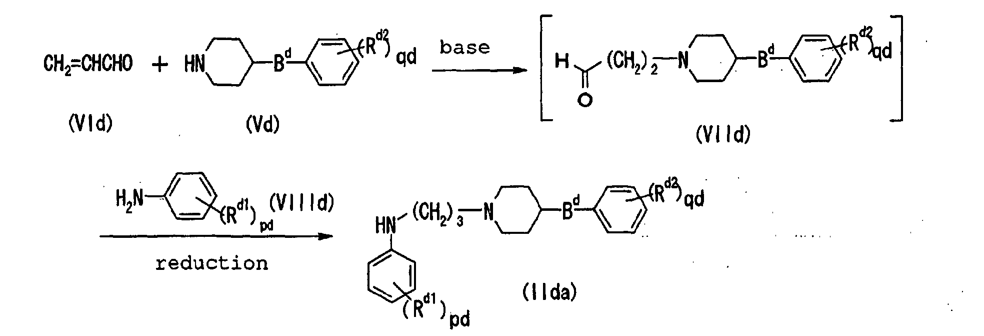 (wherein each symbol has the same meaning as defined above)

The compound can be produced by reacting acrolein derivatives (VId) with Compound (Vd), followed by reacting the resulting compound (VIId) with Compound (VIIId) under a condition of reduction. The reaction of Compound (VId) with Compound (Vd) is usually carried out in a solvent inert to the reaction in the presence of a base. Examples of the base include 1) a strong base such as hydride of alkali metal or alkaline earth metal (e.g., lithium hydride, sodium hydride, potassium hydride, calcium hydride, etc.), an amide of an alkali metal or an alkaline earth metal (e.g., lithium amide, sodium amide, lithium diisopropylamide, lithium dicyclohexylamide, lithium hexamethyldisilazide, sodium hexamethyldisilazide, potassium hexamethyldisilazide, etc.), a lower alkoxide of alkali metal or alkaline earth metal (e.g., sodium methoxide, sodium ethoxide, potassium t-butoxide, etc.), etc., 2) an inorganic base such as a hydroxide of an alkali metal or an alkaline earth metal (e.g., sodium hydroxide, potassium hydroxide, lithium hydroxide, barium hydroxide, etc.), a carbonate of an alkali metal or an alkaline earth metal (e.g., sodium carbonate, potassium carbonate, cesium carbonate, etc.), a bicarbonate of alkali metal or alkaline earth metal (e.g., sodium hydrogencarbonate, potassium hydrogencarbonate, etc.), etc., 3) an organic base etc., such an amine as triethylamine, diisopropylethylamine, N-methylmorpholine, dimethylaminopyridine, DBU(1,8-diazabicyclo[5.4.0]-7-undecene), DBN(1,5-diazabicyclo[4.3.0]non-5-ene), etc., etc., and such basic heterocyclic Compound, etc., as pyridine, imidazole, 2,6-lutidine, etc. Examples of the solvent include those mentioned in the reaction of Compound (IId) with Compound (IIId). These solvents can be used solely or in combination. Compound (VIId) can be obtained in the reaction.

In the reaction between Compound (VIId) and Compound (VIIId), examples of the reducing agent include sodium borohydride, lithium borohydride, sodium cyanoborohydride, sodium triacetoxyborohydride etc. The amount of the reducing agent used is usually 1 to 10 equivalents relative to 1 equivalent of Compound (VIId), preferably 1 to 4 equivalent. The reaction temperature is -20 to 50 °C , preferably 0 °C to ambient temperature, and the reaction time is 0.5 to 24 hours.

The reaction can also be carried out by a catalytic reduction reaction.
The catalytic reduction reaction is carried out in the presence of a catalytic amount of a metal catalyst such as Raney nickel, platinum oxide, metallic palladium, palladium-carbon, etc., in an inert solvent (e.g., an alcohol solvent such as methanol, ethanol, isopropanol, t-butanol, etc.), at room temperature to 100 °C, under a hydrogen pressure of 1 to 100 atm for 1 to 48 hours to produce Compound (IIda).

### Production 2

As shown below, Compound (IV) can be produced by reacting a compound (IVd) or its salt (herein after referred to as Compound (IVd)) with a compound (Vd) or its salt (herein after referred to as Compound (Vd)). 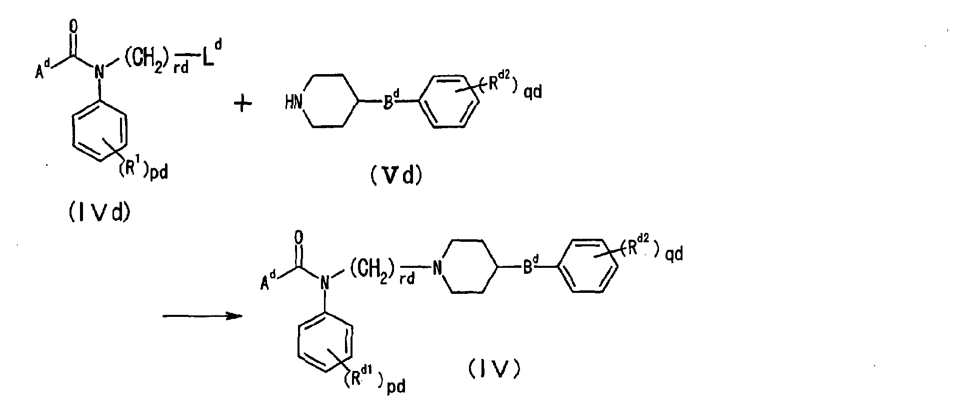 (wherein L^{d} is a leaving group, and the other symbols have the meaning given above)
Examples of the leaving group represented by L^{d} include, for example, a halogen atom (e.g., a chlorine atom, a bromine atom, an iodine atom, etc.), an alkyl or arylsulfonyloxy group (e.g., methanesulfonyloxy, trifluoromethanesulfonyloxy, ethanesulfonyloxy, benzenesulfonyloxy, p-toluenesulfonyloxy, etc.), etc.

The reaction can be carried out by a manner similar to that described in Organic Functional Group Preparations 2nd ed., (Academic Press, Inc.).

The reaction is usually carried out in a solvent inert to the reaction. Examples of the solvent include an alcohol solvent, an ether solvent, a halogenated solvent, an aromatic solvent, acetonitrile, N,N-dimethylformamide (DMF), acetone, methylethyl ketone, dimethylsulfoxide (DMSO), etc. These solvents can be used solely or in combination. Among them, acetonitrile, dimethylformamide, acetone, ethanol, etc., are preferable. The reaction temperature ranges usually from room temperature to 100 °C, preferably from room temperature to 50 °C and the reaction time is usually 0.5 to 1 day. In this reaction, a base is usually added in an amount of 1 to 3 equivalents relative to 1 equivalent of Compound (IVd), but it is not essential. Examples of the base include those mentioned in the reaction of Compound (II) with Compound (III).

Compound (IVd) used as the starting material in this reaction can be produced by a known conventional manner with the use of Compound (IIId) as a starting material. Examples of the salt of a compound of the formulas (I), (II), (III), (IV) and (eI) of the present invention include an acid addition salt such as a salt of an inorganic acid (e.g., hydrochloric acid salt, sulfuric acid salt, hydrobromic acid salt, phosphoric acid salt, etc.), a salt of an organic acid (e.g., acetic acid salt, trifluoroacetic acid salt, succinic acid salt, maleic acid salt, fumaric acid salt, propionic acid salt, citric acid salt, tartaric acid salt, lactic acid salt, oxalic acid salt, methanesulfonic acid salt, p-toluenesulfonic acid salt, etc.), etc., a salt with a base (e.g. an alkali metal salt such as potassium salt, sodium salt, lithium salt, etc., an alkaline earth metal salt such as calcium salt, magnesium salt, etc., ammonium salt, a salt with an organic base such as trimethylamine salt, triethylamine salt, tert-butyl dimethylamine salt, dibenzyl methylamine salt, benzyl dimethylamine salt, N,N-dimethylaniline salt, pyridine salt, quinoline salt, etc.).

The compounds having a CCR antagonistic effect used in the present invention include not only the compounds of the formulas (I), (II), (III), (IV) and (eI) and salts thereof but also compounds shown in WO00/38680, WO00/39125, EP1013276, WO00/66558, WO00/66559, WO02/074770, etc.

The preventing agent of the present invention shows an excellent preventing effect against HIV infection for animals, especially mammals (e.g. human, monkey, pig, dog, cat, rabbit, guinea pig, rat, mouse, etc.) and is useful for the prevention of HIV infection during blood transfusion or when blood products are used.

The preventing agent of the present invention is low-toxic, and when used as drug, it can be used as it is or by mixing with pharmaceutically acceptable carriers, excipients or diluents in a conventional manner to prepare a pharmaceutical preparation such as powders, granules, tablets, capsules (including soft capsule and microcapsule), liquid drug, injections, suppositories, etc. The preventing agent can be safely administered orally or parenterally, and the preventing agent itself can be administered by directly adding to blood for transfusion or blood product.

In the present application, the parenteral administration includes administration by subcutaneous injection, intravenous injection, intramuscular injection, intraperitoneal injection, intravenous drip, etc. An injectable preparation, for example, sterilized injectable aqueous suspension or oily suspension can be prepared by a method known in this field with the use of a suitable dispersing agent, wetting agent or suspending agent. The modifier for the sterilized injectable preparation may be diluents such as an aqueous solution which is nontoxic and capable of being parenterally administered, a sterilized injectable solution dissolved in a solvent or suspension. The carriers or solvents which can be used include water, Ringer solution, isotonic sodium chloride solution, etc. Furthermore, a solvent or a sterilized fixed oil as a suspension can usually be used. For this purpose, any fixed oil or any fatty acid can be used. For example, natural, synthesized or semisynthesized fatty oil or fatty acid, natural, synthesized or semisynthesized mono-, di- or triglyceride can be used.

Suppositories for rectal administration can be prepared by mixing the drug and a suitable non-irritative excipient such as cocoa butter, polyethylene glycols, etc., which is a solid in ambient temperature and a liquid at the temperature in the intestinal tract, and which will melt and release the drug in the intestinal tract.

Solid preparations for oral administration include powders, granules, tablets, pills, capsules, etc. In the preparations, active ingredient can be mixed with at least one additive, for example, saccharose, lactose, cellulose sugar, mannitol, maltitol, dextran, starchs, agar, alginates, chitins, chitosans, pectins, tragacanth chewing gums, acacias, gelatins, collagens, caseins, albumins, or synthesized or semisynthesized polymers or glycerides. The preparations can contain further additives, for example, inert diluent, lubricant such as magnesium stearate, etc., preservative such as parabens, sorbic acid, etc. antioxidant such as ascorbic acid, α-tocopherol, cysteine, etc., disintegrator, binding agent, thickener, buffer agent, sweetening agent, flavoring agent, and perfume, etc. Tablets and pills can also be prepared by enteric coating. Liquid drugs for oral administration include pharmaceutically acceptable emulsion, syrup, elixir, suspension, and liquid, etc., which may include inert diluents, eg, water, commonly used in the field.

The dosage of the preparation of the present invention can be selected with kind, age, weight and symptom of the patient, interval of the administration, administration route, dosage form, etc.

The dosage for one patient depends on age, weight, general health status, sex, meal, administration interval, administration method, excretion speed, the condition of the patient at the time of treatment and other conditions For example, when blood transfusion is carried out or blood product is used, the single dose of the present preparation which is orally administered to an adult human (body weight 60 kg) in order to prevent HIV infection is about 0.02 to 50mg/kg, preferably 0.05 to 30 mg/kg, more preferably 0.1 to 10 mg/kg as a compound having a CCR antagonistic effect. The preparation is administered at the above dose 1 to 3 times in a day.

The dosage range can be divided by the effective unit base. However, as mentioned above, taking the character and level of symptom, age, weight, general health status, sex, meal, administration interval, administration method, excretion speed and other factors into consideration, the dosage can be decided. The administration method of the preparation of the present invention can be properly selected, and the preparation can be added directly to blood for transfusion or blood product before blood transfusion or the use of blood product, respectively.

In this case, the preparation is desirably mixed with blood for transfusion or blood product immediately to 24 hours before the use, preferably immediately to 12 hours before the use and the most preferably immediately to 6 hours before the use At blood transfusion or when blood product is used, in case that the preventing agent of the present invention for HIV infection, is administered separately with the blood for transfusion or blood product, it is preferable to administer the preventing agent 1 hour before the blood transfusion or blood product use to the time of the blood transfusion or blood product use, and it is preferable to keep administering the preventing agent once to three time a day for four weeks.

The present invention is hereinafter described in more detail by means of the following Experiment and Formulation Example but are not construed as limitative to the present invention.

### Experiment

By using N,N-dimethyl-N-(4-(((2-(4-methylphenyl)-6,7-dihydro-5H-benzocyclohepten-8-yl)carbonyl)amino)benzyl)-N-(4-tetrahydropyranyl)ammonium chloride (Compound A) represented by the formula: 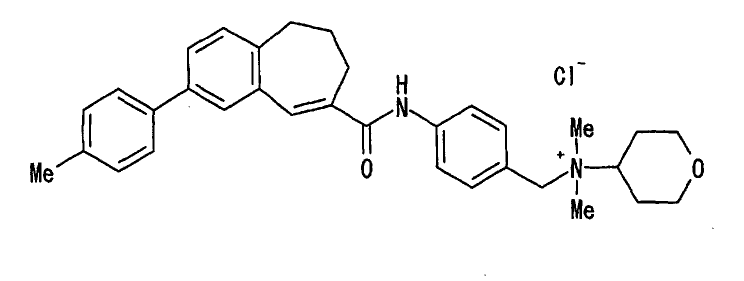 and compounds B to G represented by the formula:

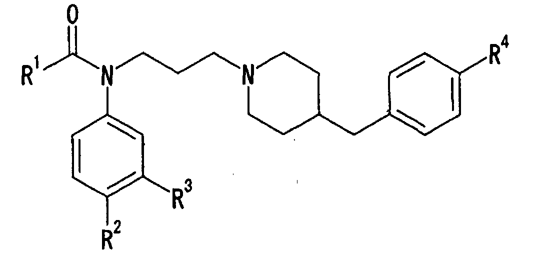

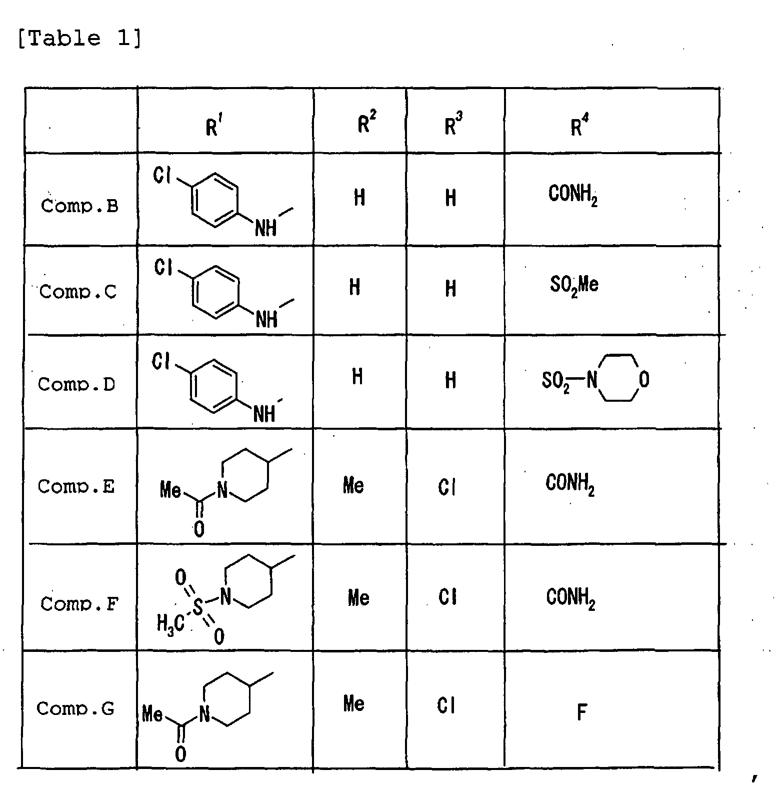

each of which is CCR5 antagonistic drug, inhibitory effect on HIV growth against HIV infection was examined.

### [Method]

### Cell

MOLT-4/CCR5 cell (AIDS Res.Hum.Retrovir 16, 935-941(2000)) was used.

### Drug

The compound was dissolved to DMSO and properly diluted with the RPMI 1640 medium including 10% FBS and 1mg/mL G418 (GIBCO).

### Virus

Ba-L strain which is a laboratory strain of R5 HIV-1 was used.

### Infection

Infection operation was carried out by adding virus of 1000CCID₅₀ to cells (4 ×10⁶ cells/1 mL) and cultivating it for 6 hours. After the viruses unadsorbed to cells were removed by washing, the infected cells were suspended in 2 ml of medium. To a 96 well plate were added 100 µL of the suspension of the infected cells and 100 µL of a test compound, and the mixture was cultivated at 37°C under 5% CO₂ atmosphere for 3 days. Three days after infection, the mixture was diluted to five times with a medium containing the test compound at the same concentration and further cultivated for 2 days. After cultivation, an amount of p24 in supernatant was measured with ELISA kit (ZeptoMetrix). The inhibitory rate of infection was calculated as an amount of p24 of the drug administration group to the amount of p24 of the control group.

### [Result]

To HIV-1 infected cells was added the test compounds and the mixture was cultivated for 5 days. Each of the test compounds shows strong inhibitory effect on infection at the concentration of 1000 nmol/L (Fig. 1).

### Pharmaceutical preparation 1 (Capsule)

| | | |
|---|---|---|
| (1) | N,N-Dimethyl-N-(4-(((2-(4-methylphenyl)-6,7-dihydro-5H-benzocyclo-hepten-8-yl)carbonyl)amino)-benzyl)-N-(4-tetrahydropyranyl)ammonium chloride | 40mg |
| (2) | Lactose | 70mg |
| (3) | Fine crystalline cellulose | 9mg |
| (4) | Magnesium stearate | 1mg |
| | One capsule | 120mg |
| (1), (2), (3) and 1/2 of (4) are mixed, and then granulated. To the granules, the reminder of (4) is added, and the whole is filled into a gelatin capsule. | | |

### Pharmaceutical Preparation 2 (Tablet)

| | | |
|---|---|---|
| (1) | N,N-dimethyl-N-(4-(((2-(4-methylphenyl)-6,7-dihydro-5H-benzocycloheten-8-yl)carbonyl)amino)-benzyl)-N-(4-tetrahydropyranyl)-ammonium chloride | 40mg |
| (2) | Lactose | 58mg |
| (3) | Corn starch | 18mg |
| (4) | Fine crystalline cellulose | 3.5mg |
| (5) | Magnesium stearate | 0.5mg |
| | One tablet | 120mg |
| (1), (2), (3), 2/3 of (4) and 1/2 of (5) are mixed and then granulated. To the granules, the reminders of (4) and (5) are added, followed by subjecting the mixture to compression molding to prepare a tablet. | | |

### Pharmaceutical Preparation 3 (Injection)

In Distilled water for injection suitable for standard of pharmacopoeia of Japan (10 ml) were dissolved N,N-dimethyl-N-(4-(((2-(4-methylphenyl)-6,7-dihydro-5H-benzocyclohepten-8-yl)carbonyl)amino)-benzyl)-N-(4-tetrahydropyranyl)ammonium chloride (500 mg), mannitol (1000 mg) and Polysorbate 80 (100 mg), and to the solution distilled water for injection suitable for standard of pharmacopoeia of Japan was added to make the volume to 20 ml. The solution was subjected to filtration under sterilization condition 0.2 ml each of the solution is filled in a vial for injection under sterilization condition and sealed.

### INDUSTRIAL APPLICABILITY

The preventing agent of the present invention which contains a compound having a CCR antagonistic effect can be used for the prevention of HIV infection in transfusing blood or using a blood product.

## Claims

1. An agent for preventing HIV infection in transfusing blood or using a blood product, which comrises a compound having a CCR antagonistic effect.

2. An agent as claimed in claim 1, wherein CCR is CCR5 and/or CCR2.

3. An agent as claimed in claim 1, wherein the compound having CCR antagonistic effect is N-(3,4-dichlorophenyl)-1-(methylsulfonyl)-N-{3-[4-({4-[(methylsulfonyl)amino]phenyl}sulfonyl)-1-piperidinyl]propyl]-4-piperidinecarboxamide, N-(3-chlorophenyl)-1-(methylsulfonyl)-N-(3-{4-[4-(methylsulfonyl)benzyl]-1-piperidinyl}propyl)-4-piperidinecarboxamide, N-(3-{4-[4-(aminocarbonyl)benzyl]-1-piperidinyl}propyl)-N-(3,4-dichlorophenyl)-1-(methylsulfonyl)-4-piperidinecarboxamide, 1-acetyl-N-(3-{4-[4-(aminocarbonyl)benzyl]-1-piperidinyl}propyl)-N-(3-chloro-4-methylphenyl)-4-piperidinecarboxamide, N-(3,4-dichlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(isopropylsulfonyl)benzyl]-1-piperidinyl}propyl)-1-(methylsulfonyl)-4-piperidinecarboxamide, N-(3-chlorophenyl)-N-(3-{4-[4-(ethylsulfonyl)benzyl]-1-piperidinyl}propyl)-1-(methylsulfonyl)-4-piperidinecarboxamide, N-(3,4-dichlorophenyl)-1-(methylsulfonyl)-N-(3-{4-[4-(methylsulfonyl)benzyl]-1-piperidinyl}propyl)-4-piperidinecarboxamide, N-(3-{4-[4-(aminocarbonyl)benzyl]-1-piperidinyl}propyl)-N-(3-chloro-4-methylphenyl)-1-(methylsulfonyl)-4-piperidinecarboxamide, N-[3-(4-benzyl-1-piperidinyl)propyl]-N'-(4-chlorophenyl)-N-phenylurea, N'-(4-chlorophenyl)-N-{3-[4-(4-fluorobenzyl)-1-piperidinyl]propyl}-N-phenylurea, N'-(4-chlorophenyl)-N-(3-{4-[4-(4-morpholinylsulfonyl)benzyl]-1-piperidinyl}propyl)-N-phenylurea, N'-(4-chlorophenyl)-N-(3-{4-[4-(4-methylsulfonyl)benzyl]-1-piperidinyl}propyl)-N-phenylurea, 4-{[1-(3-([(4-chloroanilino)carbonyl]anilino}propyl)-9-piperidinyl]methyl}benzamide, N-[3-(4-benzyl-1-piperidinyl)propyl]-N-(3,4-dichlorophenyl)-1-methyl-5-oxo-3-pyrrolidinecarboxamide, 1-benzyl-N-[3-(4-benzyl-1-piperidinyl)propyl]-5-oxo-N-phenyl-3-pyrrolidinecarboxamide, N-[3-(4-benzyl-1-piperidinyl)propyl]-1-(2-chlorobenzyl)-5-oxo-N-phenyl-3-pyrrolidinecarboxamide, N-(3,4-dichlorophenyl)-N-{3-[4-(4-fluorobenzyl)-1-piperidinyl]propyl}-1-methyl-5-oxo-3-pyrrolidinecarboxamide, N-[3-(4-benzyl-1-piperidinyl)propyl]-5-oxo-N-phenyl-1-(2,2,2-trifluoroethyl)-3-pyrrolidinecarboxamide, N-(3,4-dichlorophenyl)-N-(3-{4-[4-(methylsulfonyl)benzyl]-1-piperidinyl}propyl)-2-[1-(methylsulfonyl)-4-piperidinyl]acetamide, N-(3,4-dichlorophenyl)-N-(3-{4-[4-(isopropylsulfonyl)benzyl]-1-piperidinyl}propyl)-2-[1-(methylsulfonyl)-4-piperidinyl]acetamide, 3-(1-acetyl-4-piperidinyl)-N-(3,4-dichlorophenyl)-N-(3-{4-[4-(methylsulfonyl)benzyl]-1-piperidinyl}propyl)propanamide, or N-(3,4-dichlorophenyl)-4-hydroxy-1-(methylsulfonyl)-N-(3-{4-[4-(methylsulfonyl)benzyl]-1-piperidinyl}propyl)-4-piperidinecarboxamide or a salt thereof.

4. An agent as claimed in claim 1, wherein the compound having CCR antagonistic effect is N-methyl-N-[4-[[[2-(4-methylphenyl)-6,7-dihydro-5H-benzocyclohepten-8-yl]carbonyl]amino]benzyl]piperidinium iodide, N-methyl-N-[4-[[[7-(4-methylphenyl)-2,3-dihydro-1-benzoxepin-4-yl]carbonyl]amino]benzyl]piperidinium iodide, N-[4-[N-methyl-N-(tetrahydropyran-4-yl)aminomethyl]phenyl]-7-(4-methylphenyl)-2,3-dihydro-1-benzoxepin-4-carboxamide, N-[4-[N-methyl-N-(tetrahydropyran-4-yl)aminomethyl]phenyl]-7-(4-morpholinophenyl)-2,3-dihydro-1-benzoxepin-4-carboxamide, 7-(4-ethoxyphenyl)-N-[4-[N-methyl-N-(tetrahydropyran-4-yl)aminomethyl]phenyl]-2,3-dihydro-1-benzoxepin-4-carboxamide, N,N-dimethyl-N-[4-[[[2-(4-methylphenyl)-6,7-dihydro-5H-benzocyclohepten-8-yl]carbonyl]amino]benzyl]-N-(tetrahydropyran-4-yl) ammonium iodide, N-methyl-N-[4-[[[7-(4-methylphenyl)-3,4-dihydronaphthalen-2-yl]carbonyl]amino]benzyl]piperidinium iodide, N,N-dimethyl-N-(4-(((2-(4-methylphenyl)-6,7-dihydro-5H-benzocyclohepten-8-yl)carbonyl)amino)benzyl)-N-(4-tetrahydropyranyl)ammonium chloride, N,N-dimethyl-N-(((7-(4-methylphenyl)-2,3-dihydro-1-benzoxepin-4-yl)carbonyl)amino)benzyl)-N-(4-oxocyclohexyl)ammonium chloride, N-(4-(((7-(4-ethoxyphenyl)-2,3-dihydro-1-benzoxepin-4-yl)carbonyl)amino)benzyl)-N,N-dimethyl-N-(4-tetrahydropyranyl)ammonium chloride, N-[4-[N-methyl-N-(tetrahydropyran-4-yl)aminomethyl]phenyl]-7-(4-propoxyphenyl)-1,1-dioxo-2,3-dihydro-1-benzothiepin-4-carboxamide, 7-(4-butoxyphenyl)-N-[4-[N-methyl-N-(tetrahydropyran-4-yl)aminomethyl]phenyl]-1,1-dioxo-2,3-dihydro-1-benzothiepin-4-carboxamide, 7-[4-[N-methyl-N-(2-propoxyethyl)amino]phenyl]-N-[4-[[N-methyl-N-(tetrahydropyran-4-yl)amino]methyl]phenyl]-1,1-dioxo-2,3-dihydro-1-benzothiepin-4-carboxamide, 7-[4-(2-ethoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, N-[4-[[N-methyl-N-(tetrahydropyran-4-yl)amino]methyl]phenyl]-7-[4-(2-propoxyethoxy)phenyl]-1,1-dioxo-2,3-dihydro-1-benzothiepin-4-carboxamide, 7-[4-(2-butoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, 7-[4-(2-ethoxyethoxy)-3,5-dimethylphenyl]-N-[4-[[N-methyl-N-(tetrahydro-2H-pyran-4-yl)amino]methyl]phenyl]-1,1-dioxo-2,3-dihydro-1-benzothiepin-4-carboxamide, 7-[2-chloro-4-(2-propoxyethoxy)phenyl]-N-[4-[[N-methyl-N-(tetrahydropyran-4-yl)amino]methyl]phenyl]-1,1-dioxo-2,3-dihydro-1-benzothiepin-4-carboxamide, 7-(3-methyl-4-propoxyphenyl)-N-[4-[[N-methyl-N-(tetrahydropyran-4-yl)amino]methyl]phenyl]-1,1-dioxo-2,3-dihydro-1-benzothiepin-4-carboxamide, 7-(3,4-dipropoxyphenyl)-N-(4-((N-methyl-N-(tetrahydro-2H-pyran-4-yl)amino)methyl)phenyl)-1,1-dioxo-2,3-dihydro-1-benzothiepin-4-carboxamide, 7-[4-(2-ethoxyethoxy)phenyl]-1-ethyl-N-[4-[[N-methyl-N-(tetrahydropyran-4-yl)amino]methyl]phenyl]-2,3-dihydro-1-benzoazepin-4-carboxamide, 1-ethyl-7-[4-(2-propoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-ethyl-N-[4-[[N-methyl-N-(tetrahydropyran-4-yl)amino]methyl]phenyl]-2,3-dihydro-1-benzoazepin-4-carboxamide, 7-[4-(2-ethoxyethoxy)phenyl]-1-formyl-N-[4-[[N-methyl-N-(tetrahydropyran-4-yl)amino]methyl]phenyl]-2,3-dihydro-1-benzoazepin-4-carboxamide, 1-formyl-7-[4-(2-propoxyethoxy)phenyl]-N-[9-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-formyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-5-yl)amino]methyl]phenyl]-1-propyl-2,3-dihydro-1-benzoazepin-4-carboxamide, N-[4-[[N-methyl-N-(tetrahydropyran-5-yl)amino]methyl]phenyl]-7-[4-(2-propoxyethoxy)phenyl]-1-propyl-2,3-dihydro-1-benzoazepin-4-carboxamide, 1-benzyl-7-[4-(2-butoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-cyclopropylmethyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-phenyl-2,3-dihydro-1-benzoazepin-4-carboxamide, 7-[4-(2-butoxyethoxy)phenyl]-1-(3,4-methylenedioxy)phenyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(2-methyloxazol-5-yl)-N-[4-[[N-methyl-N-(tetrahydropyran-4-yl)amino]methyl]phenyl]-2,3-dihydro-1-benzoazepin-4-carboxamide, 1-allyl-7-[4-(2-butoxyethoxy)phenyl]-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(3-thienyl)methyl-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(thiazol-2-yl)methyl-2,3-dihydro-1-benzoazepin-4-carboxamide, 7-[4-(2-butoxyethoxy)phenyl]-1-(1-methylpyrazol-4-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(3-methylisothiazol-5-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-(1-ethylpyrazol-4-yl)methyl-N-[4-[[N-methyl-N-(tetrahydropyran-4-yl)amino]methyl]phenyl]-2,3-dihydro-1-benzoazepin-4-carboxamide, 7-[4-(2-butoxyethoxy)phenyl]-1-isobutyl-N-[4-[[N-methyl-N-(tetrahydropyran-5-yl)amino]methyl]phenyl]-2,3-dihydro-1-benzoazepin-4-carboxamide, 1-isobutyl-N-[4-[[N-methyl-N-(tetrahydropyran-5-yl)amino]methyl]phenyl]- 7-[4-(2-propoxyethoxy)phenyl]-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(thiazol-5-yl)methyl-2,3-dihydro-1-benzoazepin-4-carboxamide, 7-[4-(2-butoxyethoxy)phenyl]-N-[4-[[N-methyl-N-(tetrahydropyran-4-yl)amino]methyl]phenyl]-1-(1-methyltetrazol-5-yl)methyl-2,3-dihydro-1-benzoazepin-4-carboxamide, or 7-[4-(2-butoxyethoxy)phenyl]-N-[4-[[N-methyl-N-(tetrahydropyran-4-yl)amino]methyl]phenyl)-1-(2-methyltetrazol-5-yl)methyl-2,3-dihydro-1-benzoazepin-4-carboxamide or a salt thereof.

5. Blood for transfusion or blood product, which contains a compound having a CCR antagonistic effect.

6. A method for preventing HIV infection which comprises administering a blood for transfusion or blood product each of which contains a compound having CCR antagonistic effect.

7. A method for preventing HIV infection which comprises administering an effective amount of a compound having CCR antagonistic effect in transfusing blood transfusion or using a blood product.

8. A method as claimed in claim 7, wherein the time of blood transfusion or blood product use is from one hour before blood transfusion or blood product use to the time of blood transfusion or blood product use.
